# EUROPEAN PATENT APPLICATION

(11) **EP 3 763 715 A1**
(43) Date of publication of application: **13.01.2021**
(21) Application number: 20158101.4
(22) Date of filing: 18.02.2020
(51) Int. Cl.: C07D 491/04, C07D 491/20, A61K 31/55, A61P 35/00

(54) **HARRINGTONINES SALTS, IN PARTICULAR RETINOATES, THEIR PROCESS OF PREPARATION AND THEIR USES IN THE TREATMENT OF LEUKEMIAS, CANCERS, AUTOIMMUNE, SKIN, ALZHEIMER'S AND INFLAMMATORY BOWEL DISEASES AND VIRAL INFECTIONS, COMBINED WITH MYELOPOIESIS STIMULATING AGENTS**

(30) Priority: 11.07.2019 US 201962872819 P
(71) Applicant: Robin, Jean-Pierre, 1920 Martigny (CH)
(72) Inventor:
(74) Representative: Regimbeau

(57) **Abstract**

The present invention relates to salt of the following formula (I):

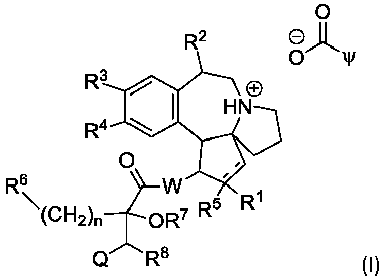

Another object of the invention is a method for preventing or treating diseases selected among cancer, leukemias, lymphomas, myelodysplastic syndrome, autoimmune diseases, skin diseases, neurological diseases such as Alzheimer's disease, inflammatory bowel disease, such as Crohn's disease, and viral infections including papillomavirus comprising administering to a patient in need thereof an effective dose of the salt of formula (I) or a pharmaceutical composition comprising thereof.

The present invention also relates to a method of preparation of the salt of formula (I).

## Description

### Field of the invention

The present invention relates to novel water-soluble harringtonines retinoates as a series of highly bioactive alkaloid salts, to their process of preparation and to their use as a drug, in particular in the treatment of cancer, leukemias, lymphomas, myelodysplastic syndrome, autoimmune diseases, skin diseases, neurological diseases such as Alzheimer's disease, inflammatory bowel disease, such as Crohn's disease, and viral infections preferably combined with myelopoiesis stimulating agents and, eventually radiation therapies given simultaneously, sequentially and/or iteratively.

### Background of the invention

Harringtonines (1) are highly active particular esters of cephalotaxines (2). Cephalotaxines themselves are devoid of biological activity. Cephalotaxanes (including (1) and (2)), occurs in all parts of the tree, a rare and threatened Asian coniferous species pertaining to the genus Cephalotaxus.

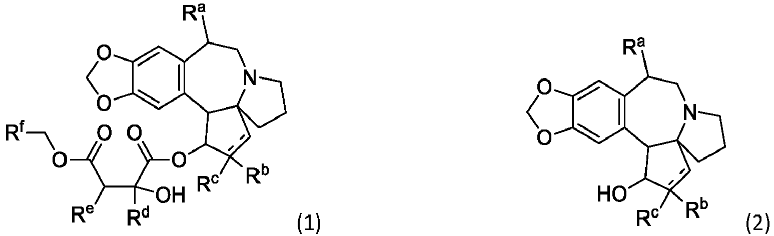

Although known for nearly fifty years for their anticancer activity, cephalotaxine esters have been the subject of renewed interest in recent years because of the activity of some of their representatives in the myeloid leukemias [(i) Kantarjian HM, et al. Cancer 2001 p591 (ii) Jin J, et al. Lancet Oncol 2013 p599] and all recently in several solid tumors such as breast cancers [Radosevic et al. WO2018055136A1]. Namely, one of these alkaloids, homoharringtonine, has even been recently approved in the United States in the treatment of chronic myeloid leukemia [Alvandi et al The Oncologist 2013 "U.S. Food and Drug Administration Approval Summary Omacetaxine Mepesuccinate as Treatment for Chronic Myeloid Leukemia"]*.* Since the development of the first hemi-synthesis of homoharringtonine by Robin *et al.* from 1998 [Robin et al, US 6,831,180, Tetrahedron Lett. 1999 p2931], the quantitative (rarity) and qualitative (purity) factors that hitherto limited the clinical development of homoharringtonine (namely omacetaxine or HHT) have been overcome.

Very recently, the hope of a new commercial development has been born, due to the invention of a purification method involving the formation of water-soluble crystalline salts as new chemical entity [Robin et al. WO 2015128463, 2015]. Thus, this new approach yielded a product of natural origin exhibiting the same level of quality than the one of semisynthetic origin one and thus making it possible to place a generic form on the market at an affordable price [Blood 2013(121) p.4439 and Kantarjian et al., J. Clin. Onc., 2013 p3600]. Apart from their salt forms, harringtonines are well known for their broad spectrum of antiviral activity and more particularly with regard to non human Coronaviridae [Dong et al, Viruses, 2018 p 601 "The Natural Compound Homoharringtonine Presents Broad Antiviral Activity In Vitro and In Vivo*".* Indeed, in 2015, HHT was included in 3 inhibitors selected among 727 compounds [J Cao et al, Antiviral Research, 2015, p.1 "A screen of the NIH Clinical Collection small molecule library identifies potential anti-coronavirus drugs"]. This strong activity of harringtonines with regards to non human Coronaviridae activity was pointed out in the recent U.S. patent application of Robin and Radosevic [Robin et al., Application Number 62872819, 11-Jul-2019 "Harringtonines, retinoates salts, their process of preparation and their uses in the treatment of leukemias, cancers, autoimmune, skin, Alzheimer's and inflammatory bowel diseases and viral infections, alone or combined with other therapies including radiation"].

Semisynthetic analogs such as OP5215 **(3)** (by F. Dumas et al. Chap. IV "Cephalotaxus Alkaloids, 5. Synthetic Analogs and their Biologic Properties, 5.2.1. Robin's analogs" in The Alkaloids Chemistry and Biology Vol 78 H-J Knölker Ed, Academic Press, 2017) having antileukemic activity 5 times greater than that of HHT, have been described by Radosevic et al. [*"*OP5215, a new semisynthetic homoharringtonine derivative, is highly cytotoxic on multidrug-resistant cell lines." Blood. 2000 96:306a].

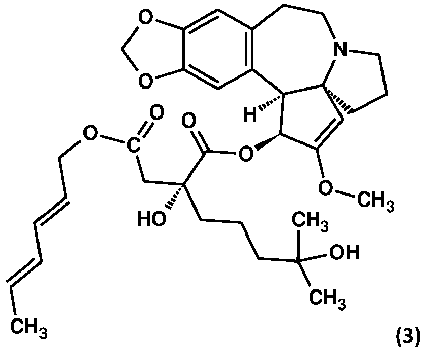

However, salts of retinoates of harringtonines have never been described in the prior art.

Retinoids are polyunsaturated or aromatic acids originally derived from vitamin A and possessing pharmacological properties of extraordinary richness. For example, but not limited to, retinoids are used in chemotherapy of cancers, leukemias, in various skin diseases and in a number of chronic diseases [Aryal, Int. J. Pharm Sc Res, 2017 p 3630 "A Brief Review on systemic retinoids"; The Retinoids Biology, biochemistry, and Disease", Ed by Dollé and Niederreither, Wiley Blackwell, 2015, Hoboken, New jersey, Kindle Edition and cited references]. For an example, among retinoids, all-trans-retinoic acid (ATRA or atRA) **(4)** has revolutionized the treatment, of Acute Promyelocytic Leukemia (APL), a very serious type of myeloid leukemia also present in young adult [Wang ZY et al. Blood 2008 p2505"Acute promyelocytic leukemia: From highly fatal to highly curable."].

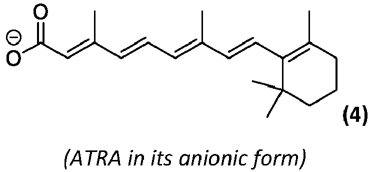

Some retinoids are used in certain skin diseases such as psoriasis and AIDS related Kaposi's sarcoma and also in inflammatory bowel disease such as Crohn disease. They are also known for their protective effect against certain solid tumors. Finally, these products are active on several skin diseases including, but not limited to, those of autoimmune origin such as psoriasis or systemic erythematous lupus and in neuropsychiatric disease such as Alzheimer's disease. Retinoids exhibit also potent antiviral activities including but not limited to activity on papillomavirus, a family of virus with high carcinogenetic potential [Borger et al, virology 2000, p397, "Retinoic Acid Resistance at Late Stages of Human Papillomavirus Type 16-Mediated Transformation of Human Keratinocytes Arises Despite Intact Retinoid Signaling and Is Due to a Loss of Sensitivity to Transforming Growth Factor-beta].

There are 3 generations of retinoids: the first generation includes, among others, the well-known all-*trans*-retinoic acid (ATRA) and its less oxidized derivatives, whose natural mixture constitutes "vitamin A" itself. The second generation comprises semi-synthetic derivatives in which the terminal cyclic substituent has been modified such as **(5-1)** and **(5-2).**

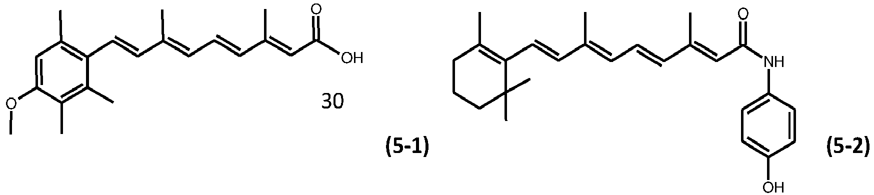

In the 3rd generation derivatives, the chain has been replaced by unsaturated or aromatic polycyclic substituents mimicking the conjugated polyethylenic chain of the 1st generation compounds such as, but not limited to, compounds having formulas **(6-1)** to **(6-16).** There are hundreds of 3^{rd} generation derivatives described in literature active or potentially active in above cited diseases which represent a huge market.

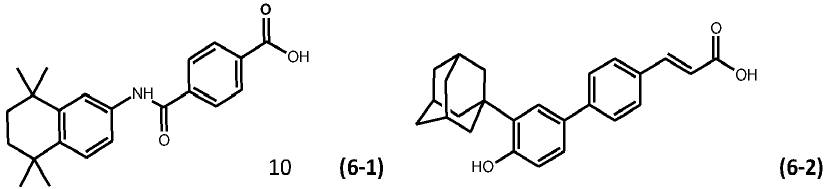

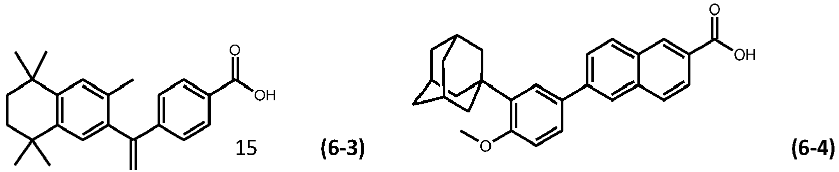

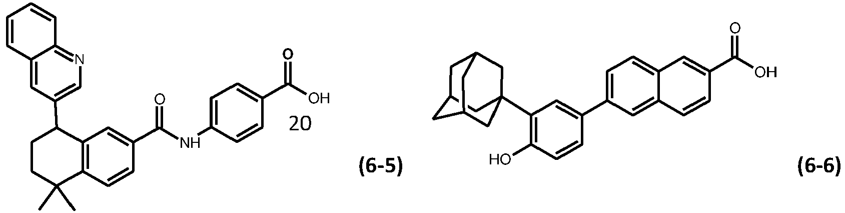

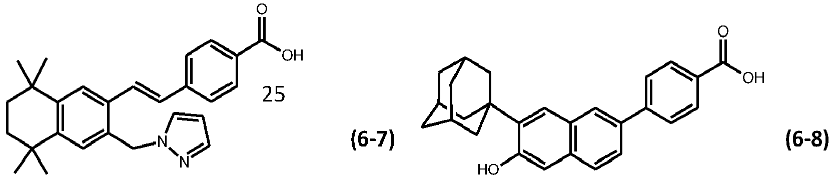

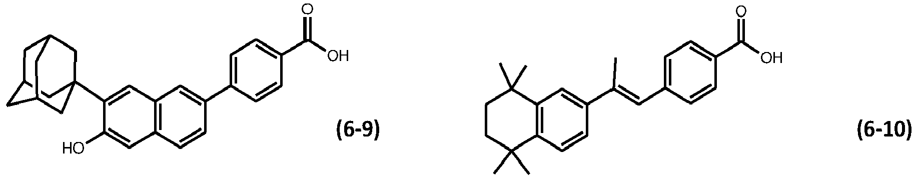

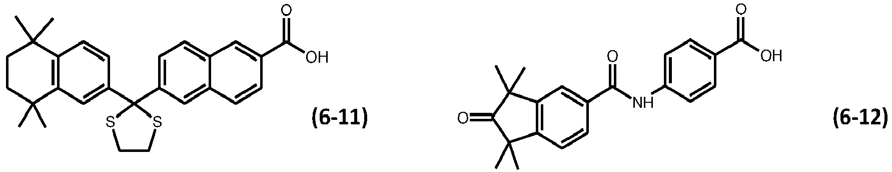

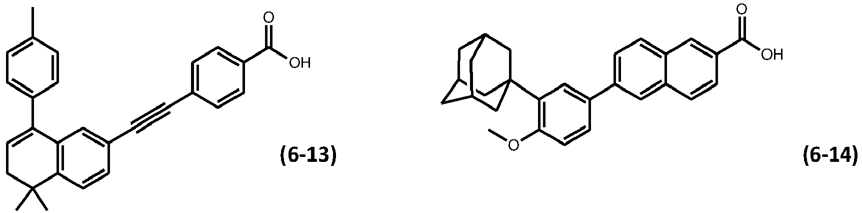

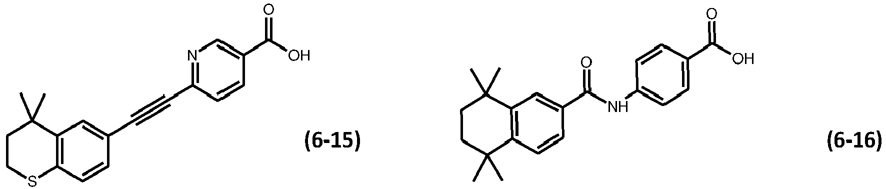

For the first time in literature, the present invention describes retinoates of harringtonines salts having a great therapeutic potential.

Myelopoiesis stimulating agents include
(i) Macrophage colony-stimulating factor (M-CSF)
(ii) Granulocyte colony-stimulating factor (G-CSF)
(iii) Granulocyte macrophage colony-stimulating factor (GM-CSF)
(iv) Megakaryocyte growth and development factor (MGDF)
(v) Erythropoiesis-stimulating agents (erythropoietine and related agents

(i) Macrophage colony-stimulating factor (M-CSF), is a secreted cytokine which causes hematopoietic stem cells to differentiate into macrophages or other related cell types. Eukaryotic cells also produce M-CSF in order to combat intercellular viral infection. It is one of the three experimentally described colony-stimulating factors. M-CSF binds to the colony stimulating factor 1 receptor. It may also be involved in development of the placenta
(ii) Granulocyte-colony stimulating factor (G-CSF or GCSF), also known as colony-stimulating factor 3 (CSF 3), is a glycoprotein that stimulates the bone marrow to produce granulocytes and stem cells and release them into the bloodstream. Functionally, it is a cytokine and hormone, a type of colony-stimulating factor, and is produced by a number of different tissues. G-CSF also stimulates the survival, proliferation, differentiation, and function of neutrophil precursors and mature neutrophils.
(iii) Granulocyte-macrophage colony-stimulating factor (GM-CSF), also known as colony-stimulating factor 2 (CSF2), is a monomeric glycoprotein secreted by macrophages, T cells, mast cells, natural killer cells, endothelial cells and fibroblasts that functions as a cytokine. Unlike granulocyte colony-stimulating factor, which specifically promotes neutrophil proliferation and maturation, GM-CSF affects more cell types, especially macrophages and eosinophils.
(iv) Megakaryocyte growth and development factor (MGDF) or Thrombopoietin (THPO) is glycoprotein hormone produced by the liver and kidney which regulates the production of platelets. i.e. Eltrombopag, an agonist of the c-mpl (TpoR) receptor, which is the target of the hormone thrombopoietin
(v) Erythropoiesis-stimulating agents (ESA) are drugs which stimulate the bone marrow to make red blood cells. [Information on Erythropoiesis-Stimulating Agents (ESA) i.e. Epoetin alfa (marketed as Procrit, Epogen), Darbepoetin alfa (marketed as Aranesp)". *www.fda.gov.* Retrieved 14 October 2017]

### Historic use of harringtonines and retinoids with myelopoiesis stimulating agent(s) Harringtonines (harringtoids)

HHT has a manageable extra-hematological toxicity, to the point that it can be used in the elderly man suffering from leukemia. HHT is also one of the rare anticancer drugs to lack mutagenic activity due to its lack of interaction with DNA. Also, very early on, medicine sought to compensate for the myelosuppressant effect of this product. At this point of reasoning, it should be noted that the use of stimulators of myelopoiesis antagonizes the antileukemic effect proper. As early as 1986 the clinical application of G-CSF in myeloid leukemia has been controversial, because it stimulates myeloid leukemia cells as well as normal granulocyte progenitors in vitro ["Recombinant human granulocyte colony-stimulating factor: Effects on normal and leukemic myeloid cells." Science 1986, p 232]. In the case of the use of HHT to treat diseases other than those affecting the hematopoietic organs such as for example viral diseases, an "immoderate" usage of hematopoietic stimulators can be carried out. In 1998, Chinese authors described the combined effect of G-CSF, DM-CSF or IL-3 with harringtonine, a biosynthetic congener of HHT ["Effects of hematopoietic growth factor on the anti-leukemia drugs to acute myeloid leukemia cells in vitro" Z. Lianfu et al. 29(1) p15 (1998)]. Regarding HHT itself, a number of papers described its combination with G-CSF. The article of Ustwani et al reviews this combination in AML therapy ["Homoharringtonine and granulocyte colony-stimulating factor priming for acute myeloid leukemia: is it ready for prime time?" Utswani et al. Leukemia and Lymphomia 2013 p 2100].

### Retinoids

Regarding the drug combination between retinoids and G-CSF, the situation is even better because most of retinoids synergizes myeloid expansion ["Retinoid agonist Am80-enhanced neutrophil bactericidal activity arising from granulopoiesis in vitro and in a neutropenic mouse model" W. Ding et al. Blood 2013 p 996; *"*Am80-GCSF synergizes myeloid expansion and differentiation to generate functional neutrophils that reduce neutropenia-associated infection and mortality" Li et al. Molecular Medecine 2016 p 1340]. It should be pointed out that certain retinoids have inhibitory as well as stimulatory effects on myelopoiesis ["All-trans retinoic acid directly inhibits granulocyte colony-stimulating factor-induced proliferation of CD34+ human hematopoietic progenitor cells" Smeland et al. Blood 84 1994 p 2940]. A study indicates that ATRA can be safely combined with G-CSF in patients with multiple myeloma and indolent lymphoma ["Safety and efficacy of combining ATRA with G-CSF in HSPC mobilization; a pilot study in multiple myeloma and non-Hodgkin's lymphoma patients" Herbert et al, Bone Marrow Transplantation 40 2007 p 801].

### Brief description of the invention

In a first aspect, the present invention relates to harringtonines retinoate salts, having the following formula (I): 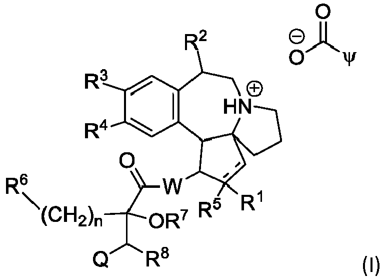 wherein
W represents O, S or NH,
   Q and R⁶ are each independently selected in the group consisting of:
   a linear or branched C₁-C₁₂ hydrocarbonated chain, said hydrocarbonated chain being optionally interrupted by one or more heteroatoms or groups selected among O, S, N or NH and optionally substituted with one or more groups selected among C₁-C₆ alkyl, OH, O-(C₁-C₆)-alkyl, oxo, halogen, an aryl and a heteroaryl,
   an aryl,
   a heteroaryl
   a (C₃-C₇)-cycloalkyl, and
   a heterocycle,
   R¹ is selected in the group consisting of H, OH, O-(C₁-C₁₂)-alkyl, O-aryl-(C₁-C₁₂)-alkyl, O-(C₂-C₁₂)-alkenyl and O-(C₃-C₇)-cycloalkyl, and
   R² is selected in the group consisting of H or OH, or
   R¹ and R² form together -O-,
R³ and R⁴ both represent an O-CH₃ group or R³ and R⁴ form together -OCH₂O-,
   R⁵ is selected in the group consisting of H, C₁-C₁₂ alkyl or O-(C₁-C₆)-alkyl,
   R⁷ is selected in the group consisting of H and (C₁-C₆)alkyl,
   or R⁶ and R⁷ form together -CMe₂-,
R⁸ is H, OH, C₁-C₁₂ alkyl or O-C₁-C₁₂ alkyl, 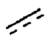 represents a single or a double bond,
   n is an integer between 0 and 8,
ψ is selected in the group consisting of the following formulas (A), (B) and (C): 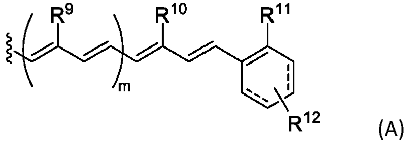 wherein:
   R⁹ to R¹¹ are each independently selected in the group consisting of
      a linear or branched C₁-C₁₂ hydrocarbonated chain, said hydrocarbonated chain being optionally interrupted by one or more heteroatoms or groups selected among O, S, N or NH and optionally substituted with one or more groups selected among C₁-C₆ alkyl, OH, O-(C₁-C₆)-alkyl, oxo, halogen, an aryl, a heteroaryl, a (C₃-C₇)-cycloalkyl and a heterocyclyl,
      an aryl,
      a heteroaryl
      a (C₃-C₇)-cycloalkyl, and
      a heterocyclyl,
   R¹² represent one or more substituents each independently selected in the group consisting of OH, halogen, (C₁-C₆)-alkyl, (C₂-C₆)-alkenyl, C₂-C₆-alkynyl and O-(C1-C6)-alkyl,
   m is an integer between 0 and 6 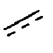
   represents a single or a double bond, 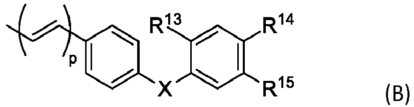
   wherein:
   X represents a single bond or a (C₁-C₆) hydrocarbonated chain, said hydrocarbonated chain being optionally interrupted by one or more heteroatoms or groups selected among O, S, N or NH and optionally substituted with one or more groups selected among C₁-C₆ alkyl, C₂-C₆ alkenyl, OH, O-(C₁-C₆)-alkyl, oxo and halogen,
   R¹³ is H or (C₁-C₆)alkyl, optionally substituted with a heteroaryl, and
      R¹⁴ and R¹⁵ are each independently selected in the group consisting of
      a linear or branched C₁-C₁₂ hydrocarbonated chain, said hydrocarbonated chain being optionally interrupted by one or more heteroatoms or groups selected among O, S, N or NH and optionally substituted with one or more groups selected among C₁-C₆ alkyl, OH, O-(C₁-C₆)-alkyl, oxo, halogen, an aryl, a heteroaryl, a (C₃-C₇)-cycloalkyl and a heterocyclyl,
      an OH group,
      an aryl,
      a heteroaryl
      a (C₃-C₇)-cycloalkyl, and
      a heterocyclyl, or
      R¹⁴ and R¹⁵ form together a carbocycle or a heterocycle optionally substituted with one or more groups selected among C₁-C₆ alkyl, OH, O-(C₁-C₆)-alkyl, oxo, halogen, an aryl, a heteroaryl, a (C₃-C₇)-cycloalkyl and a heterocyclyl,
   p is 0 or 1, 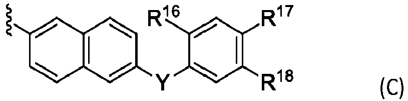 wherein
      Y represents a single bond or a (C₁-C₆) -hydrocarbonated chain, said hydrocarbonated chain being optionally interrupted by one or more heteroatoms or groups selected among O, S, N or NH and optionally substituted with one or more groups selected among C₁-C₆ alkyl, C₂-C₆ alkenyl, OH, O-(C₁-C₆)-alkyl, oxo, halogen, an aryl, a heteroaryl, a (C₃-C₇)-cycloalkyl or a heterocyclyl,
      R¹⁶ is H or (C₁-C₆)alkyl, and
         R¹⁷ and R¹⁸ are each independently selected in the group consisting of
         a linear or branched C₁-C₁₂ hydrocarbonated chain, said hydrocarbonated chain being optionally interrupted by one or more heteroatoms or groups selected among O, S, N or NH and optionally substituted with one or more groups selected among C₁-C₆ alkyl, OH, O-(C₁-C₆)-alkyl, oxo, halogen, an aryl, a heteroaryl, a (C₃-C₇)-cycloalkyl and a heterocyclyl,
         an OH group,
         an aryl,
         a heteroaryl
         a (C₃-C₇)-cycloalkyl, and
         a heterocyclyl, or
   R¹⁷ and R¹⁸ form together a carbocycle or a heterocycle optionally substituted with one or more groups selected among C₁-C₆ alkyl, OH, O-(C₁-C₆)-alkyl, oxo, halogen, an aryl, a heteroaryl, a (C₃-C₇)-cycloalkyl and a heterocyclyl.

In a second aspect, the present invention relates to a method of preparation of compounds of formula (I).

In a third aspect, the present invention relates to pharmaceutical composition comprising a compound of formula (I) and at least one pharmaceutically acceptable excipient.

According to a fourth aspect, the present invention also relates to a method for preventing or treating diseases cancer, leukemias, lymphomas, myelodysplastic syndrome, autoimmune diseases, skin diseases, neurological diseases such as Alzheimer's disease, inflammatory bowel disease, such as Crohn's disease, and viral infection comprising administering to a patient in need thereof an effective dose of the compound of formula (I) as described above or a pharmaceutical composition of the present invention.

A fifth aspect of the present invention relates to a method for preventing or treating viral infection comprising administering to a patient in need thereof an effective dose of a compound of the following formula (III): 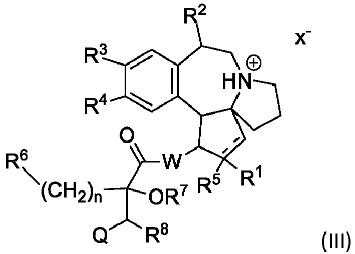
wherein Q, W and R¹ to R⁸ are as described above for compound of formula (I) and,
X⁻ is a mineral anion such as, but not limited to, hydrochorid, sulfate and phosphate or an organic carboxylate comprising 1 to 12 carbon atoms, preferably selected among fumarate, maleate, citrate, malate, tartrate, tartronate, succinate, itaconate, pamoate, citramalate, malonate, benzoate, benzenesulfonate, methanesulfonate, 1,5-naphtalene disulfonate, cyclohexanesulfamate, formate, lactate, mandelate, vanillate, oxaloacetate, glutarate, adipate, squarate, 3,4,5-trimethoxybenzoic, ascorbate and salicylate.

The present invention relates to a method for preventing or treating viral infection comprising administering to a patient in need thereof an effective dose of a pharmaceutical composition comprising a compound of formula (III) and at least one pharmaceutically acceptable excipient.

### Definitions:

The term "stereoisomers" used in this invention refers to configurational stereoisomers and more particularly to optical isomers.
In the present invention, the optical isomers result from the different position in space of substituents or lone pair of electrons on an atom (such as a carbon atom) comprising four different substituents (including potentially a lone pair of electrons). This atom thus represents a chiral or asymmetric centre. Optical isomers that are not mirror images of one another are thus designated as "diastereoisomers", and optical isomers, which are non-superimposable mirror images are designated as "enantiomers". An equimolar mixture of two enantiomers of a chiral compound is designated as a racemic mixture or racemate.
For the purpose of the invention, the term "pharmaceutically acceptable" is intended to mean what is useful to the preparation of a pharmaceutical composition, and what is generally safe and non-toxic, for a pharmaceutical use.

The term "halogen", as used in the present invention, refers to a fluorine, bromine, chlorine or iodine atom.
The term "(C₁-C₁₂)-hydrocarbonated chain", as used in the present invention, refers to a (C₁-C₁₂)-alkyl group, a (C₂-C₁₂)alkenyl group or a (C₂-C₁₂)-alkynyl group as defined below.
The term "(C₁-C₁₂)-alkyl", as used in the present invention, refer to a straight or branched monovalent saturated hydrocarbon chain containing respectively from 1 to 12 carbon atoms including, but not limited to, methyl, ethyl, n-propyl, iso-propyl, n-butyl, iso-butyl, sec-butyl, *t*-butyl, n-pentyl, n-hexyl, and the like. Preferably it is a C₁-C₆ alkyl.
The term "(C₂-C₁₂)-alkenyl", as used in the present invention, refers to a straight or branched monovalent unsaturated hydrocarbon chain containing from 2 to 12 carbon atoms and comprising at least one double bond including, but not limited to, ethenyl, propenyl, butenyl, pentenyl, hexenyl and the like. Preferably it is a C₁-C₆ alkenyl.
The term "(C₂-C₁₂)-alkynyl", as used in the present invention, refers to a straight or branched monovalent unsaturated hydrocarbon chain containing from 2 to 12 carbon atoms and comprising at least one triple bond including, but not limited to, ethynyl, propynyl, propynyl, butynyl, pentynyl, hexynyl and the like. Preferably it is a C₁-C₆ alkynyl.
The term "carbocycle" refers to a non-aromatic hydrocarbon ring, saturated or unsaturated, typically comprising from 3 to 20 carbons and comprising one or more fused or bridged ring(s). In particular, it is a saturated hydrocarbon cycle, especially a C₃-C₇ cycloalkyl, including, but not limited to, cyclopropyl, cyclobutyl, cyclopentyl, cyclohexyl, cycloheptyl, and the like.
The term "C₃-C₇ cycloalkyl" refers to a saturated hydrocarbon ring comprising from 3 to 7 carbons, including cyclopropyl, cyclobutyl, cyclopentyl, cyclohexyl and cycloheptyl. Preferably it is a C3-C7 cycloalkyl.
The term "heterocycle" as used in the present invention refers to a non-aromatic, saturated or unsaturated monocycle or polycycle (comprising fused, bridged or spiro rings) comprising preferably 3 to 12, notably 5 or 6, atoms in the ring(s), in which the atoms of the ring(s) consist of carbon atoms and one or more, advantageously 1 to 4, and more advantageously 1 or 2, heteroatoms, such as a nitrogen, oxygen or sulphur atom, the remainder being carbon atoms. A heterocycle can be notably piperidinyl, piperizinyl, pyrrolidinyl, pyrazolidinyl, imidazolidinyl, azepanyl, thiazolidinyl, isothiazolidinyl, oxazocanyl, thiazepanyl, benzimidazolonyl.
The term "aryl" refers to an aromatic hydrocarbon group preferably comprising from 6 to 12 carbon atoms and comprising one or more fused rings, such as, for example, a phenyl or naphthyl group. Advantageously, it is a phenyl group.
The term "heteroaryl", as used in the present invention, refers to an aromatic group comprising one or several, notably one or two, fused hydrocarbon cycles in which one or several, notably one to four, advantageously one or two, carbon atoms each have been replaced with a heteroatom selected from a sulfur atom, an oxygen atom and a nitrogen atom, preferably selected from an oxygen atom and a nitrogen atom. It can be a furyl, thienyl, pyrrolyl, pyridyl, oxazolyl, isoxazolyl, thiazolyle, isothiazolyl, imidazolyl, pyrazolyl, oxadiazolyl, thiadiazolyl, triazolyl, tetrazolyl, pyridazinyl, pyrimidinyl, pyrazinyl, triazinyl, quinolyl, isoquinolyl, quinoxalyl or indyl.

According to the present invention, the term patient refers to an animal, in particular to a mammal, such as a human being.

### Detailed description of the invention

### Salt of formula (I)

The present invention consists in forming salts, i.e. retinoates of harringtonines, between each of the two above classes of natural substances, namely harringtonines and retinoic acids, making them to react on contact either in solution, or neat (pure), by mixing the molten reactants.

The salts according to the present invention can be in the form of a stereoisomer or a mixture of stereoisomers, such as a mixture of enantiomers, notably a racemic mixture.

According to a particular embodiment, the salt of the invention may correspond to the following stereoisomer:

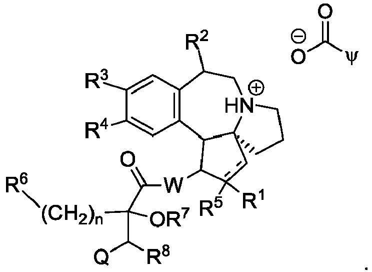

According to a preferred embodiment, in the salt of formula (I), W is an oxygen atom.

Advantageously, R² is a hydrogen atom.

According to a particular embodiment, R¹ and R² form together -O-.

According to a preferred embodiment, R³ and R⁴ form together a -OCH₂O- group.

According to another particular embodiment, R⁵ is also a hydrogen atom.

R⁸ is preferably also a hydrogen atom.

The salt of the invention preferably corresponds to a salt of the following formula (I'):

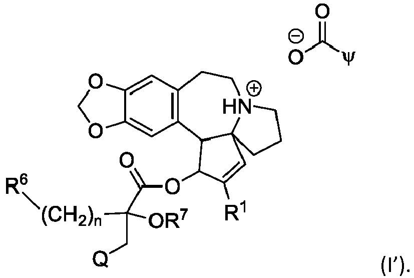

According to this previous embodiment, R¹ is preferably selected in the group consisting of H, OH and O-(C₁-C₁₂)-alkyl, in particular OH and O-(C₁-C₁₂)-alkyl. R¹ is notably O-(C₁-C₁₂)-alkyl, and in particular an O-CH₃ group.

Advantageously, in salt of formula (I'), Q is a linear or branched (C₁-C₁₂)-hydrocarbonated chain, said hydrocarbonated chain being optionally interrupted by one or more heteroatoms or groups selected among O, S, N or NH and optionally substituted with one or more groups selected among C₁-C₆ alkyl, OH, O-(C₁-C₆)-alkyl, oxo, halogen, an aryl and a heteroaryl.
In particular, Q represents a linear or branched C₁-C₁₂ hydrocarbonated chain, said hydrocarbonated chain being optionally interrupted by one or more oxygen atoms and optionally substituted with one or more groups selected among C₁-C₆ alkyl, OH, O-(C₁-C₆)-alkyl, oxo and halogen, in particular oxo. Preferably, Q represents an ester having the following formula -C(=O)-O-(C₁-C₆)-alkyl or -C(=O)-O-(C₁-C₆)-alkene. More preferably, Q is of formula -C(=O)-O-CH₃ or -C(=O)-O-CH₂-CH=CH-CH=CH-CH₃.

According to a particular embodiment, in salt of formula (I'), R⁶ is a linear or branched C₁-C₁₂ hydrocarbonated chain, said hydrocarbonated chain being optionally interrupted by one or more heteroatoms or groups selected among O, S, N or NH and optionally substituted with one or more groups selected among C₁-C₆ alkyl, OH, O-(C₁-C₆)-alkyl, oxo, halogen, an aryl and a heteroaryl.
In particular, R⁶ represents a linear or branched C₁-C₁₂ hydrocarbonated chain, said hydrocarbonated chain being optionally interrupted by one or more oxygen atoms and optionally substituted with one or more groups selected among C₁-C₆ alkyl, OH, O-(C₁-C₆)-alkyl, oxo, halogen and aryl. Preferably, R⁶ represents a (C₁-C₆) alkyl, advantageously substituted with one or more groups selected among OH, O-(C₁-C₆)-alkyl, oxo, halogen and aryl. More preferably, R⁶ is a (C₁-C₆) alkyl, in particular a branched (C₁-C₆) alkyl advantageously substituted with one group selected among OH and aryl, in particular substituted with OH.

In salt of formula (I'), R⁷ is notably a hydrogen atom.
n is preferably an integer between 1 and 4, notably 2 or 3.

According to another particular embodiment, R⁶ and R⁷ form together -C(Me)₂-. According to this previous embodiment, n is equal to 2 or 3, in particular 3.

The cationic part of the salt of formula (I) is preferably selected in the group consisting of:

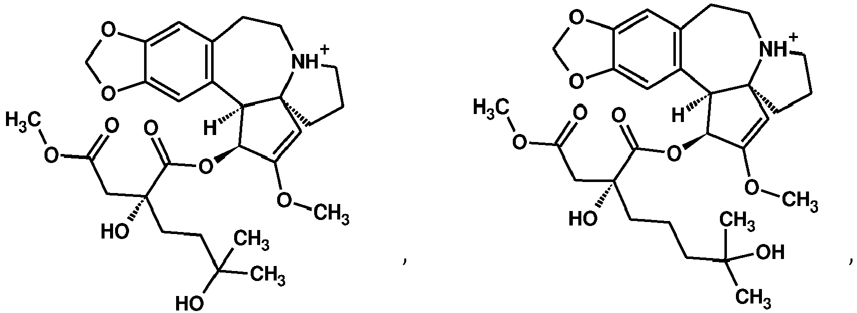

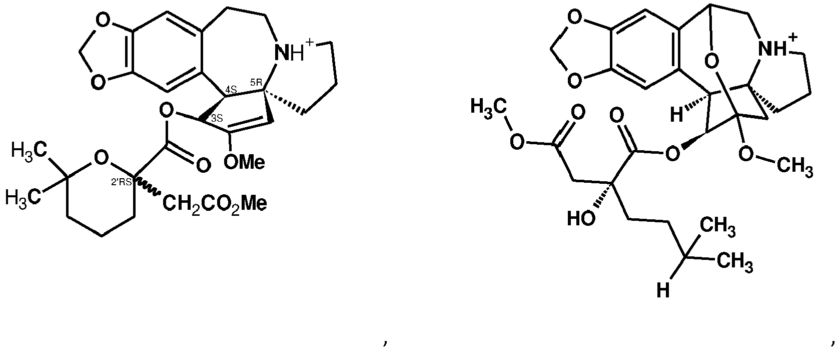

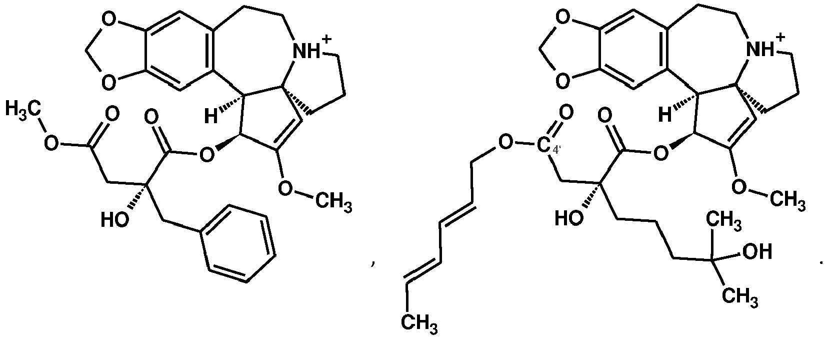

According to a particular embodiment, ψ is of formula (A): 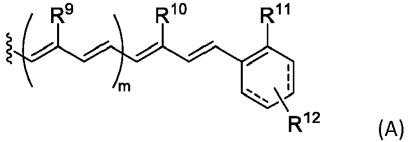 Preferably, R⁹ to R¹¹ are each a linear or branched C₁-C₁₂ hydrocarbonated chain, said hydrocarbonated chain being optionally interrupted by one or more heteroatoms or groups selected among O, S, N or NH and optionally substituted with one or more groups selected among C₁-C₆ alkyl, OH, O-(C₁-C₆)-alkyl, oxo, halogen, an aryl, a heteroaryl, a (C₃-C₇)-cycloalkyl and a heterocyclyl. In particular, R⁹ to R¹¹ each independently represent a (C₁-C₆)-alkyl, in particular a methyl.

R¹² advantageously represents one or more substituents each independently selected in the group consisting of OH, (C₁-C₆)-alkyl and O-(C₁-C₆)-alkyl. Particularly, R¹² represents one or more substituents, notably two or three, each independently selected in the group consisting of (C₁-C₆)-alkyl and O-(C₁-C₆)-alkyl.

m is typically an integer between 1 and 3, preferably m is 1.

According to another particular embodiment, ψ is of formula (B): 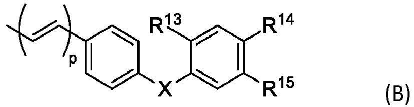 X represents advantageously a single bond or a (C₁-C₆) hydrocarbonated chain, said hydrocarbonated chain being optionally interrupted by one or more heteroatoms or groups selected among O, S, N or NH and optionally substituted with one or more groups selected among C₁-C₆ alkyl and C₂-C₆ alkenyl. Preferably X represents a single bond, a -NH-C(=O) group or a (C₁-C₆) hydrocarbonated chain said hydrocarbonated chain being optionally substituted with one or more groups selected among C₁-C₆ alkyl and C₂-C₆ alkenyl. In particular, said hydrocarbon chain is (C₁-C₆) alkyl, (C₂-C₆)-alkene or (C₂-C₆)-alkyne, optionally substituted with one or more groups selected among C₁-C₆ alkyl and C₂-C₆ alkenyl.

R¹³ is preferably H or a methyl. When R¹³ is a methyl, it is optionally substituted with a heteroaryl, in particular with a pyrazolyl group.
R¹⁴ and R¹⁵ are preferably each independently selected in the group consisting of an OH group, a O-(C₁-C₆ alkyl), such as a methoxy group, and a (C₃-C₇)-cycloalkyl. The (C₃-C₇)-cycloalkyl is in particular an adamantyl group of the following formula: 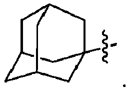 According to another embodiment, R¹⁴ and R¹⁵ form together a carbocycle or a heterocycle, optionally substituted with one or more groups selected among C₁-C₆ alkyl, oxo, an aryl and a heteroaryl, such as quinoline.
According to another particular embodiment, ψ is of formula (C): 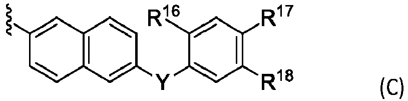 Y advantageously represents a single bond or a (C₁-C₆)-alkyl. When Y is a (C₁-C₆)-alkyl, it is preferably substituted with one group selected among C₁-C₆ alkyl, oxo, an aryl or a heterocyclyl, in particular a heterocyclyl, such as dithiolane.

R¹⁶ is notably H.

R¹⁷ and R¹⁸ are preferably each independently selected in the group consisting of an OH group, a O-(C₁-C₆ alkyl), such as a methoxy group, and a (C₃-C₇)-cycloalkyl. The (C₃-C₇)-cycloalkyl is in particular an adamantyl group of the following formula: 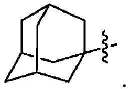 According to another embodiment, R¹⁷ and R¹⁸ form together a carbocycle or a heterocycle, optionally substituted with one or more groups selected among C₁-C₆ alkyl, oxo, an aryl and a heteroaryl, such as quinoline.
R¹⁷ and R¹⁸ form together a carbocycle, advantageously substituted with one or more, in particular two or four, C₁-C₆ alkyl, such as methyl.

The anionic part of the salt of formula (I) is preferably selected in the group consisting of:

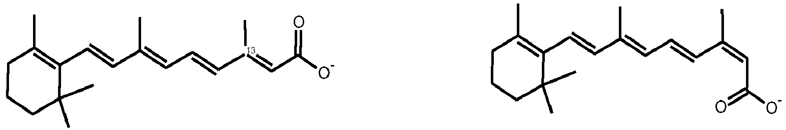

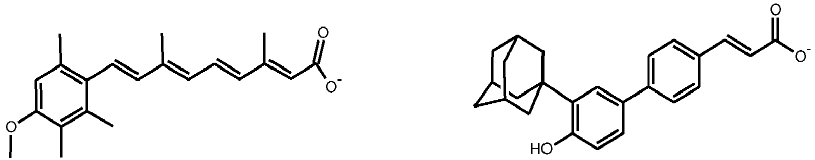

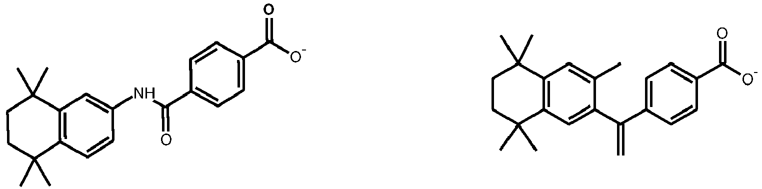

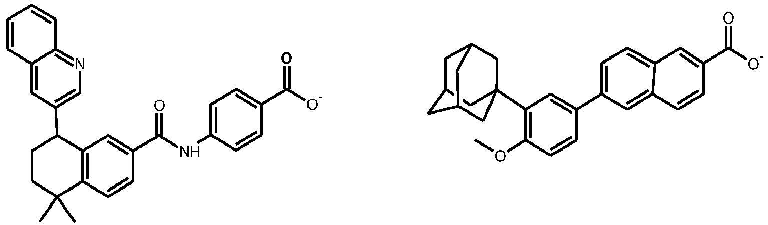

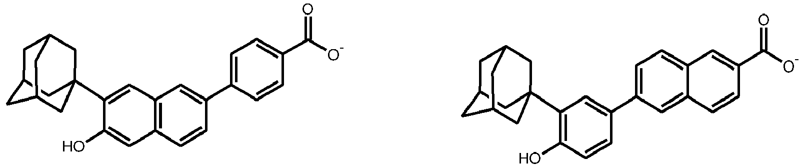

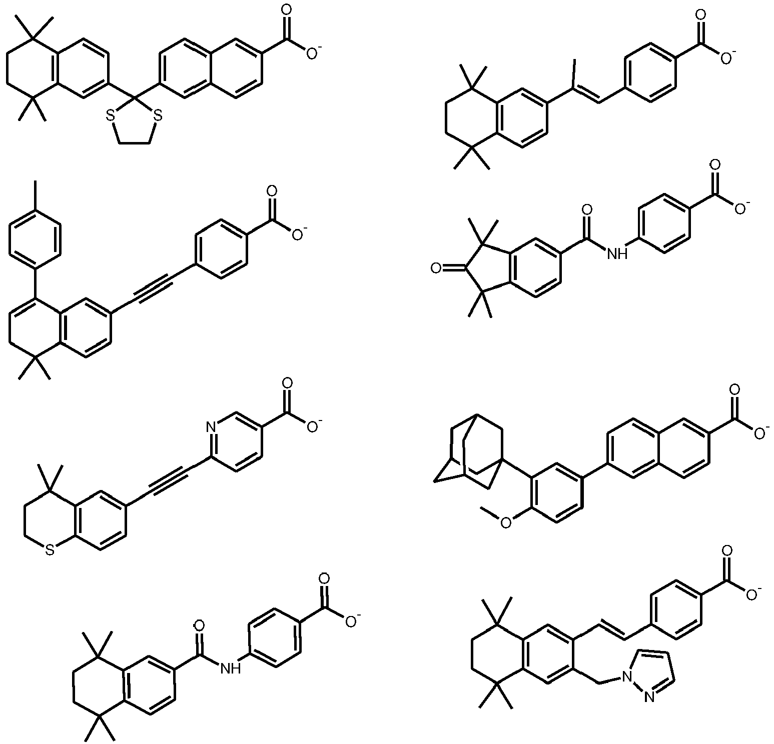

According to a preferred embodiment, the salt of formula (I) is selected among:

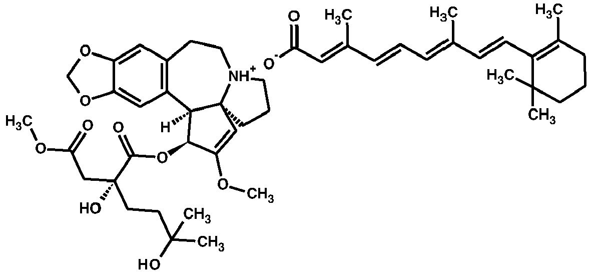

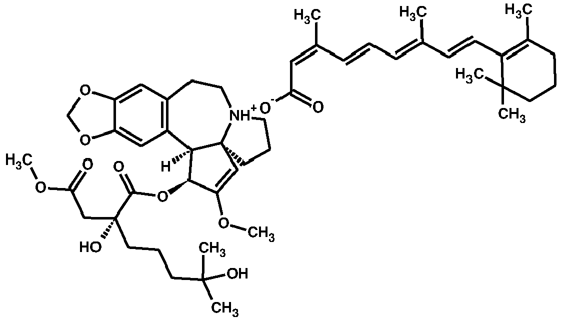

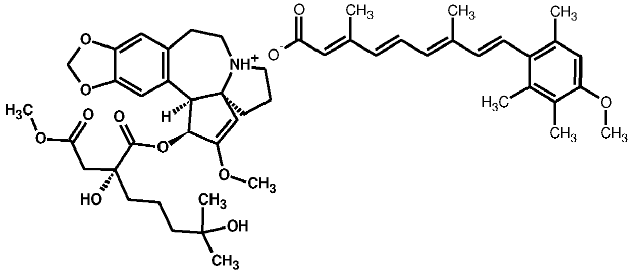

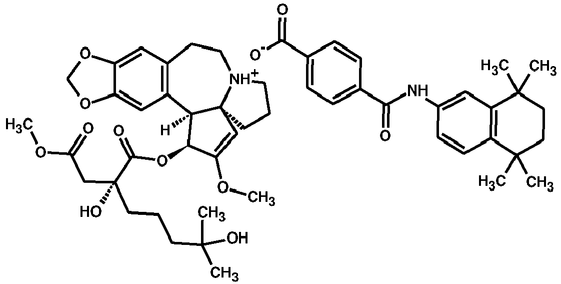

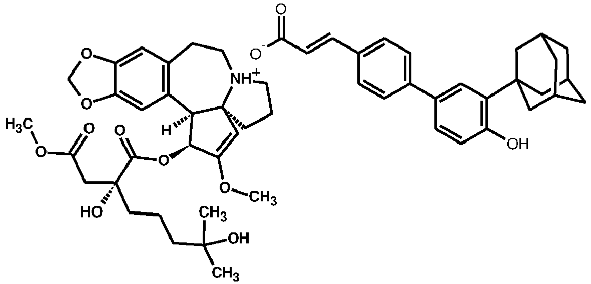

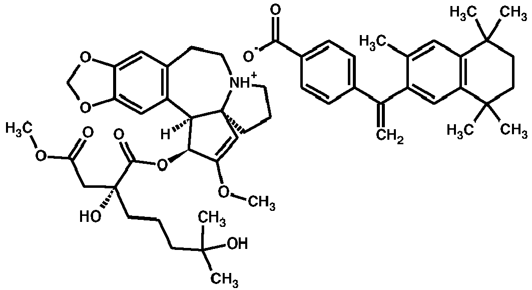

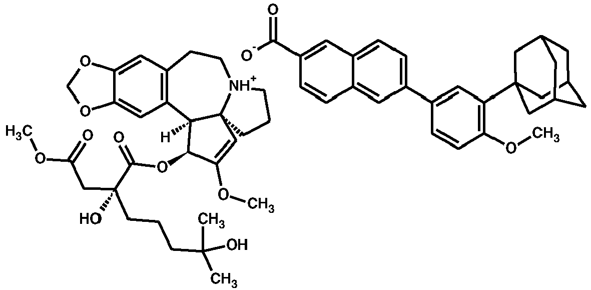

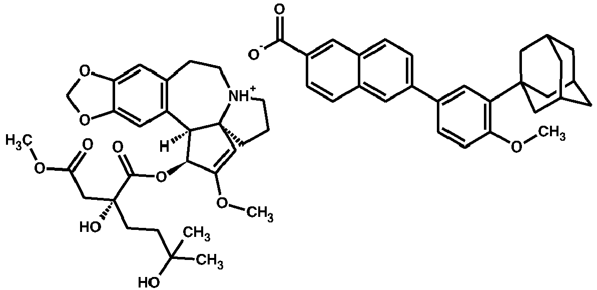

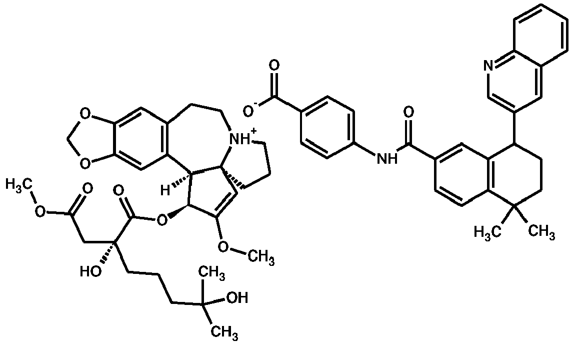

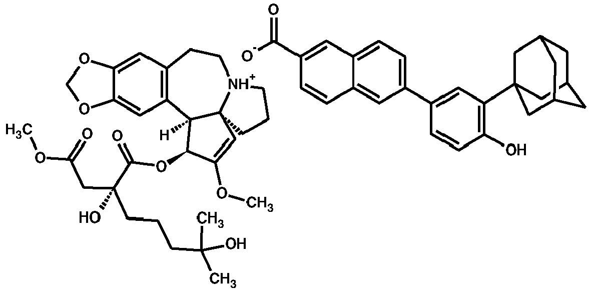

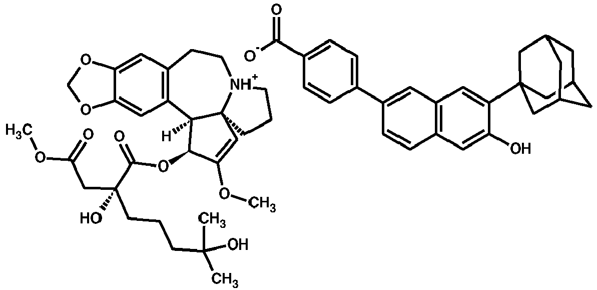

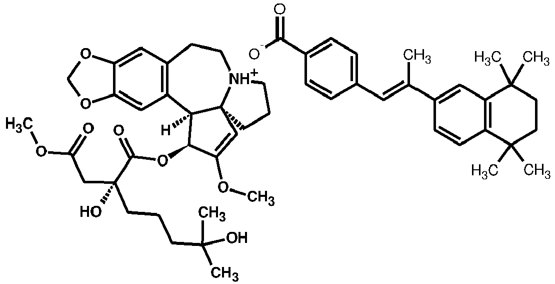

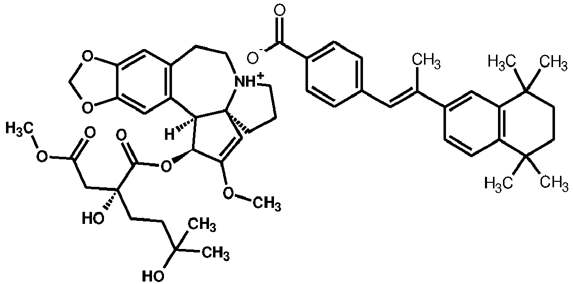

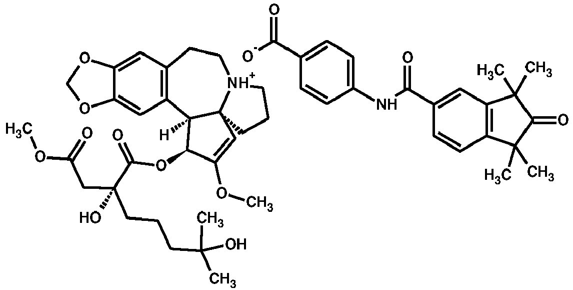

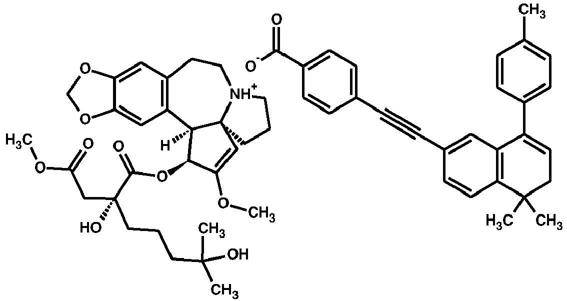

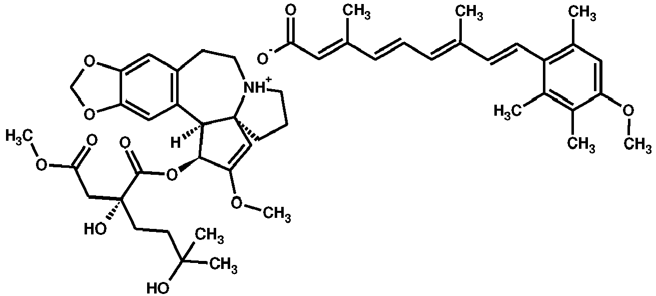

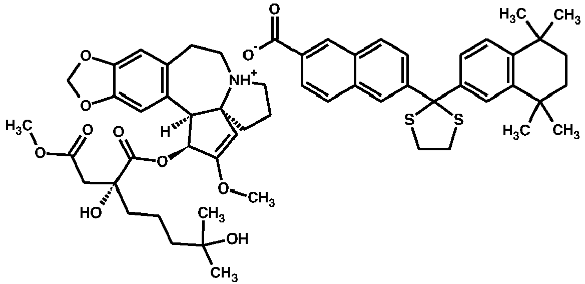

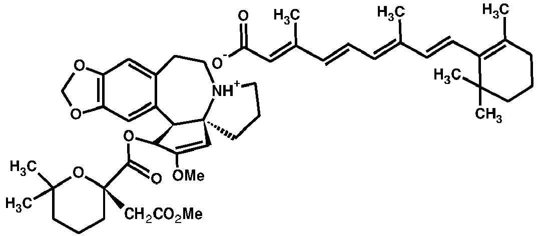

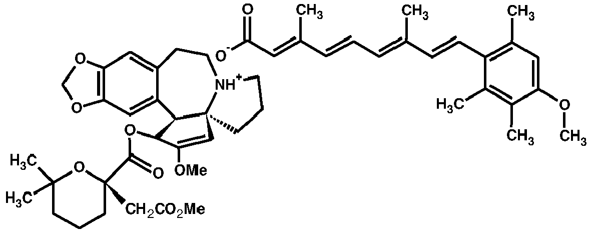

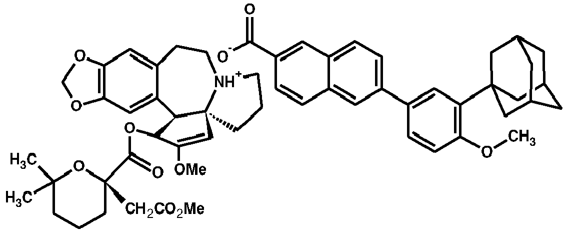

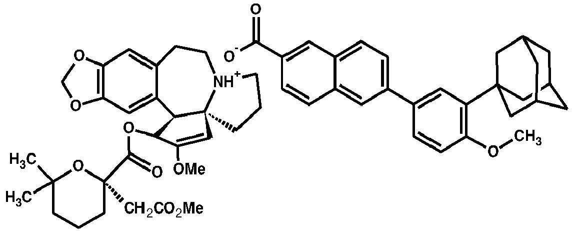

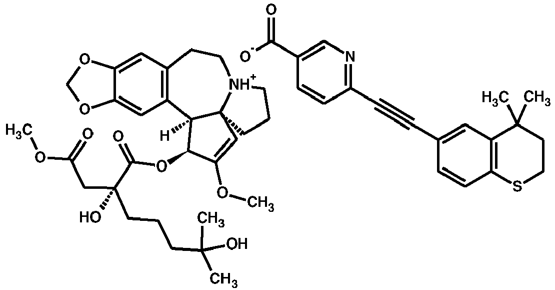

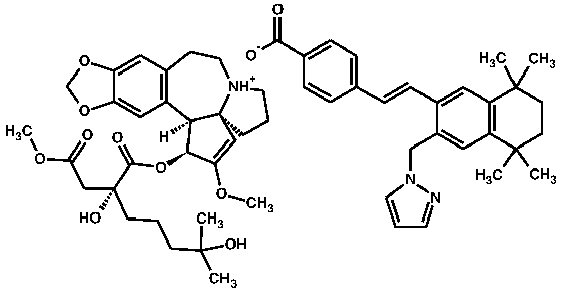

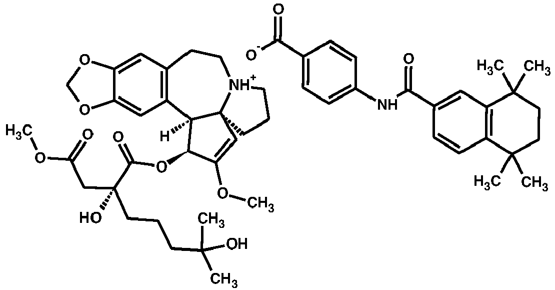

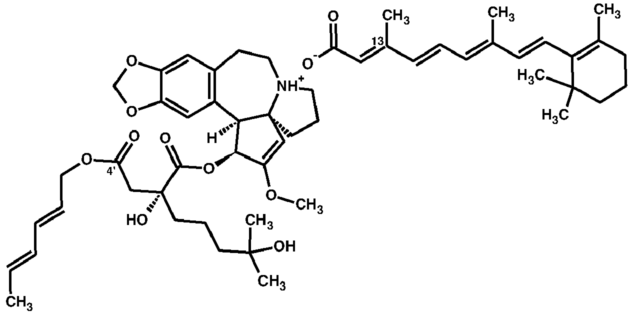

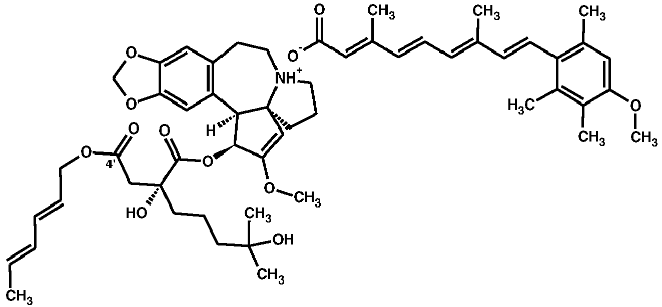

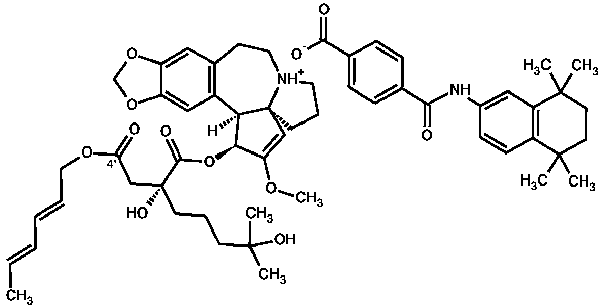

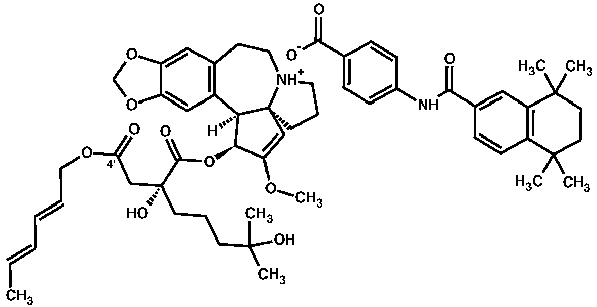

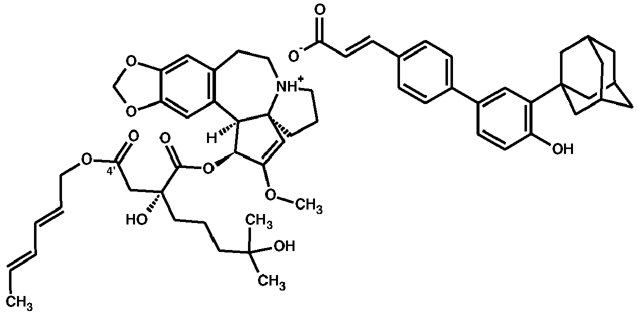

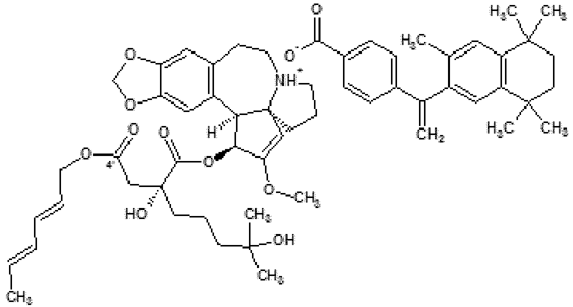

### Pharmaceutical composition

The present invention also relates to a pharmaceutical composition comprising at least one salt of formula (I), and at least one pharmaceutically acceptable excipient. Said pharmaceutically acceptable excipient can be an active substance or an inactive substance.

In particular, the pharmaceutical composition according to the present invention comprises at least one salt of formula (I) in combination with one harringtonine derivative corresponding to the cationic part of said salt of formula (I).

According to another embodiment, the pharmaceutical composition according to the present invention comprises at least one salt of formula (I) in combination with one retinoid derivative corresponding to the anionic part of said salt of formula (I).

According to another embodiment, the pharmaceutical composition according to the present invention comprises at least one salt of formula (I) in combination with one retinoid derivative corresponding to the anionic part of said salt of formula (I) and one harringtonine derivative corresponding to the cationic part of said salt of formula (I).

According to another embodiment, the pharmaceutical composition according to the present invention comprises at least one salt of formula (I) and one or more myelopoiesis stimulating agent such as (i) macrophage colony-stimulating factor (M-CSF) and/or granulocyte colony-stimulating factor (G-CSF) and/or granulocyte macrophage colony-stimulating factor (GM-CSF) and/or megakaryocyte growth and development factor (MGDF) and/or erythropoiesis-stimulating-agents (ESA) such as, but not limited to recombinant human erythropoietin (rhEPO), these stimulating agents being administrated simulateneously, sequentially, iteratively, preferably before hand.
According to another embodiment, the pharmaceutical composition according to the present invention comprises at least one salt of formula (I) in combination with one retinoid derivative corresponding to the anionic part of the salt of formula (I) and /or one harringtonine corresponding to the cationic part of the salt of formula (I), preferably in combination with at least one myelopoiesis stimulating agent such as (i) macrophage colony-stimulating factor (M-CSF) and/or granulocyte colony-stimulating factor (G-CSF) and/or granulocyte macrophage colony-stimulating factor (GM-CSF) and/or megakaryocyte growth and development factor (MGDF) and/or erythropoiesis-stimulating-agents (ESA) such as, but not limited to recombinant human erythropoietin (rhEPO), these stimulating agents being administrated simultaneously, sequentially, iteratively, preferably beforehand. Advantageously, in the previous embodiment, when present, the harringtonine is in molar excess.

Advantageously, the pharmaceutical composition according to the present invention comprises at least one salt of formula (I) and a chemotherapeutic agent. For example, a chemotherapeutic agent may be selected among tyrosine kinase inhibitors.

Advantageously, the pharmaceutical composition according to the present invention comprises at least one salt of formula (I) and an immunomodulating agent.
The pharmaceutical compositions of the invention can be intended to oral, rectal, topical or parenteral (e.g. subcutaneous, nasal, epidural, intramuscular, intravenous, intraarterial, intraperitoneal, intrathecal, transcutaneous) administration, preferably oral or intravenous administration. The active ingredient can be administered in unit forms for administration, mixed with conventional pharmaceutical carriers, to animals, preferably mammals including humans.
For oral administration, the pharmaceutical composition can be in a solid or liquid (solution or suspension) form.
A solid composition can be in the form of tablets, gelatin capsules, powders, granules and the like. In tablets, the active ingredient can be mixed with pharmaceutical vehicle(s) such as gelatin, starch, lactose, magnesium stearate, talc, gum arabic and the like before being compressed. The tablets may be further coated, notably with sucrose or with other suitable materials, or they may be treated in such a way that they have a prolonged or delayed activity. In powders or granules, the active ingredient can be mixed or granulated with dispersing agents, wetting agents or suspending agents and with flavor correctors or sweeteners. In gelatin capsules, the active ingredient can be introduced into soft or hard gelatin capsules in the form of a powder or granules such as mentioned previously or in the form of a liquid composition such as mentioned below.
A liquid composition can contain the active ingredient together with a sweetener, a taste enhancer or a suitable coloring agent in a solvent such as water. The liquid composition can also be obtained by suspending or dissolving a powder or granules, as mentioned above, in a liquid such as water, juice, milk, etc. It can be for example a syrup or an elixir.
For parenteral administration, the composition can be in the form of an aqueous suspension or solution which may contain suspending agents and/or wetting agents. The composition is advantageously sterile. It can be in the form of an isotonic solution (in particular in comparison to blood).

The compounds of the invention can be used in a pharmaceutical composition at a dose ranging from 0.01 mg to 1000 mg a day, administered in only one dose once a day or in several doses along the day, for example twice a day in equal doses. The daily administered dose is advantageously comprised between 5 mg and 500 mg, and more advantageously between 10 mg and 200 mg. However, it can be necessary to use doses out of these ranges, which could be noticed by the person skilled in the art.

### Treatment

The present invention relates to a compound of formula (I) according to the invention, or a pharmaceutical composition according to the present invention for use as a drug, in particular in the treatment of diseases selected among cancer, leukemias, lymphomas, myelodysplastic syndrome, autoimmune diseases, skin diseases, neurological diseases, inflammatory bowel disease, such as Crohn's disease, and viral infections including papillomavirus.

The present invention also relates to the use of a compound of formula (I) according to the invention or a pharmaceutical composition according to the present invention for the manufacture of a drug, notably intended in the treatment of diseases selected among cancer, leukemias, lymphomas, myelodysplastic syndrome, autoimmune diseases, skin diseases, neurological diseases, inflammatory bowel disease, such as Crohn's disease, and viral infections including papillomavirus.

The present invention also relates to the use of a compound of formula (I) according to the invention or a pharmaceutical composition according to the present invention for the treatment of diseases selected among cancers, leukemias, lymphomas, myelodysplastic syndrome, autoimmune diseases, skin diseases, neurological diseases, inflammatory bowel disease, such as Crohn's disease, and viral infections including papillomavirus.

The present invention also relates to a method for preventing or treating diseases cancer, leukemias, lymphomas, myelodysplastic syndrome, autoimmune diseases, skin diseases, neurological diseases such as Alzheimer's disease, inflammatory bowel disease, such as Crohn's disease, and viral infection comprising administering to a patient in need thereof an effective dose of the compound of formula (I) as described above or a pharmaceutical composition of the present invention.

The present invention also relates to the use of a compound of formula (III) according to the invention or a pharmaceutical composition comprising thereof for the treatment of viral infections.

The present invention also relates to a method for preventing or treating viral infection comprising administering to a patient in need thereof an effective dose of a compound of the following formula (III): 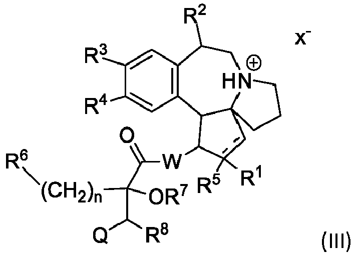
wherein Q, W and R¹ to R⁸ are as described above for compound of formula (I) and,
X⁻ is a mineral anion such as, but not limited to, hydrochorid, sulfate and phosphate or an organic carboxylate comprising 1 to 12 carbon atoms, preferably selected among fumarate, maleate, citrate, malate, tartrate, tartronate, succinate, itaconate, pamoate, citramalate, malonate, benzoate, benzenesulfonate, methanesulfonate, 1,5-naphtalene disulfonate, cyclohexanesulfamate, formate, lactate, mandelate, vanillate, oxaloacetate, glutarate, adipate, squarate, 3,4,5-trimethoxybenzoic, ascorbate and salicylate.

In formula (III), W, Q and R₁ to R₈ preferably correspond to the preferred embodiments described above for compound of formula (I).

According to a particular embodiment, cancer may be selected among lung cancer such as non small cells lung carcinoma (NSCLC) and eventually their metastasis, colon cancer, prostate cancer, osteocarcinoma, skin cancer, including melanoma, brain cancer, breast cancer and cervical cancer.

In particular, the cancer is breast cancer, lung cancer or brain cancer. Notably, the breast cancer is a triple negative breast cancer (TNBC), HER2 positive cancer, ER positive breast cancer, PR positive breast cancer or inflammatory breast cancer. Brain cancer is in particular a neuroblastoma, a glioblastoma, a medulloblastoma, a lower-grade astrocytoma.

Examples of autoimmune diseases are multiple sclerosis, psoriasis and atopic dermatidis, rheumatoid arthritis, dermatomyositis, Hashimoto's thyroiditis or systemic lupus erythematosus (SLE).

According to a particular embodiment, leukemias are chronic myeloid leukemia (CML), acute lymphoblastic leukemias (ALL), chronic lymphocytic leukemias (CLL), every acute myeloid leukemia (AML) including, but not limited to, acute promyelocytic leukemia (APL), myeloproliferative neoplasms, including but not limited to polycythemia Vera (PV), essential thrombocytemia and myelofibrosis, myelodysplastic syndrome and related diseases.

Lymphomas are for example cutaneous T-cell lymphoma.
Neurological diseases are for example Alzheimer's disease.

In particular, viral infection may be selected among papillomavirus, non human coronoviridae, porcine epidemic diarrhea virus (PEDV) (which is a coronaviridae), vesicular stomatitis virus (VSV), Newcastle disease virus (NDV), herpes virus HSV-1, pseudorabies virus (PRV), avian influenza virus (AIV), chikungunya virus (CHIKV), hepatitis B virus (HBV), bovine viral diarrhoea virus (BVDV), varicella-zoster virus (VZV), foot and mouth disease virus (FMDV), hepatitis C virus (HCV), retrovirus murine leukemia virus, (MuLV), transmissible gastroenteritis virus (TGEV), porcine reproductive and respiratory syndrome virus (PRRSV).

The present invention advantageously relates to a method for preventing or treating disease selected among cancer, leukemias, lymphomas, myelodysplastic syndromes autoimmune diseases, skin diseases, neurological diseases, inflammatory bowel disease, namely Crohn's disease and virus infection including but not limited to papillomavirus comprising administering an effective dose of a salt of formula (I) and an additional effective dose of a harringtonine derivative corresponding to the cationic part of said salt to a patient in need thereof, the harringtonine derivative being optionally administered simultaneously, separately or/and sequentially over time.

The present invention advantageously relates to a method for preventing or treating disease selected among cancer, leukemias, lymphomas, myelodysplatic syndrome, autoimmune diseases, skin diseases, neurological diseases, inflammatory bowel disease, namely Crohn's disease, and virus infection including but not limited to papillomavirus comprising administering an effective dose of a salt of formula (I) and an additional effective dose of a retinoid derivative corresponding to the anionic part of said salt to a patient in need thereof, the retinoid derivative being optionally administered simultaneously, separately or/and sequentially over time.

Typically, the present invention relates to a method for treating cancers such as solid tumors or sarcomas, particularly, but not limited to brain cancer such as for example neuroblastomas eventually their metastasis, lung cancer such as for example non small cells lung carcinoma eventually their metastasis, said method comprising administering to a patient in need thereof a salt of formula (I) alone or combined with another chemotherapeutic agent including agent for targeted therapy and/or with irradiation.

In addition the present invention relates to a method for treating leukemias, in particular, acute leukemias and miscellaneous myeloproliferative neoplasm (MPNs), such as chronic myeloid leukemias, essential thrombocythemia (ET), polycythemia vera (PV) myelofibrosis, and acute and chronic lymphoid leukemias, lymphoma, myelomas, myelodysplastic syndromes and related diseases, said method comprising administering to a patient in need thereof a salt of formula (I) alone or combined with another chemotherapeutic agent including agent for targeted therapy and/or with irradiation.

Typically, the present invention relates to a method for treating autoimmune diseases, such as for example but not limited to multiple sclerosis (MS), psoriasis and atopic dermatitis, rheumatoid arthritis, dermatomyositis, Hashimoto's thyroiditis or systemic lupus erythematosus, said method comprising administering to a patient in need thereof a salt of formula (I) alone or combined with another chemotherapeutic agent or immunomodulating agent including agent for targeted therapy and/or with irradiation.

Particularly, the present invention relates to a method for conditioning regimen for bone marrow transplantation, said method comprising administering to a patient in need thereof a salt of formula (I) alone or combined with another chemotherapeutic agent including agent for targeted therapy and/or with irradiation.

### Method of preparation

The present invention relates to a method of preparation of salt of formula (I), said method comprising the following steps:
(a) mixing the harringtonine of the following formula (II): 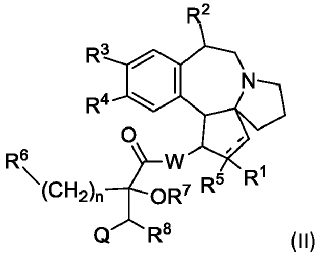 with a carboxylic acid of formula ψ-COOH,
(b) bringing the mixture resulting from step (a) to the liquid state and heating it,
(c) monitoring the kinetic of the reaction using a spectrometric method to control the disappearance of the acid function of ψ-COOH,
(d) removing the solvent and drying the resulting solid under vacuum to afford crystal salt of formula (I).

The harringtonine of formula (II) is commercially available.

The liquid state of step (b) can be induced by heating the mixture of step (a) being in a solid state at a temperature ranged from 50°C to 250 °C, without solvent.

According to another embodiment, the liquid state of step (b) can be induced by dissolving the mixture resulting from step (a), being in a solid state, in a polar solvent, such as an alcohol, water, an ether, for example tetrahydrofuran or its deuterated version, or their mixture.

During step (b), the temperature is typically comprised between 50 and 280°C.

Optionally, the harringtonine of formula (II) and the carboxylic acid are introduced in non-stoichiometric quantity.

The kinetic of the reaction is typically monitored by infrared spectrometry, in particular Fourrier transform infrared spectrometry, by controlling the disappearance of the absorbance band of the C=O of the acid function stretching resonance, in a range between 1670 to 1700 cm⁻¹. Said monitoring is achieved *in situ.*

The salt obtained can be separated from the reaction medium by methods well known to the person skilled in the art, such as by extraction, evaporation of the solvent or by precipitation or crystallisation (followed by filtration).
The compound can be also purified if necessary, by methods well known to the person skilled in the art, such as by recrystallisation, by distillation, by chromatography on a column of silica gel or by preparative high performance liquid chromatography (HPLC).

### Description of the figures:

Figure 1a: IR ATR diamond spectrum of the HHT derivative used for the preparation of the salt of example 1, in solid state.
Figure 1b: IR ATR diamond spectrum of the HHT derivative used for the preparation of the salt of example 1, film from methanol solution.
Figure 1c: IR ATR diamond spectrum of all-*trans*-retinoic acid used for the preparation of the salt of example 1, film from tetrahydrofuran (THF) solution.
Figure 1d: IR ATR diamond spectrum of the salt of example 1, film from THF solution.
Figure 2a: IR ATR diamond spectrum of the HHT derivative used for the preparation of the salt of example 2, film from THF solution.
Figure 2b: IR ATR diamond spectrum of the salt of example 2, film from THF solution.
Figure 3a: IR ATR diamond spectrum of 13-*cis*-retinoic acid (13-cRA) used for the preparation of the salt of example 3 in solid state.
Figure 3b: IR ATR diamond spectrum of the salt of example 3, in solid state.
Figure 4a: IR ATR diamond spectrum of 2^{nd} generation retinoid acitretin, (TMPP), used for the preparation of the salt of example 4, film from THF.
Figure 4b: Homoharringtonine acitretinate, film from THF IR ATR diamond spectrum Reaction time 10 minutes.
Figure 4c: Homoharringtonine acitretinate, film from THF IR ATR diamond spectrum Reaction time 75 min.
Figure 4d: IR ATR diamond spectrum of the salt of example 4, prisms from THF.
Figure 5a: IR ATR diamond spectrum of 3^{rd} generation retinoid tamibarotene (AM80), used for the preparation of the salt of example 5, film.
Figure 5b: IR ATR diamond spectrum of the salt of example 5, film.
Figure 6a: IR ATR diamond spectrum of 3^{rd} generation retinoid adarotene (ST 1926), used for the preparation of the salt of example 6, film.
Figure 6b:IR ATR diamond spectrum of the salt of example 6, film.
Figure 7a: IR ATR diamond spectrum of 3^{rd} generation retinoid bexarotene (LG69), used for the preparation of the salt of example 7, film from MeOH.
Figure 7b: IR ATR diamond spectrum of 3^{rd} generation retinoid bexarotene (LG69), used for the preparation of the salt of example 7, film from THF.
Figure 7c: IR ATR diamond spectrum of the salt of example 7, film.
Figure 8a: IR ATR diamond spectrum of 3^{rd} generation retinoid adapalene (CD 271), used for the preparation of the salt of example 8, film.
Figure 8b: IR ATR diamond spectrum of the salt of example 8, film from THF.
Figure 9a: IR ATR diamond spectrum of 3^{nd} generation retinoid adapalene, used for the preparation of the salt of example 9, film from THF.
Figure 9b: IR ATR diamond spectrum of the salt of example 9, film from THF.
Figure 10a: IR ATR diamond spectrum of 3^{rd} generation retinoid BMS 195'614, used for the preparation of the salt of example 10, film from THF.
Figure 10b:IR ATR diamond spectrum of the salt of example 10, film from THF.
Figure 11a: IR ATR diamond spectrum of 3^{rd} generation retinoid CD 437 (AHPN), used for the preparation of the salt of example 11, film from THF.
Figure 11b: IR ATR diamond spectrum of the salt of example 11, film from THF.
Figure 12a: IR ATR diamond spectrum of 3^{rd} generation retinoid CD 1530, used for the preparation of the salt of example 12, film from THF.
Figure 12b: IR ATR diamond spectrum of the salt of example 12, film from THF.
Figure 13a: IR ATR diamond spectrum of 3^{rd} generation retinoid arotinoid acid (TTNPB, AGN 191183, Ro 13-7410), used for the preparation of the salt of example 13, film.
Figure 13b: IR ATR diamond spectrum of the salt of example 13, film from THF.
Figure 13c: IR ATR diamond spectrum of the salt of example 13, crystal from THF.
Figure 14: IR ATR diamond spectrum of the salt of example 14, film from THF.
Figure 15a: IR ATR diamond spectrum of 3^{rd} generation retinoid BMS 753, used for the preparation of the salt of example 15, film from THF.
Figure 15b: IR ATR diamond spectrum of the salt of example 15, film from THF.
Figure 16a: IR ATR diamond spectrum of 3^{rd} generation retinoid CD 3106 (AGN 193'109), used for the preparation of the salt of example 16, film from THF.
Figure 16b: IR ATR diamond spectrum of the salt of example 16, film from THF.
Figure 17a: IR ATR diamond spectrum of 2^{nd} generation retinoid acitretin, (TMPP), used for the preparation of the salt of example 17, film from THF.
Figure 17b: IR ATR diamond spectrum of the salt of example 17 film from THF.
Figure 18a: IR ATR diamond spectrum of Anhydrohomoharringtonine (AHHT) used for the preparation of the salt of example 18, film from THF.
Figure 18b: IR ATR diamond spectrum of the salt of example 18, film from THF.
Figure 19a: IR ATR diamond spectrum of the salt of example 19 (crystals from THF).
Figure 20a: IR ATR diamond spectrum of the all-*trans*-retinoic acid of example 20, film.
Figure 20b: IR ATR diamond spectrum of the salt of example 20 (film).

### Examples

### General Procedure for Experimental Methods

### 1.1 General Procedures for Salts Preparation

Cation and anion precursors were weighed and separately dissolved in the selected solvent, including, eventually, the deuterated ones, at a concentration close of saturation and at a temperature close of solvent boiling temperature, then both solutions were mixed under stirring then slowly cooled and evaporated. During the running of the reaction, small sampling of the reaction medium were collected and directly deposited on the diamond plate of an FT-IR spectrometer. After the disappearance of the residual solvent, checked in 3000 cm⁻¹ region, the amount of residual acid not yet converted into salt was evaluated by the progressive disappearance of the carbonyl band of the non-ionized acid at 1680 ±10 cm⁻¹ After a period ranging from a few minutes up to several days, solvent was evaporated by argon flushing and, eventually, prismatic crystals of salt or off-white amorphous solid were collected. A sample of the batch of crystals was kept suspended in its mother liquors for the eventual subsequent X-ray diffraction analyses. The remainder of the batch was dried under vacuum for further solid characterization, comparative stability studies and drug formulation. A weighted sample was dissolved in sterile water for further *in vitro* biological testing. Similar procedure extrapolated at the multigram-scale was performed in using full current Good Laboratory Practices conditions for preparation and analysis of sample for *in vivo* testing. Samples for clinical studies including formulated were prepared by an approved subcontractor laboratory. The later process was fully documented and supervised by two independent quality control and assurance teams. Stability studies were performed according the approved FDA procedures.

### 1.2 General Procedures for Solid State Characterization

### • Infrared Spectra

All vibrational spectra were recorded on a Perkin Elmer IR FT Spectrum 2 apparatus equipped with diamond ATR accessory that is to say using Attenuated Total Reflection (ATR) technique. The crystalline solids were crushed directly by *in situ* compression on the diamond window.

### 1.3 General Procedures for Liquid /amorphous State and Solution Characterizations

### • Infrared Spectra

All vibrational spectra were recorded on a Perkin Elmer IR FT Spectrum 2 apparatus equipped with diamond ATR accessory that is to say using Attenuated Total Reflection (ATR) technique. The amorphous state was demonstrated by dissolving the product in deuterated methanol or deuterated chloroform, then generating the film by *in situ* evaporation on the diamond window.

### • Nuclear Magnetic Resonance

NMR spectra were recorded automatically on a Bruker Avance III spectrometer NanoBay-400MHz (9.4 Tesla magnet) with a BBFO+ probe and 120-positions sampler, allows for automatic mode NMR experiments one and two dimensions mainly for nuclei: ¹H, ²H, ¹¹B, ¹³C, ¹⁵N, ¹⁹F, ²⁷Al, ³¹P, ¹¹⁹Sn, or on a Bruker Avance III-600MHz spectrometer.

Dissolving salts for ¹³C NMR: 30 mg of compound were dissolved in 600 µL (5% m/V) of methanol D₄ or deuterium oxyde (or both if specified) or tetrahydrofuran D₈.(THF-D₈).

Water suppression: The irradiation technique known as 'watergate' (Selective pulse flanked by gradient pulses) was used for proton NMR in the presence of D₂O and/or MOD₄ as solvents.

### 1.4 High Performance Liquid Chromatography

Routine experiments were performed on a Waters HPLC-MS-DAD coupled system (3100 pump, DAD 996 detector, 3100 mass detector).

### 1.5 Solubility Determination

Solubility in water at 25° C. was measured semi-quantitatively at a threshold of 5 g per 100 mL. All the homoharringtonine salts described in the below examples, unless otherwise stated, are soluble at this threshold. Homoharringtonine base itself is soluble at a threshold lower than 0.1 g per mL.

Unless otherwise stated all reagents and solvent are sourced from known commercial distributor including for starting products such as retinoid acids and homoharringtonine.

### EXAMPLE 1

### Preparation of homoharringtonine all-trans-retinoate or

### Butanedioic acid, 2-hydroxy-2-(4-hydroxy-4-methylpentyl)-, 1-[(1S,3aR,14bS)-1,5,6,8,9,14b-hexahydro-2-methoxy-4H-cyclopenta[a][1,3]dioxolo[4,5-h]pyrrolo[2,1-b][3]benzazepin-1-yl]4-methyl ester, 3,7-dimethyl-9-(2,6,6-trimethyl-1-cyclohexen-1-yl)-2E,4E,6E,8E,-nonatetraenoate (1:1)]

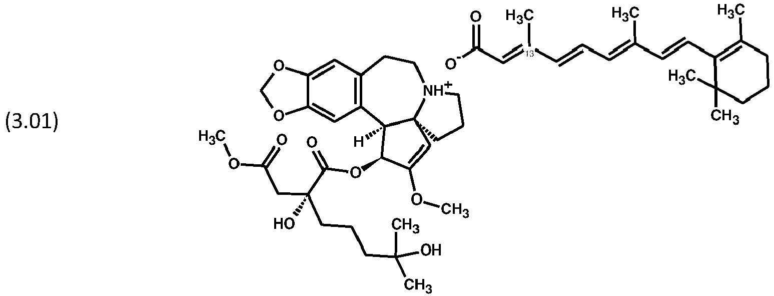

Molecular Formula (in the ionized state): C₂₉H₄₀NO₉.C₂₀H₂₇O₂ (C₄₉H₆₇NO₁₁)
Formula Weight: 847.0490348
Composition: C(69.60%) H(7.97%) N(1.65%) O(20.78%)

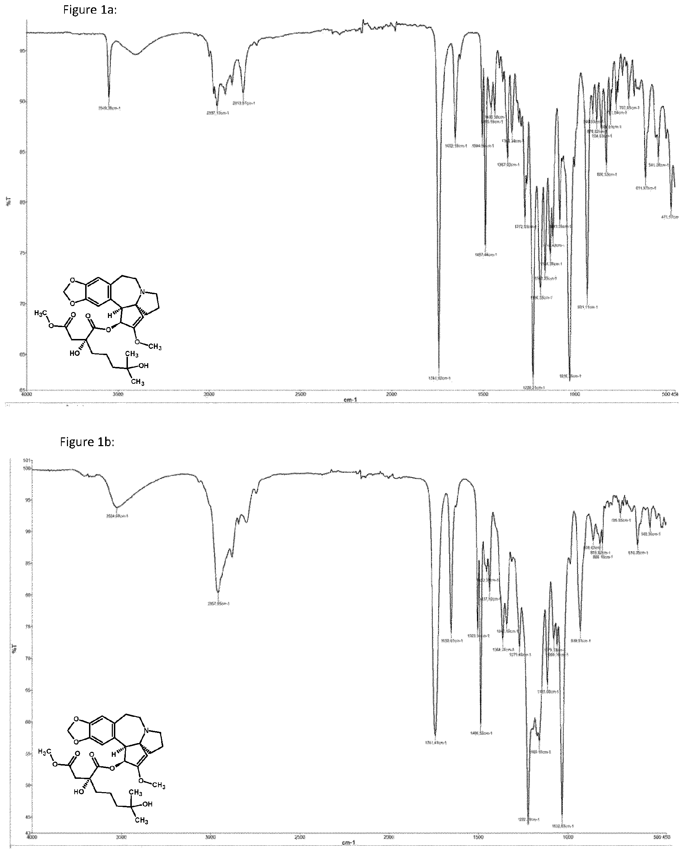

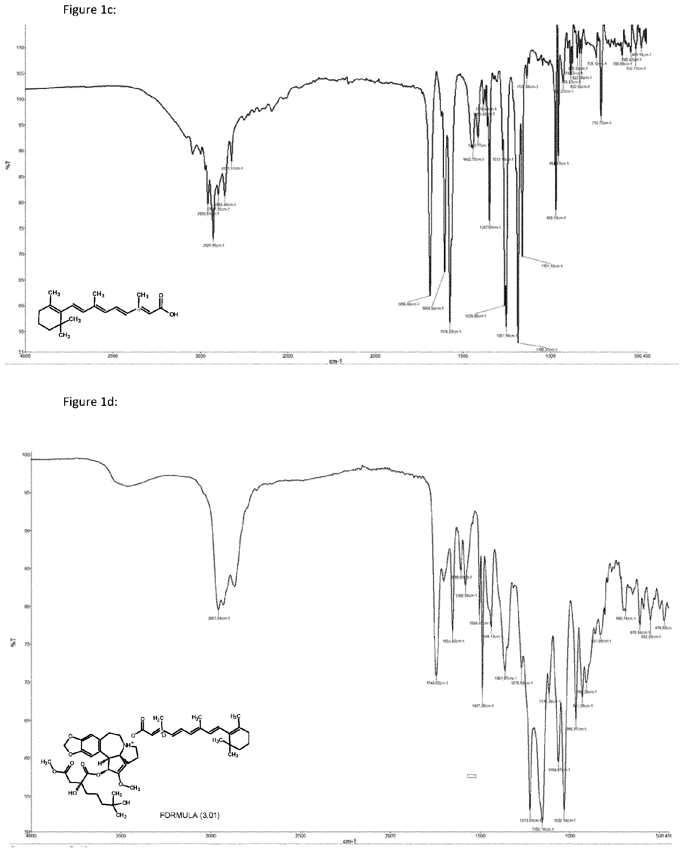

In a flask equipped with stirring and flushing of filtered argon was added amorphous purified HHT. To this stirred refluxing solution in dry methanol (EU pharmacopeia quality) or octadeuterated tetrahydrofuran 0.1 mmol of purified homoharringtonine prepared according to the method described in general procedures, was added under filtered argon atmosphere, a hot solution of commercial 3,7-Dimethyl-9-(2,6,6-trimethyl-1-cyclohexen-1-yl)-2*E*,4*E*,6*E*,8*E*,-nonatetraenoic acid (namely all-*trans*-retinoic acid) until disappearance of eventual solid particle. The heating and stirring were stopped. After 24 h at refrigerator the solution leaves deposit of white prismatic crystals of homoharringtonine all-*trans*-retinoate. A small sample of crystal in suspension in the solvent was preleved for X-ray diffraction analysis then crystallin suspension was poured in a Büchner funnel under sterile atmosphere. The drained crystallin mass was broken and dried in vacuum at 40°, 12 h. A salt having the following analytical characteristics was obtained:
Infrared spectrum, see figure 1d.

### EXAMPLE 2

### Preparation of harringtonine all-trans-retinoate or Butanedioic acid, 2-hydroxy-2-(3-hydroxy-3-methylbutyl)-, 1-[(1S,3aR,14bS)-1,5,6,8,9,14b-hexahydro-2-methoxy-4H-cyclopenta[a][1,3]dioxolo[4, 5-h]pyrrolo[2,1-b][3]benzazepin-1-yl] 4-methyl ester, 3,7-dimethyl-9-(2,6,6-trimethyl-1-cyclohexen-1-yl)-2E,4E,6E,8E,-nonatetraenoate (1:1)]

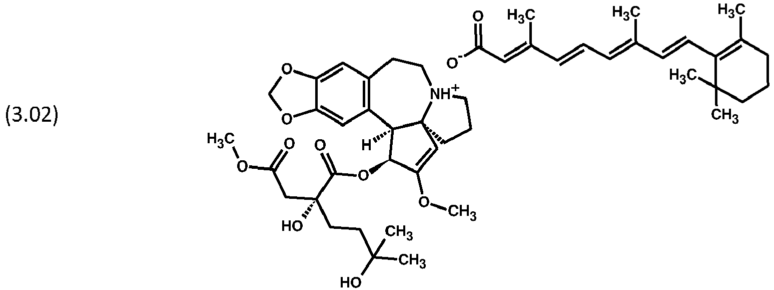

Molecular Formula (in the ionized state): C₂₈H₃₈NO₉.C₂₀H₂₇O₂ (C₄₈H₆₅NO₁₁)
Formula Weight: 833.0224548
Composition: C(69.33%) H(7.86%) N(1.68%) 0(21.13%)

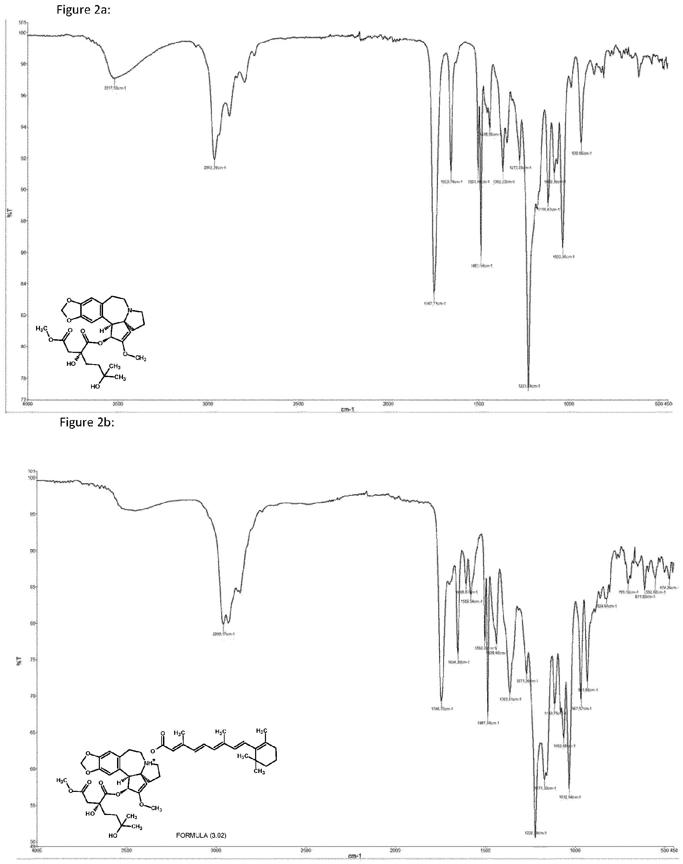

A similar procedure of preparation to the one depicted in general procedure and in example 1 was used, except that homoharringtonine was replaced with harringtonine. A salt having the following analytical characteristics was obtained:
Infrared spectrum, see figure 2b.

### EXAMPLE 3

### Preparation of homoharringtonine 13-cis-retinoate or Butanedioic acid, 2-hydroxy-2-(4-hydroxy-4-methylpentyl)-, 1-[(1S,3aR,14bS)-1,5,6,8,9,14b-hexahydro-2-methoxy-4H-cyclopenta[a][1,3] dioxolo[4,5-h]pyrrolo[2,1-b][3]benzazepin-1-yl]4-methyl ester, 13-cis-Retinoate (1:1)]

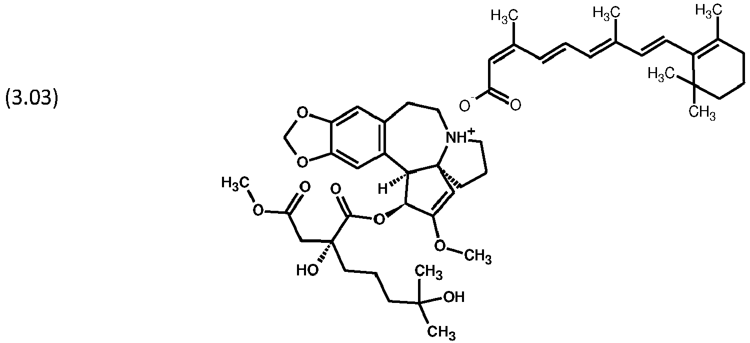

Molecular Formula (in the ionized state): C₂₉H₄₀NO₉.C₂₀H₂₇O₂ (C₄₉H₆₇NO₁₁)
Formula Weight: 847.0490348
Composition: C(69.60%) H(7.97%) N(1.65%) O(20.78%)

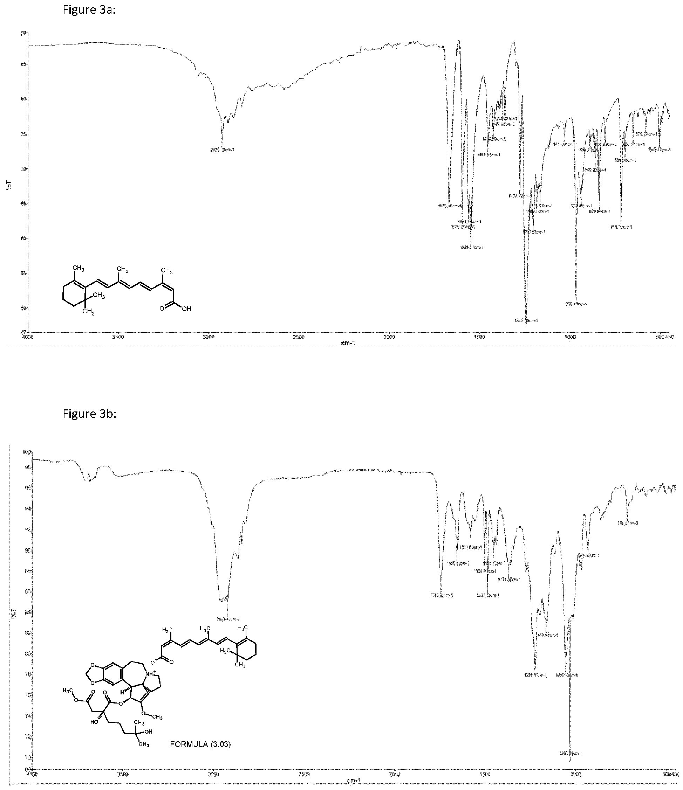

A similar procedure of preparation to the one depicted in general procedure and in example 1 was used, except that all-*trans*-retinoic acid was replaced with 13-cis-Retinoic acid, (namely AGN 190013 or isoretinoin). A salt having the following analytical characteristics was obtained:
Infrared spectrum, see figure 3b.

### EXAMPLE 4

### Preparation of homoharringtonine "acitretinoate" or Butanedioic acid, 2-hydroxy-2-(4-hydroxy-4-methylpentyl)-, 1-[(1S,3aR,14bS)-1,5,6,8,9,14b-hexahydro-2-methoxy-4H-cyclopenta[a][1,3] dioxolo[4,5-h]pyrrolo[2,1-b][3]benzazepin-1-yl]4-methyl ester, (2E,4E,6E,8E)-9-(4-methoxy-2,3,6-trimethylphenyl)-3,7-dimethyl-2,4,6,8-Nonatetraenoate

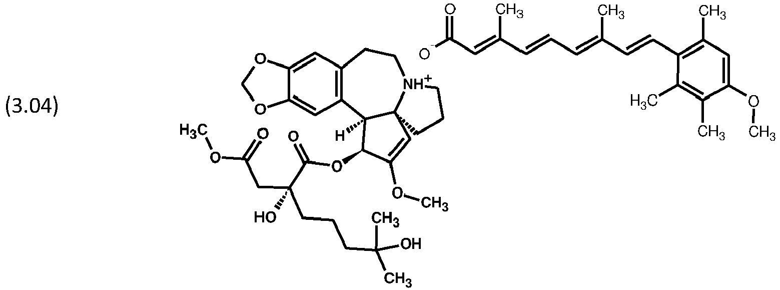

Molecular Formula (in the ionized state): C₂₉H₄₀NO₉.C₂₁H₂₅O₃ C₅₀H₆₅NO₁₂
Formula Weight: 872.0506
Composition: C(68.86%) H(7.51%) N(1.61%) O(22.02%)

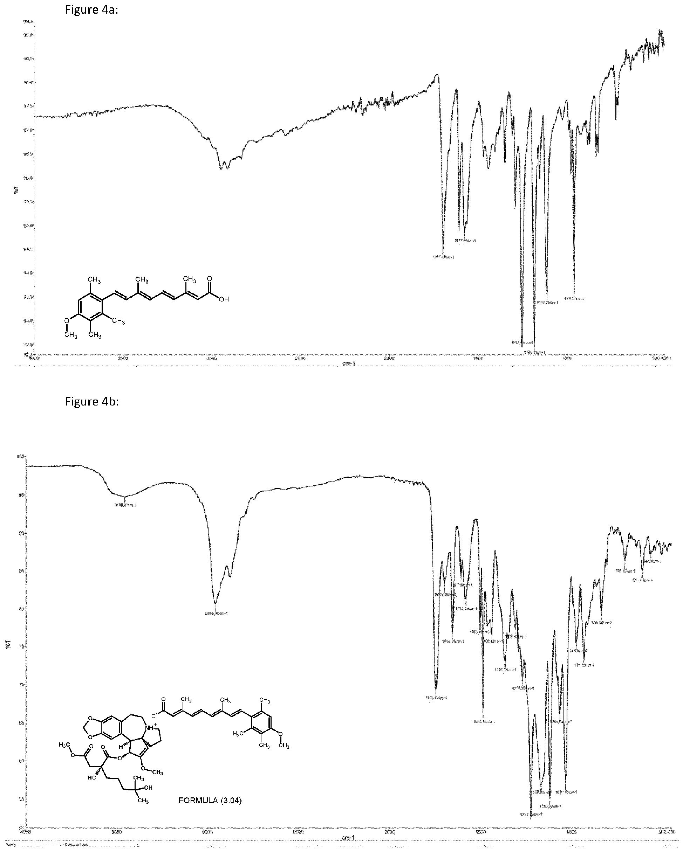

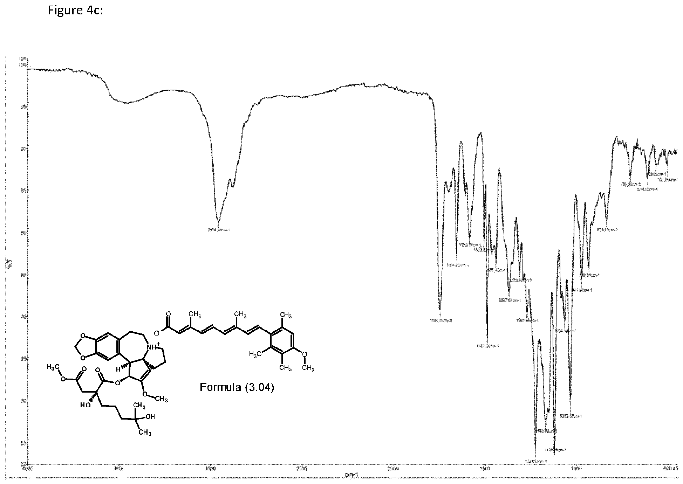

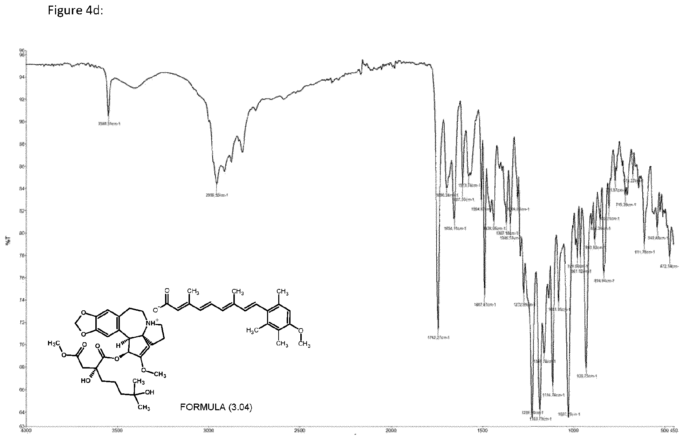

A similar procedure of preparation to the one depicted in general procedure and in example 1 was used, except that all-*trans*-retinoic acid was replaced with (2*E*,4*E*,6*E*,8*E*)-9-(4-methoxy-2,3,6-trimethylphenyl)-3,7-dimethyl-2,4,6,8-Nonatetraenoic acid,(namely acitretin or acitretinoin or etretin or Ro 10-1670 or TMPP). A salt having the following analytical characteristics was obtained:
Infrared spectrum, see figure 4b.

### EXAMPLE 5

### Preparation of homoharringtonine retinobenzoate, or homoharringtonine tamibarotenoate, or Butanedioic acid, 2-hydroxy-2-(4-hydroxy-4-methylpentyl)-, 1-[(1S,3aR,14bS)-1,5,6,8,9,14b-hexahydro-2-methoxy-4H-cyclopenta[a][1,3]dioxolo[4,5-h]pyrrolo[2,1-b][3]benzazepin-1-yl]4-methyl ester, 4-[[(5,6,7,8-Tetrahydro-5,5,8,8-tetramethyl-2-naphthalenyl)amino]carbonyl]benzoate

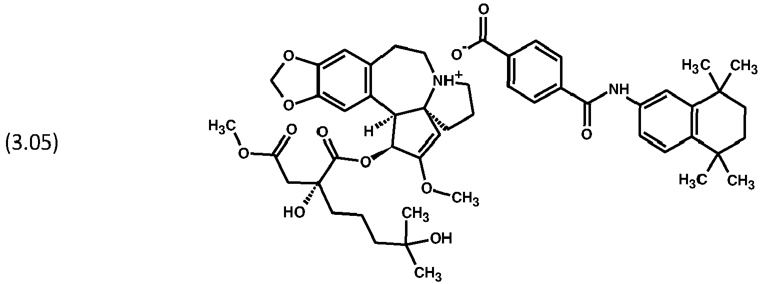

Molecular Formula (in the ionized state): C₂₉H₄₀NO₉.C₂₂H₂₄NO₃ (C₅₁H₆₄N₂O₁₂)
Formula Weight: 897.06006
Composition: C(68.28%) H(7.19%) N(3.12%) 0(21.40%)

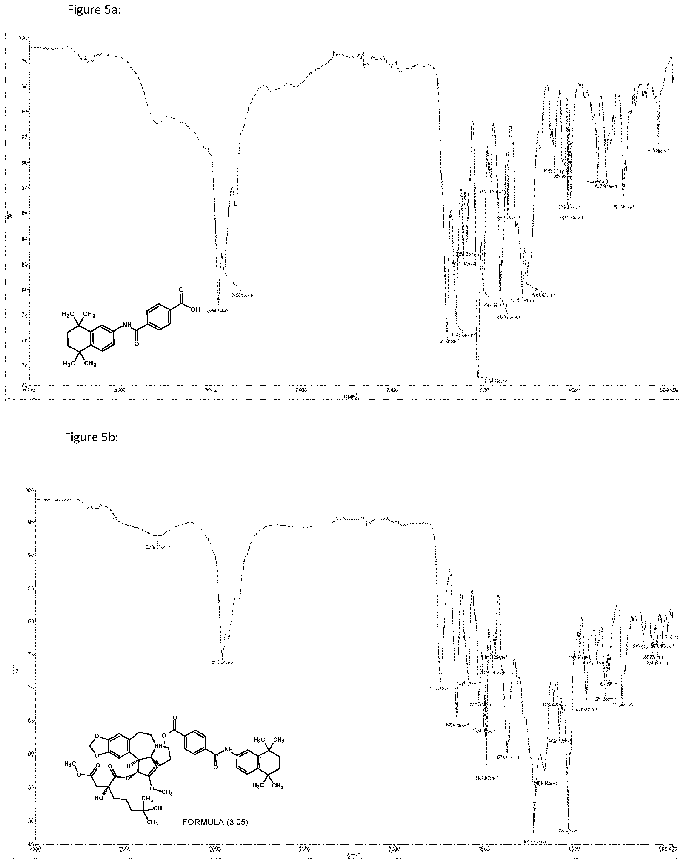

A similar procedure of preparation to the one depicted in general procedure and in example 1 was used, except that all-*trans*-retinoic acid was replaced with 4 - [[(5,6,7,8-Tetrahydro-5,5,8,8-tetramethyl-2-naphthalenyl) amino] carbonyl]benzoic acid, (namely retinobenzoic acid or AM80 or tamibarotene), A salt having the following analytical characteristics was obtained:
Infrared spectrum, see figure 5b.

### EXAMPLE 6

### Preparation of homoharringtonine "adarotenoate" or Butanedioic acid, 2-hydroxy-2-(4-hydroxy-4-methylpentyl)-, 1-[(1S,3aR,14bS)-1,5,6,8,9,14b-hexahydro-2-methoxy-4H-cyclopenta[a][1,3] dioxolo[4,5-h]pyrrolo[2,1-b][3]benzazepin-1-yl]4-methyl ester, (2E)-3-(4'-Hydroxy-3'-tricyclo[3.3.1.1^{3,7}]dec-1-yl[1,1'-biphenyl]-4-yl)-2-propenoate

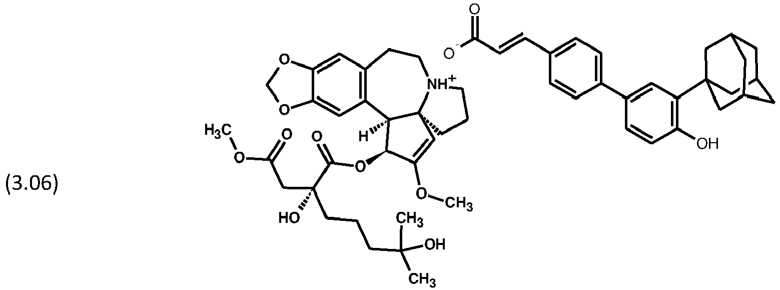

Molecular Formula (in the ionized state): C₂₉H₄₀NO₉.C₂₅H₂₅O₃ (C₅₄H₆₅NO₁₂)
Formula Weight: 920.0934
Composition: C(70.49%) H(7.12%) N(1.52%) O(20.87%)

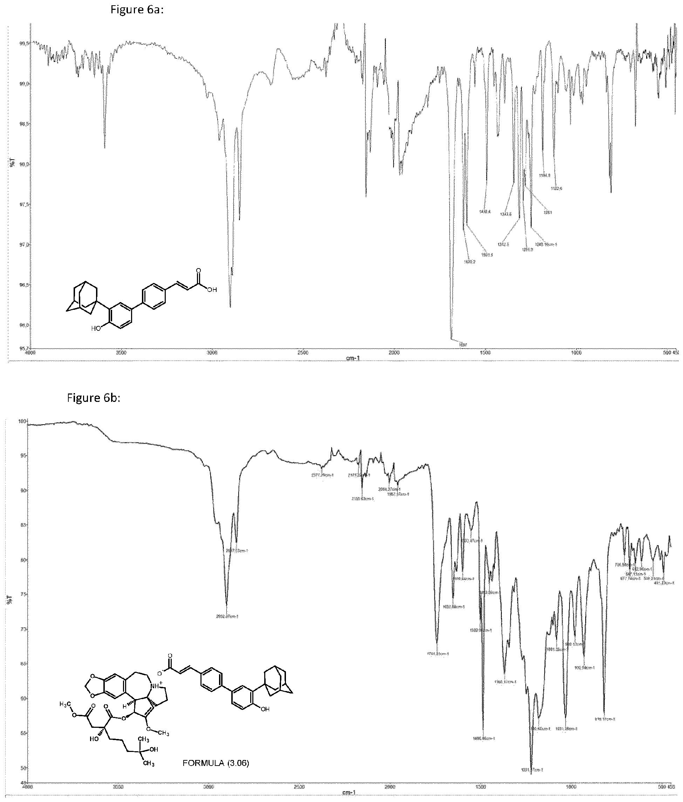

A similar procedure of preparation to the one depicted in general procedure and in example 1 was used, except that all-*trans*-retinoic acid was replaced with (2E)-3-(4'-Hydroxy-3'-tricyclo[3.3.1.1^{3,7}]dec-1-yl[1,1'-biphenyl]-4-yl)-2-propenoic acid, (namely adarotene or ST 1926 or AHPC), A salt having the following analytical characteristics was obtained:
Infrared spectrum, see figure 6b.

### EXAMPLE 7

### Preparation of homoharringtonine "bexarotenoate" or Butanedioic acid, 2-hydroxy-2-(4-hydroxy-4-methylpentyl)-, 1-[(1S,3aR,14bS)-1,5,6,8,9,14b-hexahydro-2-methoxy-4H-cyclopenta[a][1,3] dioxolo[4,5-h]pyrrolo[2,1-b][3]benzazepin-1-yl]4-methyl ester, 4-[1-(5,6,7,8-Tetrahydro-3,5,5,8,8-pentamethyl-2-naphthalenyl)ethenyl]benzoate

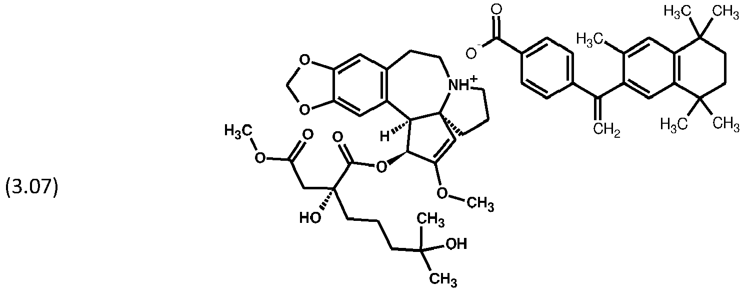

Molecular Formula (in the ionized state): C₂₉H₄₀NO₉.C₂₄H₂₇O₂ (C₅₃H₆₇NO₁₁)
Formula Weight: 894.09918
Composition: C(71.20%) H(7.55%) N(1.57%) 0(19.68%)

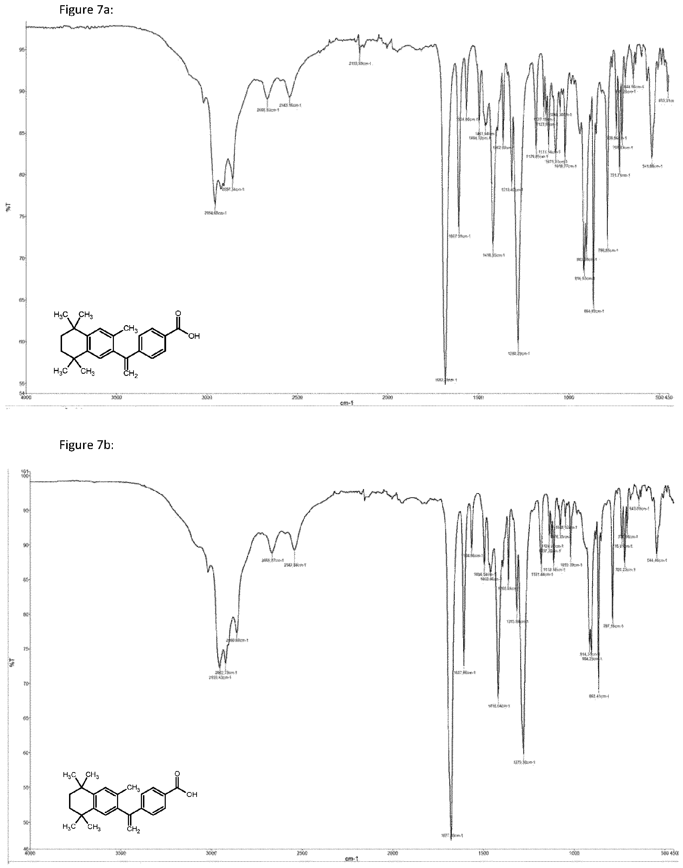

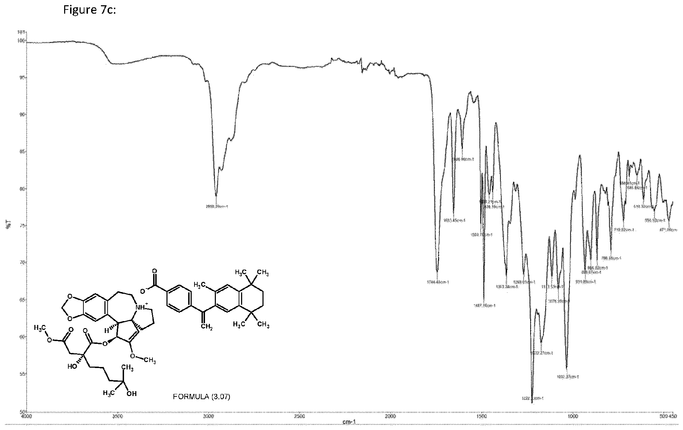

A similar procedure of preparation to the one depicted in general procedure and in example 1 was used, except that all-*trans*-retinoic acid was replaced with 4-[1-(5,6,7,8-Tetrahydro-3,5,5,8,8-pentamethyl-2-naphthalenyl)ethenyl]benzoic acid, (namely bexarotene or LG 100069 or LG 1069 or LG 69 or SR 11247 or RO 26-4455), A salt having the following analytical characteristics was obtained:
Infrared spectrum, see figure 7c.

### EXAMPLE 8

### Preparation of homoharringtonine "adapalenoate" or Butanedioic acid, 2-hydroxy-2-(4-hydroxy-4-methylpentyl)-, 1-[(1S,3aR,14bS)-1,5,6,8,9,14b-hexahydro-2-methoxy-4H-cyclopenta[a][1,3] dioxolo[4,5-h]pyrrolo[2,1-b][3]benzazepin-1-yl]4-methyl ester, 6-[3-(1-Adamantyl)-4-methoxyphenyl]-2-naphthoate

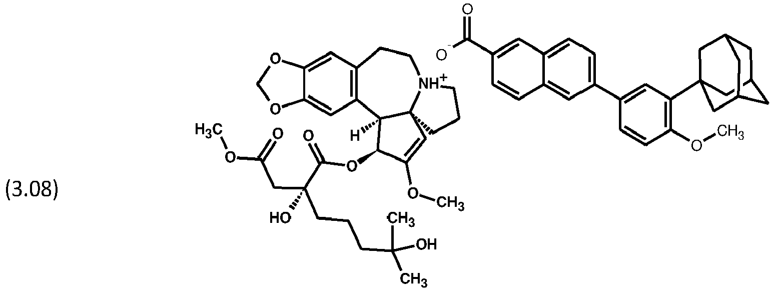

Molecular Formula (in the ionized state): C₂₉H₄₀NO₉.C₂₈H₂₇O₃ (C₅₇H₆₇NO₁₂)
Formula Weight: 958.14138
Composition: C(71.45%) H(7.05%) N(1.46%) O(20.04%)

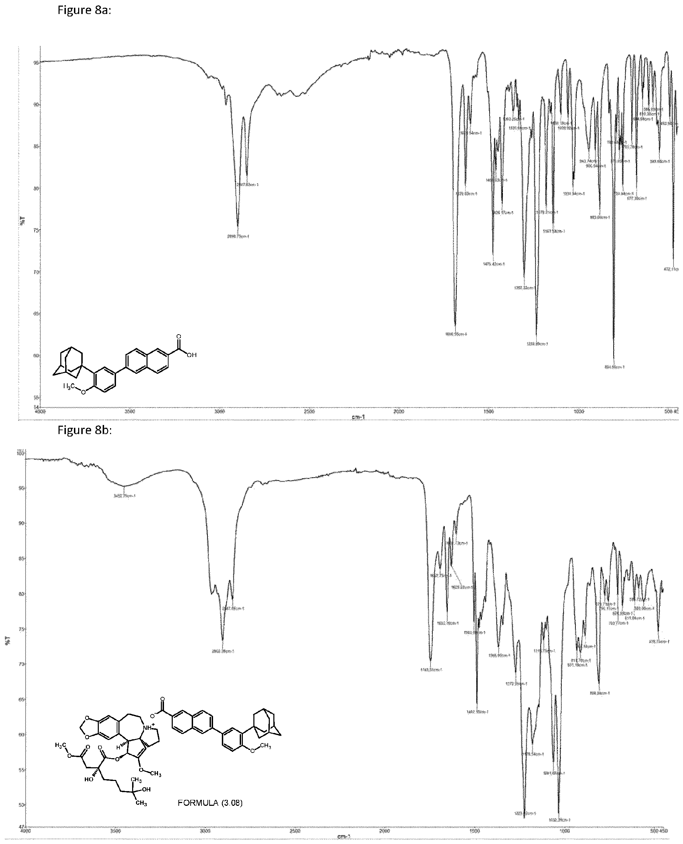

A similar procedure of preparation to the one depicted in general procedure and in example 1 was used, except that all-*trans*-retinoic acid was replaced with 6-[3-(1-Adamantyl)-4-methoxyphenyl]-2-naphthoic acid,, (namely adapalene or CD 271). A salt having the following analytical characteristics was obtained:
Infrared spectrum, see figure 8b.

### EXAMPLE 9

### Preparation of harringtonine "adapalenoate" or Butanedioic acid, 2-hydroxy-2-(3-hydroxy-3-methylbutyl)-, 1-[(1S,3aR,14bS)-1,5,6,8,9,14b-hexahydro-2-methoxy-4H-cyclopenta[a][1,3]dioxolo[4, 5-h]pyrrolo[2,1-b][3]benzazepin-1-yl]4-methyl ester, 6-[3-(1-Adamantyl)-4-methoxyphenyl]-2-naphthoate

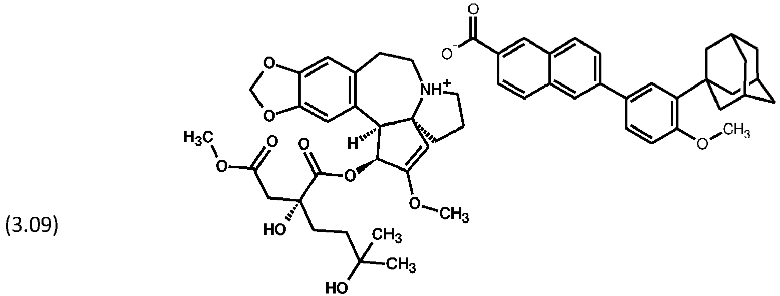

Molecular Formula (in the ionized state): C₂₈H₃₈NO₉.C₂₈H₂₇O₃ (C₅₆H₆₅NO12)
Formula Weight: 958.14138
Composition: C(71.45%) H(7.05%) N(1.46%) O(20.04%)

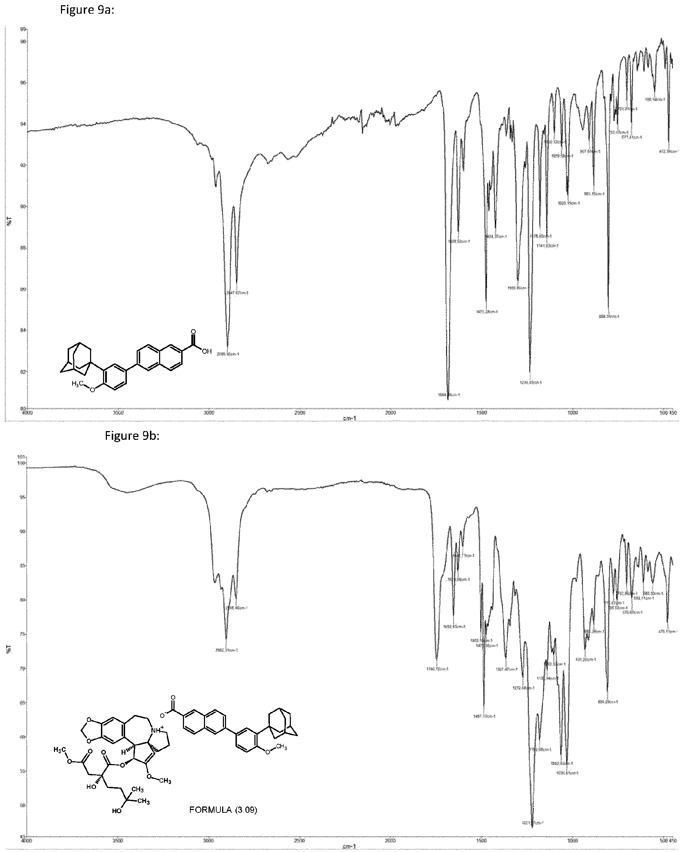

A similar procedure of preparation to the one depicted in general procedure and in example 1 was used, except that all-*trans*-retinoic acid was replaced with 6-[3-(1-Adamantyl)-4-methoxyphenyl]-2-naphthoic acid, (namely adapalene or CD 271) and homoharringtonine was replaced by harringtonine. A salt having the following analytical characteristics was obtained:
Infrared spectrum, see figure 9b.

### EXAMPLE 10

### Preparation of Butanedioic acid, 2-hydroxy-2-(4-hydroxy-4-methylpentyl)-, 1-[(1S,3aR,14bS)-1,5,6,8, 9,14b-hexahydro-2-methoxy-4H-cyclopenta[c][1,3]dioxolo[4,5-h]pyrrolo[2,1-b][3]benzazepin-1-yl]4-methyl ester, 4-[[[5,6-Dihydro-5,5-dimethyl-8-(3-quinolinyl)-2-naphthalenyl]carbonyl]amino]benzoate

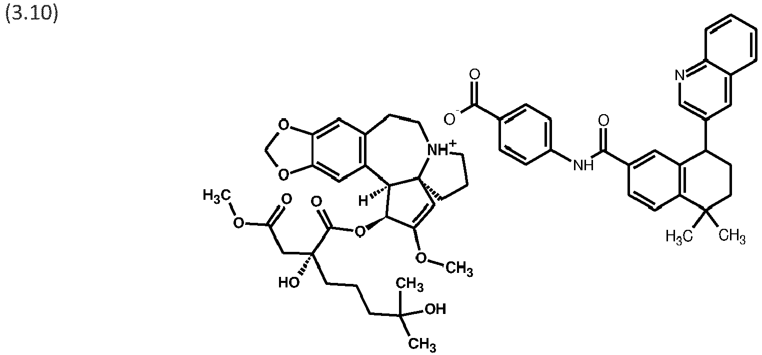

Molecular Formula (in the ionized state): C₂₉H₄₀NO₉.C₂₉H₂₅N₂O₃ (C₅₈H₆₅N₃O₁₂)
Formula Weight: 996.1496
Composition: C(69.93%) H(6.58%) N(4.22%) 0(19.27%)

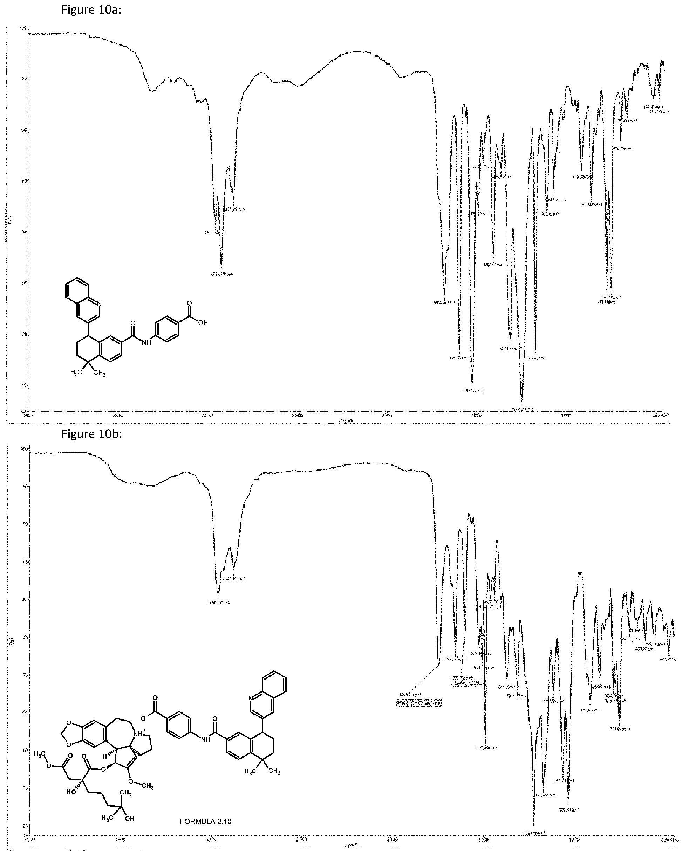

A similar procedure of preparation to the one depicted in general procedure and in example 1 was used, except that all-*trans*-retinoic acid was replaced with 4-[[[5,6-Dihydro-5,5-dimethyl-8-(3-quinolinyl)-2-naphthalenyl]carbonyl]amino]benzoic acid, (namely BMS614 or BMS 195614), A salt having the following analytical characteristics was obtained:
Infrared spectrum, see figure 10b.

### EXAMPLE 11

### Preparation of Butanedioic acid, 2-hydroxy-2-(4-hydroxy-4-methylpentyl)-, 1-[(1S,3aR,14bS)-1,5,6,8, 9,14b-hexahydro-2-methoxy-4H-cyclopenta[a][1,3]dioxolo[4,5-h]pyrrolo[2,1-b][3]benzazepin-1-yl]4-methyl ester, 6-[3-(1-adamantyl)-4-hydroxyphenyl]-2-naphtalenecarboxylate

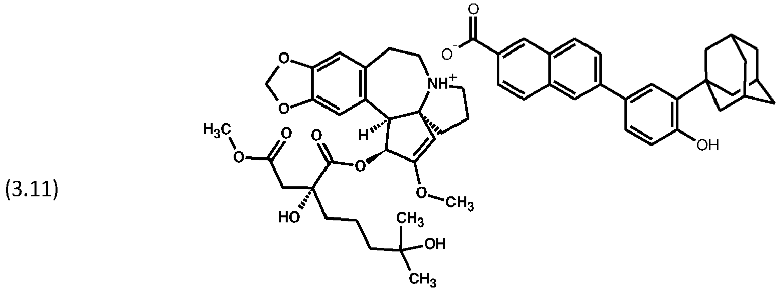

Molecular Formula (in the ionized state): C₂₉H₄₀NO₉.C₂₇H₂₅O₃ (C₅₆H₆₅NO₁₂)
Formula Weight: 944.1148
Composition: C(71.24%) H(6.94%) N(1.48%) O(20.34%)

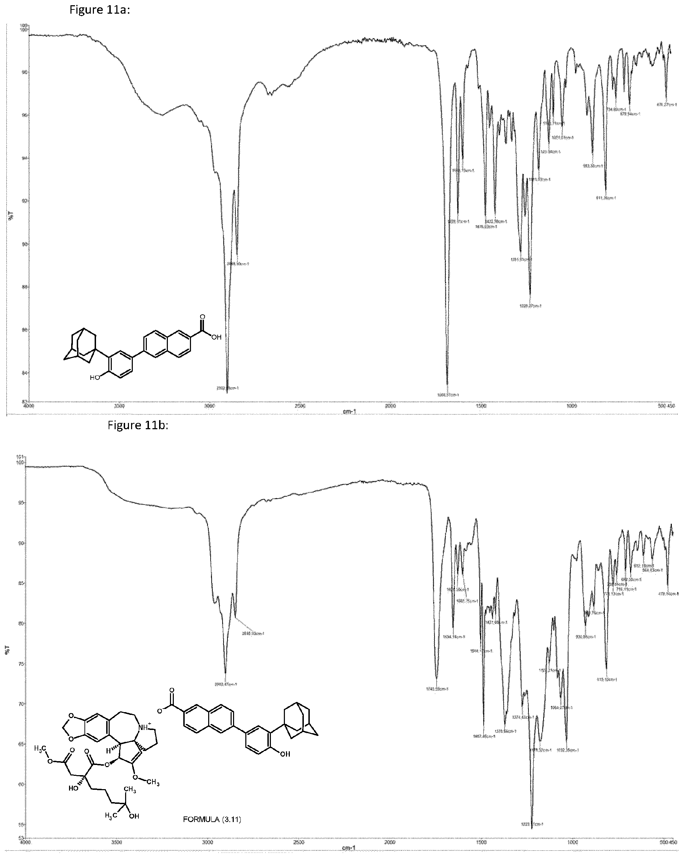

A similar procedure of preparation to the one depicted in general procedure and in example 1 was used, except that all-*trans*-retinoic acid was replaced with 6-[3-(1-adamantyl)-4-hydroxyphenyl]-2-naphtalenecarboxylic acid, (namely CD 437 or AHPN). A salt having the following analytical characteristics was obtained:
Infrared spectrum, see figure 11b.

### EXAMPLE 12

### Preparation of Butanedioic acid, 2-hydroxy-2-(4-hydroxy-4-methylpentyl)-, 1-[(1S,3aR,14bS)-1,5,6,8, 9,14b-hexahydro-2-methoxy-4H-cyclopenta[a][1,3]dioxolo[4,5-h]pyrrolo[2,1-b][3]benzazepin-1-yl]4-methyl ester, 4-(6-Hydroxy-7-tricyclo[3.3.1.1^{3,7}]dec-1-yl-2-naphthalenyl)benzoate

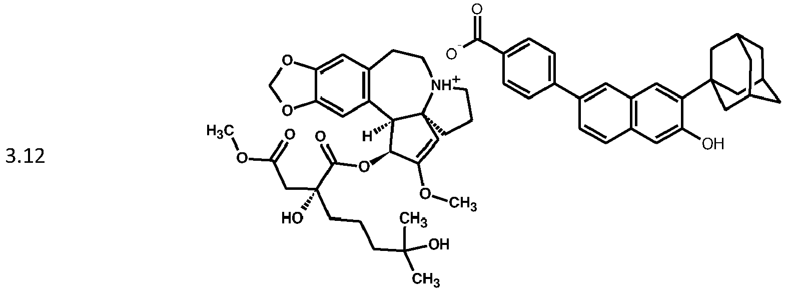

Molecular Formula (in the ionized state): C₂₉H₄₀NO₉.C₂₇H₂₅O₃ (C₅₆H₆₅NO₁₂)
Formula Weight: 944.1148
Composition: C(71.24%) H(6.94%) N(1.48%) O(20.34%)

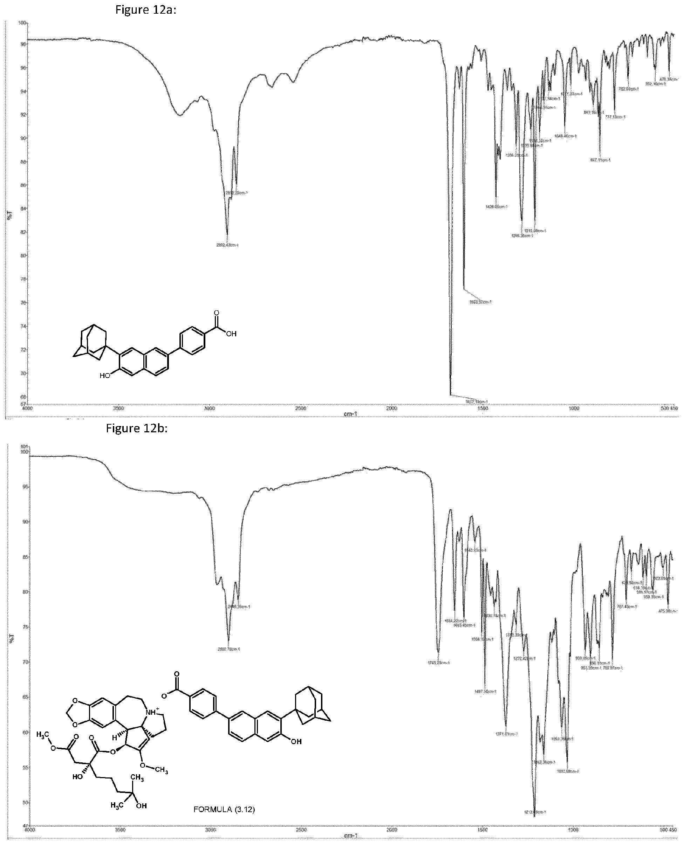

A similar procedure of preparation to the one depicted in general procedure and in example 1 was used, except that all-*trans*-retinoic acid was replaced with 4-methyl ester, 4-(6-Hydroxy-7-tricyclo[3.3.1.1^{3,7}]dec-1-yl-2-naphthalenyl)benzoic acid,(namely CD 1530). A salt having the following analytical characteristics was obtained:
Infrared spectrum, see figure 12b.

### EXAMPLE 13

### Preparation of homoharringtonine arotinoate (or "arotinoidate") or Butanedioic acid, 2-hydroxy-2-(4-hydroxy-4-methylpentyl)-, 1-[(1S,3aR,14bS)-1,5,6,8,9,14b-hexahydro-2-methoxy-4H-cyclopenta[a] [1,3]dioxolo[4,5-h]pyrrolo[2,1-b][3]benzazepin-1-yl]4-methyl ester, 4-[(1E)-2-(5,6,7,8-Tetrahydro-5,5,8,8-tetramethyl-2-naphthalenyl)-1-propen-1-yl]benzoate

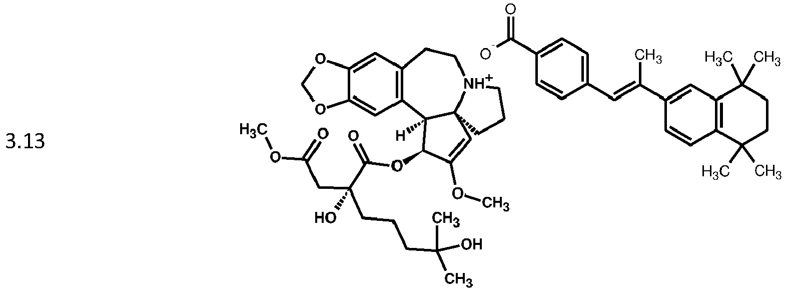

Molecular Formula (in the ionized state): C₂₉H₄₀NO₉.C₂₄H₂₇O₂ (C₅₃H₆₇NO₁₁)
Formula Weight: 894.09918
Composition: C(71.20%) H(7.55%) N(1.57%) 0(19.68%)

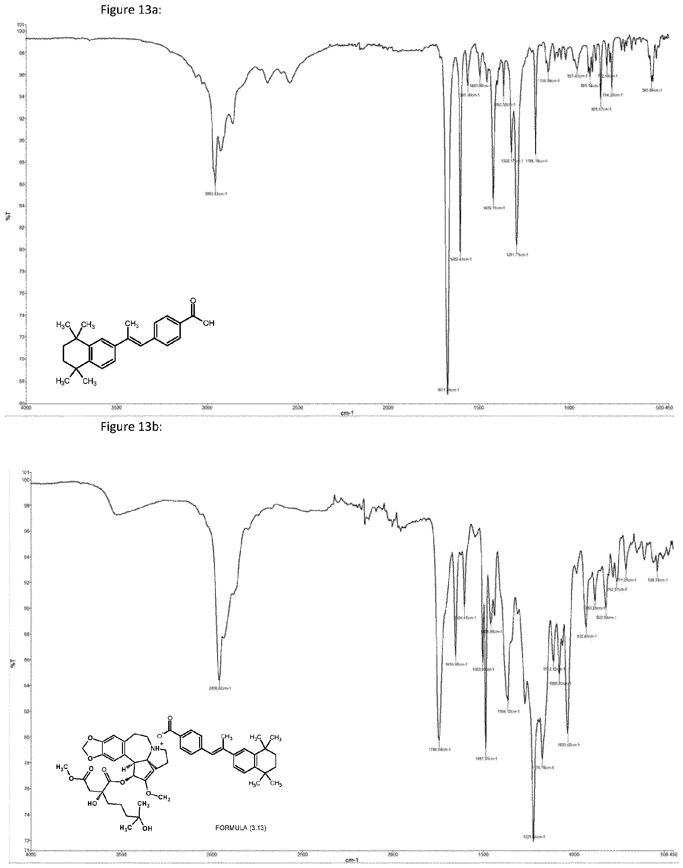

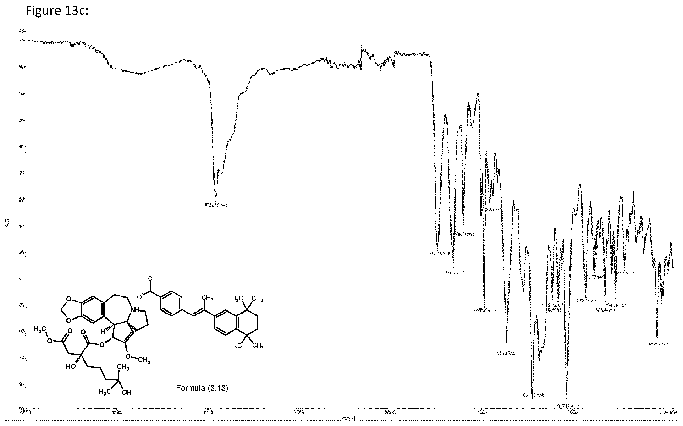

A similar procedure of preparation to the one depicted in general procedure and in example 1 was used, except that all-*trans*-retinoic acid was replaced with 4-[(1E)-2-(5,6,7,8-Tetrahydro-5,5,8,8-tetramethyl-2-naphthalenyl)-1-propen-1-yl]benzoic acid, (namely "arotinoid" acid or AGN 191183 or Ro 13-7410 or TTNPB). A salt having the following analytical characteristics was obtained:
Infrared spectrum, see figure 13b and 13c.

### EXAMPLE 14

### Preparation of harringtonine arotinoate (or "arotinoidate") or Butanedioic acid, 2-hydroxy-2-(3-hydroxy-3-methylbutyl)-, 1-[(1S,3aR,14bS)-1,5,6,8,9,14b-hexahydro-2-methoxy-4H-cyclopenta[a][1,3] dioxolo[4,5-h]pyrrolo[2,1-b][3]benzazepin-1-yl]4-methyl ester, 4-[(1E)-2-(5,6,7,8-Tetrahydro-5,5,8,8-tetramethyl-2-naphthalenyl)-1-propen-1-yl]benzoate

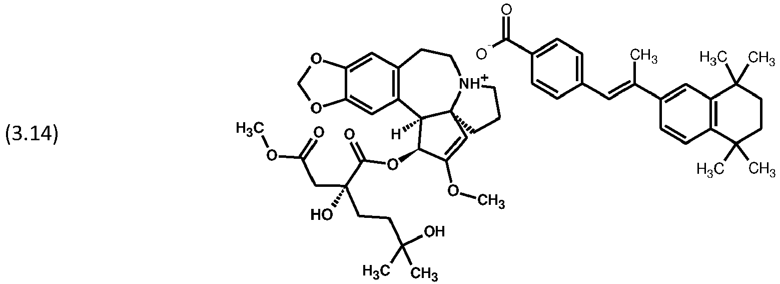

Molecular Formula (in the ionized state): C₂₈H₃₈NO₉.C₂₄H₂₇O₂ (C₅₂H₆₅NO₁₁)
Formula Weight: 880.0726
Composition: C(70.97%) H(7.44%) N(1.59%) O(20.00%)

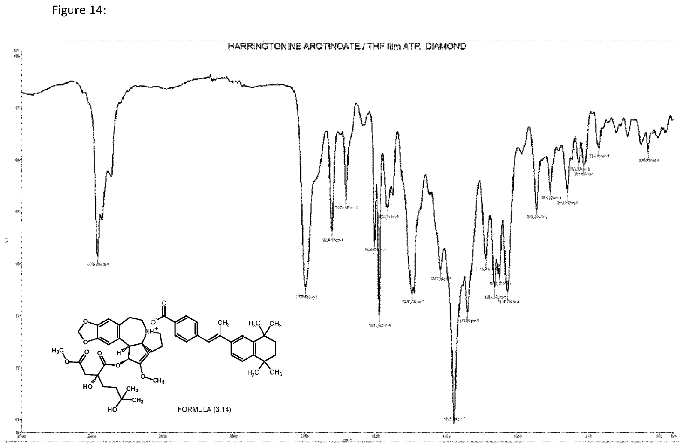

A similar procedure of preparation to the one depicted in general procedure and in example 1 was used, except that all-*trans*-retinoic acid was replaced with 4-[(1E)-2-(5,6,7,8-Tetrahydro-5,5,8,8-tetramethyl-2-naphthalenyl)-1-propen-1-yl]benzoic acid, (namely "arotinoid" acid or AGN 191183 or Ro 13-7410 or TTNPB). A salt having the following analytical characteristics was obtained: Infrared spectrum, see figure 14.

### EXAMPLE 15

### Preparation of Butanedioic acid, 2-hydroxy-2-(4-hydroxy-4-methylpentyl)-, 1-[(1S,3aR,14bS)-1,5,6,8, 9,14b-hexahydro-2-methoxy-4H-cyclopenta[a][1,3]dioxolo[4,5-h]pyrrolo[2,1-b][3]benzazepin-1-yl]4-methyl ester, 4-[[(2,3-Dihydro-1,1,3,3-tetramethyl-2-oxo-1H-inden-5-yl)carbonyl]amino]benzoate

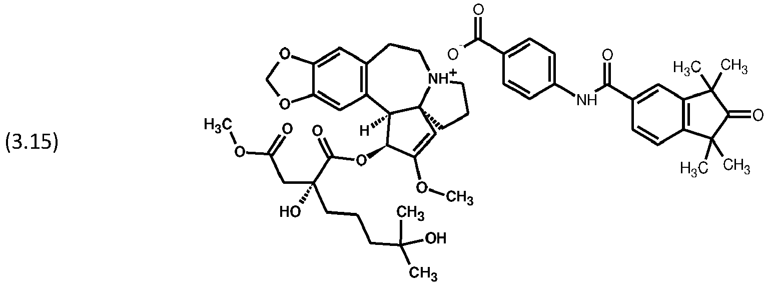

Molecular Formula (in the ionized state): C₂₉H₄₀NO₉. C₂₁H₂₀NO₄ (C₅₀H₆₀N₂O₁₃)
Formula Weight: 897.017
Composition: C(66.95%) H(6.74%) N(3.12%) 0(23.19%)

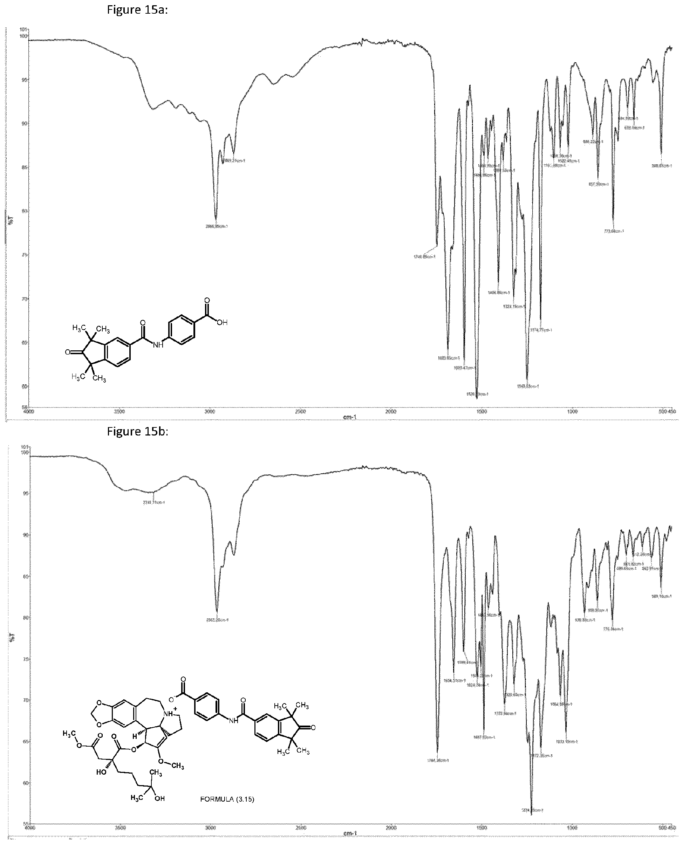

A similar procedure of preparation to the one depicted in general procedure and in example 1 was used, except that all-*trans*-retinoic acid was replaced with 4-[[(2,3-Dihydro-1,1,3,3-tetramethyl-2-oxo-1H-inden-5-yl)carbonyl]amino]benzoic acid, (namely BMS 753). A salt having the following analytical characteristics was obtained: Infrared spectrum, see figure 15b.

### EXAMPLE 16

### Preparation of Butanedioic acid, 2-hydroxy-2-(4-hydroxy-4-methylpentyl)-, 1-[(1S,3aR,14bS)-1,5,6,8, 9,14b-hexahydro-2-methoxy-4H-cyclopenta[a][1,3]dioxolo[4,5-h]pyrrolo[2,1-b][3]benzazepin-1-yl]4-methyl ester, 4-[[5,6-dihydro-5,5-dimethyl-8-(4-methylphenyl)-2-naphthalenyl]ethynyl]benzoate

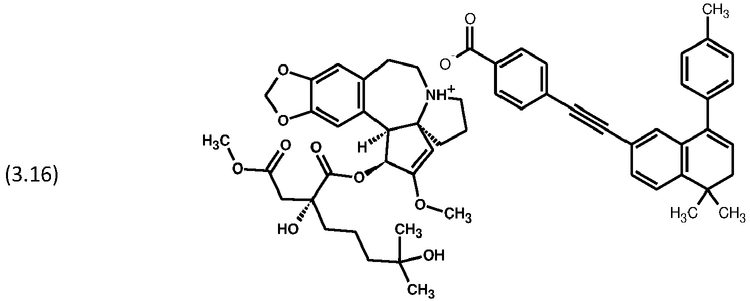

Molecular Formula (in the ionized state): C₂₉H₄₀NO₉.C₂₈H₂₃O₂ (C₅₇H₆₃NO₁₁)
Formula Weight: 938.11022
Composition: C(72.98%) H(6.77%) N(1.49%) 0(18.76%)

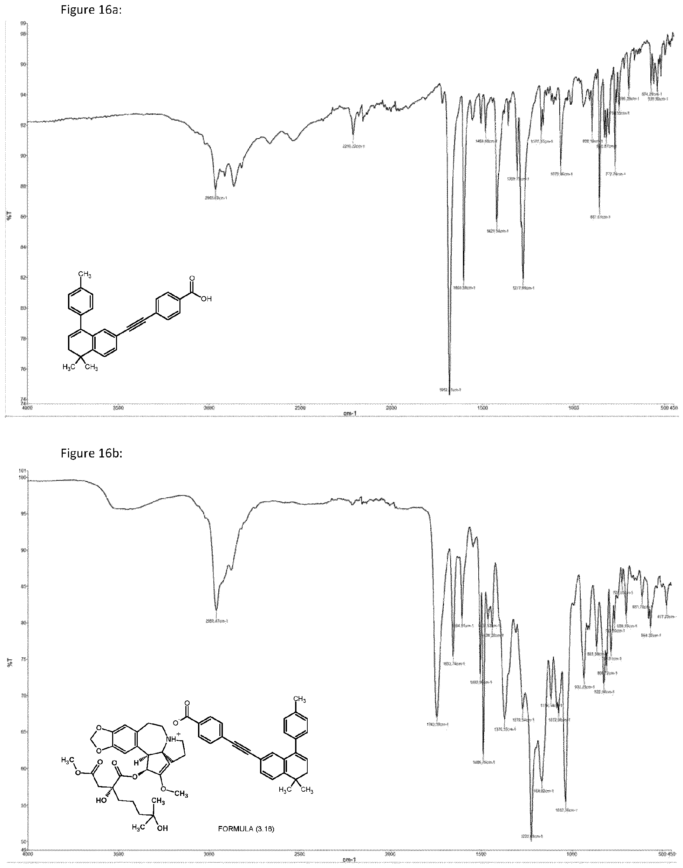

A similar procedure of preparation to the one depicted in general procedure and in example 1 was used, except that all-*trans*-retinoic acid was replaced with 4-[[5,6-dihydro-5,5-dimethyl-8-(4-methylphenyl)-2-naphthalenyl]ethynyl]benzoic acid, (namely AGN 193109 or CD 3106). A salt having the following analytical characteristics was obtained: Infrared spectrum, see figure 16b.

### EXAMPLE 17

### Preparation of harringtonine acitretinate or Butanedioic acid, 2-hydroxy-2-(3-hydroxy-3-methylbutyl) -, 1-[(1S,3aR,14bS)-1,5,6,8,9,14b-hexahydro-2-methoxy-4H-cyclopenta[a][1,3]dioxolo[4,5-h] pyrrolo[2,1-b][3]benzazepin-1-yl]4-methyl ester, (2E,4E,6E,8E)-9-(4-methoxy-2,3,6-trimethylphenyl)-3,7-dimethyl-2,4,6,8-Nonatetraenoate

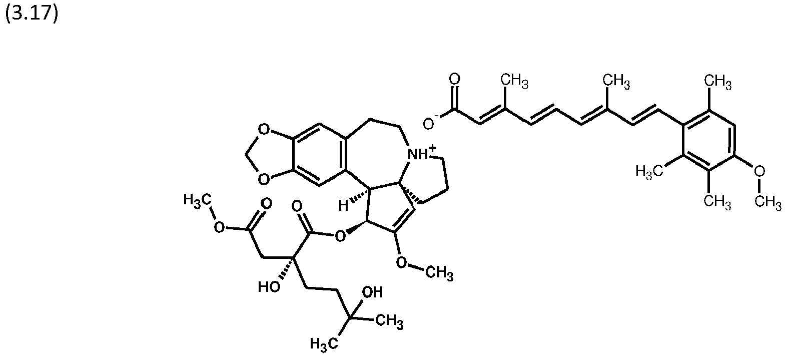

Molecular Formula (in the ionized state): C28H38NO9.C21H25O3 C49H63NO12
Formula Weight: 858.0240
Composition: C(68.59%) H(7.40%) N(1.63%) O(22.38%)

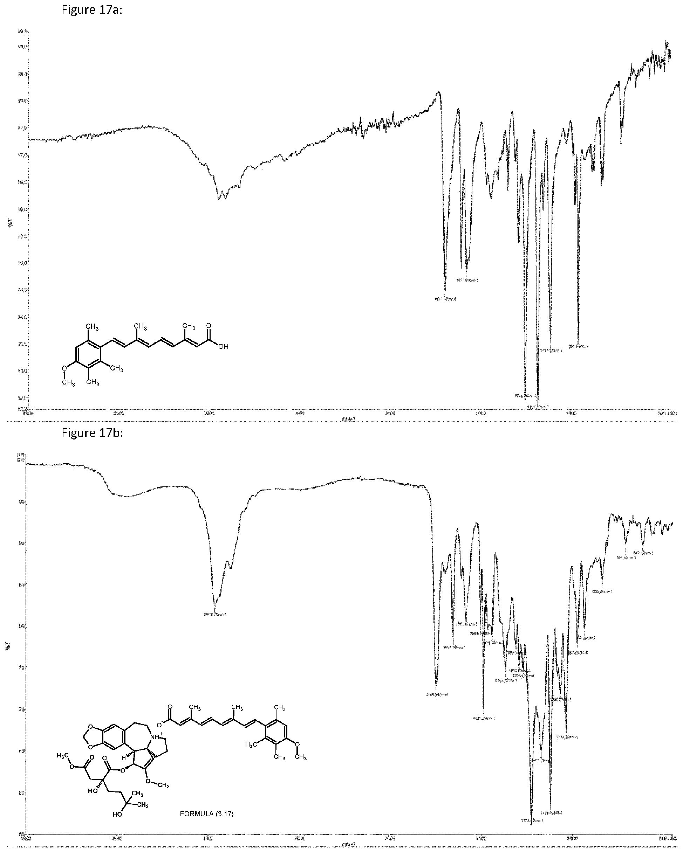

A similar procedure of preparation to the one depicted in general procedure and in example 1 was used, except that homoharringtonine was replaced with harringtonine and all-trans-retinoic acid was replaced with (2E,4E,6E,8E)-9-(4-methoxy-2,3,6-trimethylphenyl)-3,7-dimethyl-2,4,6,8-Nonatetraenoic acid, namely AGN 190013 or isoretinoin).
Infrared spectrum, see figure 17b.

### EXAMPLE 18

### Preparation of Anhydrohomoharringtonine all-trans-retinoate or [Cephalotaxine, 3-[(2R)-tetrahydro-2-(2-methoxy-2-oxoethyl)-6,6-dimethyl-2-pyrancarboxylate]], [3,7-dimethyl-9-(2,6,6-trimethyl-1-cyclohexen-1-yl)-2E,4E,6E,8E,-nonatetraenoate (1:1)]

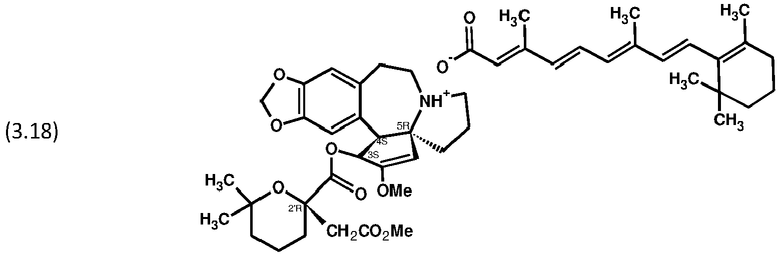

Molecular Formula (in the ionized state): C₂₉H₃₈NO₈.C₂₀H₂₇O₂ (C₄₉H₆₅NO₁₀)
Formula Weight: 829.03375
Composition C(71.11%) H(7.90%) N(1.69%) 0(19.30%)

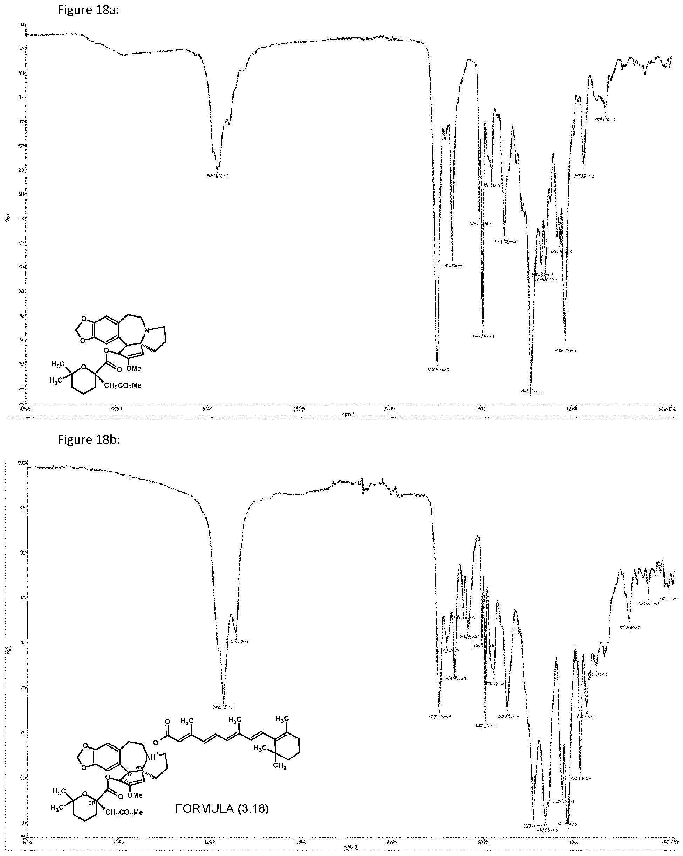

A similar procedure of preparation to the one depicted in general procedure and in example 1 was used, except that homoharringtonine was replaced with anhydrohomoharringtonine a salt having the following analytical characteristics was obtained:
Infrared spectrum, see figure 18b.

### EXAMPLE 19

### Preparation of anhydrohomoharringtonine "acitretinoate" or [Cephalotaxine, 3-[(2R)-tetrahydro-2-(2-methoxy-2-oxoethyl)-6,6-dimethyl-2-pyrancarboxylate]], (2E,4E,6E,8E)-9-(4-methoxy-2,3,6-trimethylphenyl)-3,7-dimethyl-2,4,6,8-Nonatetraenoate

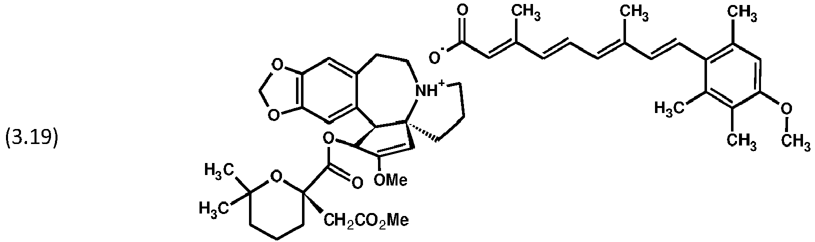

Molecular Formula (in the ionized state): C₂₉H₃₈NO₈.C₂₁H₂₅O₃ C₅₀H₆₃NO₁₁
Formula Weight: 854.03532
Composition: C(70.32%) H(7.44%) N(1.64%) 0(20.61%)

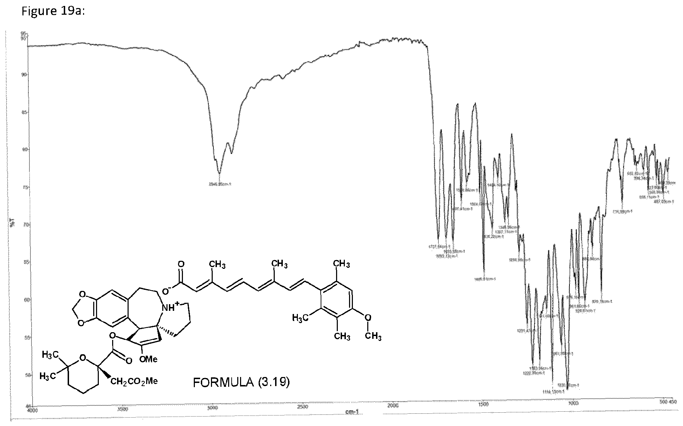

A similar procedure of preparation to the one depicted in general procedure and in example 1 was used, except that all-*trans*-retinoic acid was replaced with (2*E*,4*E*,6*E*,8*E*)-9-(4-methoxy-2,3,6-trimethylphenyl)-3,7-dimethyl-2,4,6,8-Nonatetraenoic acid, (namely acitretin or acitretinoin or etretin or Ro 10-1670 or TMPP), and homoharringtonine was replaced with anhydrohomoharringtonine, a salt having the following analytical characteristics was obtained:
Infrared spectrum, see figure 19a.

### EXAMPLE 20

### Preparation of anhydrohomoharringtonine "adapalenoate" or [Cephalotaxine, 3-[(2R)-tetrahydro-2-(2-methoxy-2-oxoethyl)-6,6-dimethyl-2-pyrancarboxylate]], 6-[3-(1-Adamantyl)-4-methoxyphenyl]-2-naphthoate

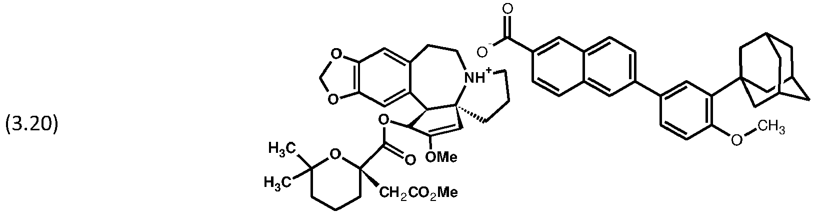

Molecular Formula (in the ionized state): C₂₉H₃₈NO₈.C₂₈H₂₇O₃ (C₅₇H₆₅NO₁₁)
Formula Weight: 940.1261
Composition: C(72.82%) H(6.97%) N(1.49%) 0(18.72%)

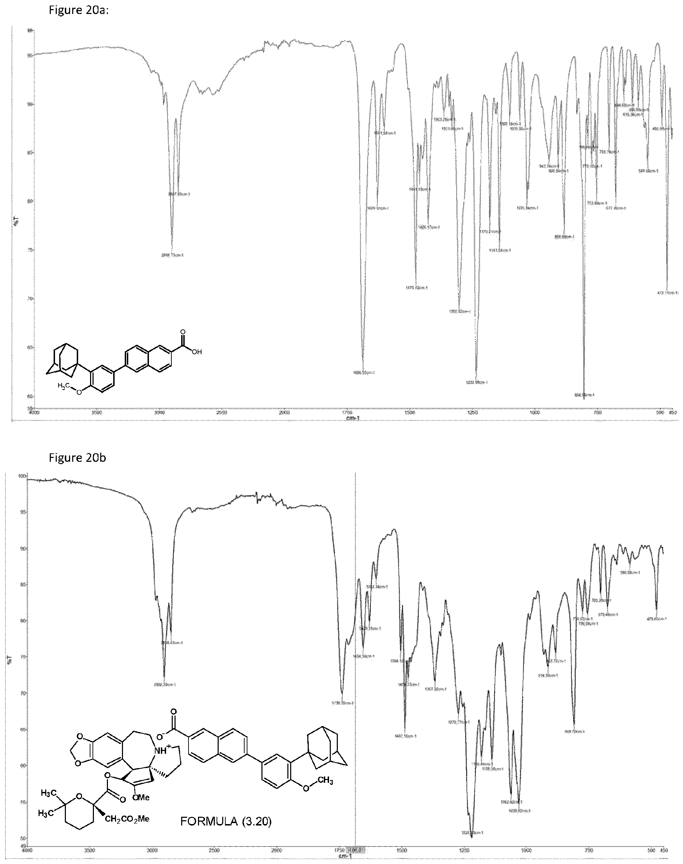

A similar procedure of preparation to the one depicted in general procedure and in example 1 was used, except that all-*trans*-retinoic acid was replaced with 6-[3-(1-Adamantyl)-4-methoxyphenyl]-2-naphthoic acid, (namely adapalene or CD 271), and homoharringtonine was replaced with anhydrohomoharringtonine. A salt having the following analytical characteristics was obtained:
Infrared spectrum, see figure 20.

### EXAMPLE 21

### Preparation of homoharringtonine tazarotenoate or Butanedioic acid, 2-hydroxy-2-(4-hydroxy-4-methylpentyl)-, 1-[(1S,3aR,14bS)-1,5,6,8,9,14b-hexahydro-2-methoxy-4H-cyclopenta[a][1,3] dioxolo[4,5-h]pyrrolo[2,1-b][3]benzazepin-1-yl]4-methyl ester, 6-[2-(3,4-Dihydro-4,4-dimethyl-2H-1-benzothiopyran-6-yl)ethynyl]-3-pyridinecarboxylate

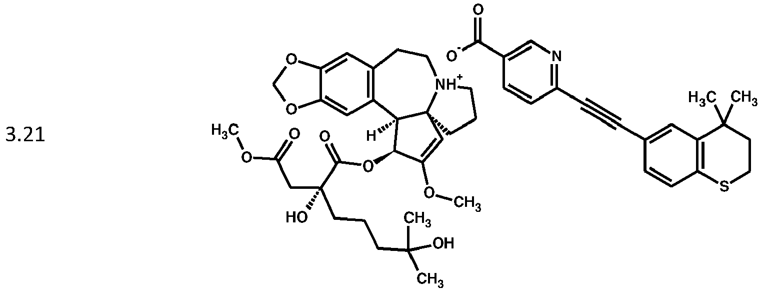

Molecular Formula (in the ionized state): C29H40NO9.C27H29N2O2 (C56H69N3O11)
Formula Weight: 960.16056
Composition: C(70.05%) H(7.24%) N(4.38%) 0(18.33%)

A similar procedure of preparation to the one depicted in general procedure and in example 1 was used, except that all-trans-retinoic acid was replaced with 6-[2-(3,4-Dihydro-4,4-dimethyl-2H-1-benzothiopyran-6-yl)ethynyl]-3-pyridinecarboxylic acid of formula namely AGN 190299).

### EXAMPLE 22

### Preparation of homoharringtonine palovarotenoate or Butanedioic acid, 2-hydroxy-2-(4-hydroxy-4-methylpentyl)-, 1-[(1S,3aR,14bS)-1,5,6,8,9,14b-hexahydro-2-methoxy-4H-cyclopenta[a][1,3] dioxolo[4,5-h]pyrrolo[2,1-b][3]benzazepin-1-yl]4-methyl ester, 4-[(1E)-2-[5,6,7,8-tetrahydro-5,5,8,8-tetramethyl-3-(1H-pyrazol-1-ylmethyl)-2-naphthalenyl]ethenyl]-benzoate

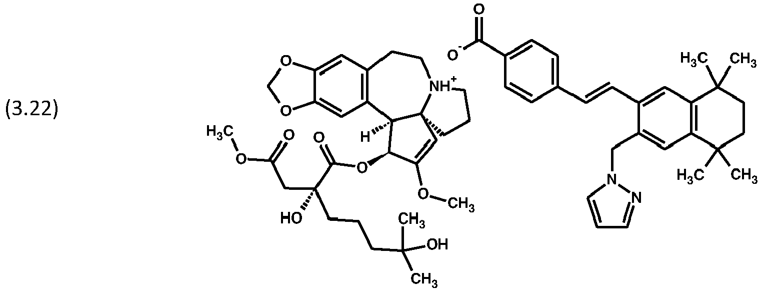

Molecular Formula (in the ionized state): C₂₉H₄₀NO₉.C₂₇H₂₉N₂O₂ (C₅₆H₆₉N₃O₁₁)
Formula Weight: 960.16056
Composition: C(70.05%) H(7.24%) N(4.38%) 0(18.33%)

A similar procedure of preparation to the one depicted in general procedure and in example 1 was used, except that all-*trans*-retinoic acid was replaced with 4-[(1*E*)-2-[5,6,7,8-tetrahydro-5,5,8,8-tetramethyl-3-(1*H*-pyrazol-1-ylmethyl)-2-naphthalenyl]ethenyl]-benzoic acid of formula (namely palovarotene or R 667 or Ro 3300074).

### EXAMPLE 23

### Preparation of homoharringtonine Am 580 salt or Butanedioic acid, 2-hydroxy-2-(4-hydroxy-4-methylpentyl)-, 1-[(1S,3aR,14bS)-1,5,6,8,9,14b-hexahydro-2-methoxy-4H-cyclopenta[a][1,3] dioxolo[4,5-h]pyrrolo[2,1-b][3]benzazepin-1-yl]4-methyl ester, 4-[[(5,6,7,8-tetrahydro-5,5,8,8-tetramethyl-2-naphthalenyl)carbonyl]amino]-benzoate

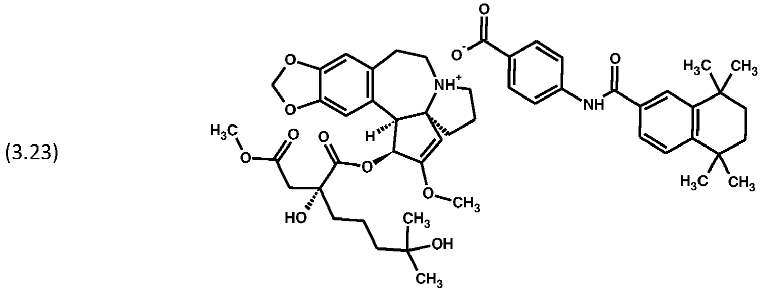

Molecular Formula (in the ionized state): C₂₉H₄₀NO₉.C₂₂H₂₄NO₃ (C₅₁H₆₄N₂O₁₂)
Formula Weight: 897.06006
Composition: C(68.28%) H(7.19%) N(3.12%) 0(21.40%)

A similar procedure of preparation to the one depicted in general procedure and in example 1 was used, except that all-*trans*-retinoic acid was replaced with 4-[[(5,6,7,8-tetrahydro-5,5,8,8-tetramethyl-2-naphthalenyl)carbonyl]amino]-benzoic acid of formula(namely Am 580 or CD 336 or Ro 40-6055).

### EXAMPLE 24

### Preparation of OP5215 all-trans-retinoate or Butanedioic acid, 2-hydroxy-2-(4-hydroxy-4-methylpentyl)-, 1-[(1S,3aR,14bS)-1,5,6,8,9,14b-hexahydro-2-methoxy-4H-cyclopenta[a][1,3]dioxolo[4,5-h] pyrrolo[2,1-b][3]benzazepin-1-yl]4-(2,4-hexadienyl) ester, [3,7-dimethyl-9-(2,6,6-trimethyl-1-cyclohexen-1-yl)-2E,4E,6E,8E,-nonatetraenoate (1:1)]

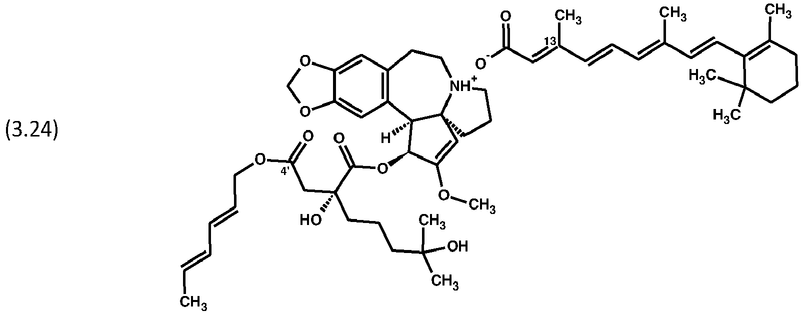

Molecular Formula (in the ionized state): C₃₄H₄₆NO₉.C₂₀H₂₇O₂ (C₅₄H₇₃NO₁₁)
Formula Weight: 913.1501748
Composition: C(71.14%) H(8.06%) N(1.53%) 0(19.27%)

A similar procedure of preparation to the one depicted in general procedure and in example 1 was used, except that homoharringtonine was replaced with its OP5215 analog and all-*trans*-retinoic acid was replaced with 3,7-dimethyl-9-(2,6,6-trimethyl-1-cyclohexen-1-yl)-2*E*,4*E*,6*E*,8*E*,-nonatetraenoic acid,(namely R667).

### EXAMPLE 25

### Preparation of OP5215 acitretinate or Butanedioic acid, 2-hydroxy-2-(4-hydroxy-4-methyl-pentyl)-, 1-[(1S,3aR,14bS)-1,5,6,8,9,14b-hexahydro-2-methoxy-4H-cyclopenta[a][1,3]dioxolo[4,5-h]pyrrolo[2,1-b][3]benzazepin-1-yl]4-(2,4-hexadienyl) ester, [(2E,4E,6E,8E)-9-(4-methoxy-2,3,6-trimethylphenyl)-3, 7-dimethyl-2,4,6,8-Nonatetraenoate (1:1)]

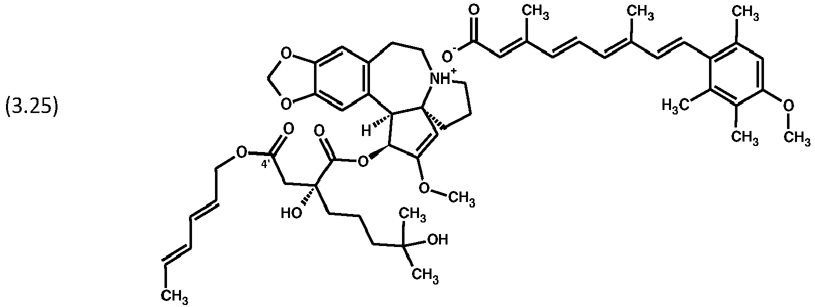

Molecular Formula (in the ionized state): C₃₄H₄₆NO₉.C₂₁H₂₅O₃ (C₅₅H₇₁NO₁₂)
Formula Weight: 938.15174
Composition: C(70.41%) H(7.63%) N(1.49%) O(20.47%

A similar procedure of preparation to the one depicted in general procedure and in example 1 was used, except that homoharringtonine was replaced with its OP5215 analog and all-*trans*-retinoic acid was replaced with [(2*E*,4*E*,6*E*,8*E*)-9-(4-methoxy-2,3,6-trimethylphenyl)-3,7-dimethyl-2,4,6,8-Nonatetraenoic acid, namely acitretin).

### EXAMPLE 26

### Preparation of OP5215 tamibarotenoate or Butanedioic acid, 2-hydroxy-2-(4-hydroxy-4-methylpentyl)-, 1-[(1S,3aR,14bS)-1,5,6,8,9,14b-hexahydro-2-methoxy-4H-cyclopenta[a][1,3]dioxolo[4,5-h] pyrrolo[2,1-b][3]benzazepin-1-yl]4-(2,4-hexadienyl) ester, 4-[[(5,6,7,8-Tetrahydro-5,5,8,8-tetramethyl-2-naphthalenyl)amino]carbonyl]benzoate

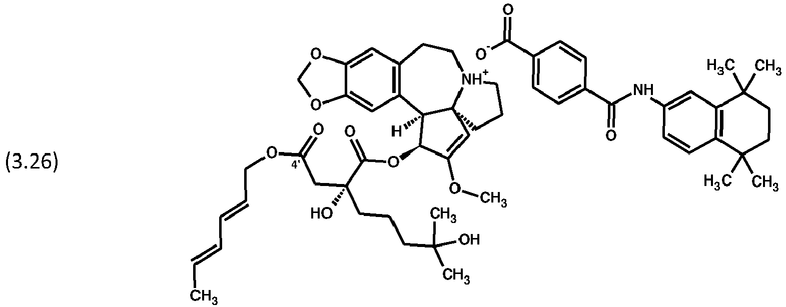

Molecular Formula (in the ionized state): C₃₄H₄₆NO₉.C₂₂H₂₄NO₃ (C₅₆H₇₀N₂O₁₂)
Formula Weight: 963.1612
Composition: C(69.83%) H(7.33%) N(2.91%) 0(19.93%)

A similar procedure of preparation to the one depicted in general procedure and in example 1 was used, except that homoharringtonine was replaced with its OP5215 analog and all-*trans*-retinoic acid was replaced 4-[[(5,6,7,8-Tetrahydro-5,5,8,8-tetramethyl-2-naphthalenyl)amino]-carbonyl]benzoic acid, (namely tamibarotene).

### EXAMPLE 27

### Preparation of OP5215, Am 580 salt or Butanedioic acid, 2-hydroxy-2-(4-hydroxy-4-methyl-pentyl)-, 1-[(1S,3aR,14bS)-1,5,6,8,9,14b-hexahydro-2-methoxy-4H-cyclopenta[a][1,3]dioxolo[4,5-h]pyrrolo[2, 1-b][3]benzazepin-1-yl]4-(2,4-hexadienyl) ester, 4-[[(5,6,7,8-tetrahydro-5,5,8,8-tetramethyl-2-naphthalenyl)carbonyl]amino]-benzoate

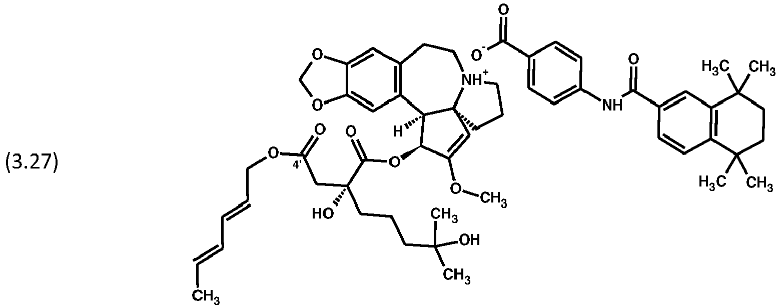

Molecular Formula (in the ionized state): C₃₄H₄₆NO₉.C₂₂H₂₄NO₃ (C₅₆H₇₀N₂O₁₂)
Formula Weight: 963.1612
Composition: C(69.83%) H(7.33%) N(2.91%) 0(19.93%)

A similar procedure of preparation to the one depicted in general procedure and in example 1 was used, except that homoharringtonine was replaced with its OP5215 analog and all-*trans*-retinoic acid was replaced with 4-[[(5,6,7,8-tetrahydro-5,5,8,8-tetramethyl-2-naphthalenyl)carbonyl]amino]-benzoic acid, (namely Am 580).

### Example 28

### Preparation of OP5215 adarotenoate or Butanedioic acid, 2-hydroxy-2-(4-hydroxy-4-methyl-pentyl)-, 1-[(1S,3aR,14bS)-1,5,6,8,9,14b-hexahydro-2-methoxy-4H-cyclopenta[a][1,3]dioxolo[4,5-h]pyrrolo[2, 1-b][3]benzazepin-1-yl]4-(2,4-hexadienyl) ester, (2E)-3-(4'-Hydroxy-3'-tricyclo[3.3.1.1^{3,7}]dec-1-yl[1,1'-biphenyl]-4-yl)-2-propenoate

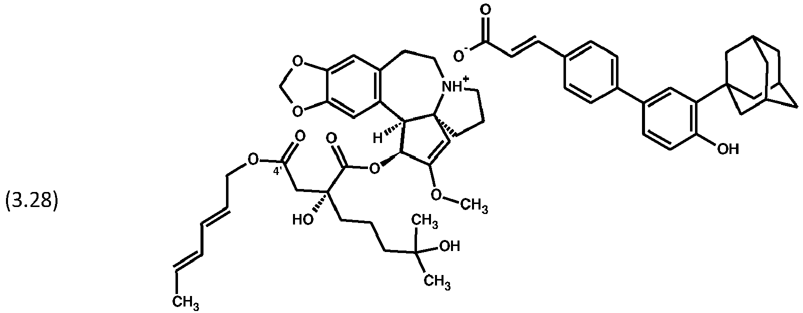

Molecular Formula (in the ionized state): C₃₄H₄₆NO₉.C₂₅H₂₅O₃ (C₅₉H₇₁NO₁₂)
Formula Weight: 986.19454
Composition: C(71.86%) H(7.26%) N(1.42%) 0(19.47%)

A similar procedure of preparation to the one depicted in general procedure and in example 1 was used, except that homoharringtonine was replaced with its OP5215 analog and all-*trans*-retinoic acid was replaced with (2E)-3-(4'-Hydroxy-3'-tricyclo[3.3.1.1^{3,7}]dec-1-yl[1,1'-biphenyl]-4-yl)-2-propenoic acid, (namely adarotene).

### EXAMPLE 29

### Preparation of OP5215 bexarotenoate or Butanedioic acid, 2-hydroxy-2-(4-hydroxy-4-methyl-pentyl) -, 1-[(1S,3aR,14bS)-1,5,6,8,9,14b-hexahydro-2-methoxy-4H-cyclopenta[a][1,3]dioxolo[4,5-h] pyrrolo[2,1-b][3]benzazepin-1-yl]4-(2,4-hexadienyl) ester, 4-[1-(5,6,7,8-Tetrahydro-3,5,5,8,8-pentamethyl-2-naphthalenyl)ethenyl]benzoate

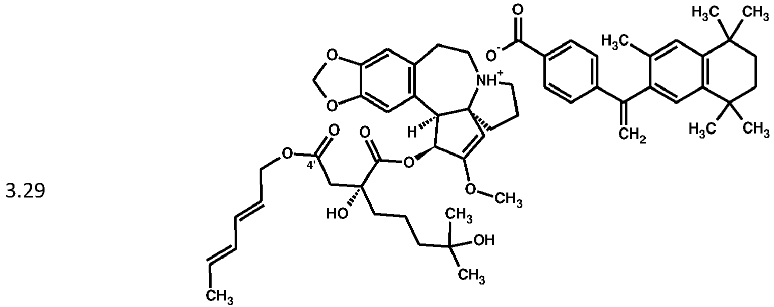

Molecular Formula (in the ionized state): C₃₄H₄₆NO₉.C₂₄H₂₇O₂ (C₅₈H₇₃NO₁₁)
Formula Weight: 960.20032
Composition: C(72.55%) H(7.66%) N(1.46%) 0(18.33%)

A similar procedure of preparation to the one depicted in general procedure and in example 1 was used, except that homoharringtonine was replaced with its OP5215 analog and all-*trans*-retinoic acid was replaced with 4-[1-(5,6,7,8-Tetrahydro-3,5,5,8,8-pentamethyl-2-naphthalenyl)ethenyl]benzoic acid, (namely bexarotene).

### EXAMPLE 30

### Testing on K562 leukemia cell line

### 1-Materials and Methods

Retinoates of harringtonines described in this invention were tested on K562 cell line which is derived from the pleural effusion of a 53-year-old female with chronic myelogenous leukemia (CML, bcr-abl positive) in terminal blast crises (Lozzio and Lozzio, Blood, 1975, p321). The cell line was purchased from ATCC (American Tissue Culture Collection) and grown in RPMI medium supplemented with 10% heat-inactivated fetal calf serum, 2 mmol/L glutamine, 50 IU/mol penicillin and 50 IU/mol streptomycin. All tested products were initially dissolved in 2N HCl (pH=2) (alkaloid base) or in aqueous solvent (salt) or in DMSO (retinoids), supplemented with ultrasound treatment during 1 h. Working concentrations were made by dilution in culture medium (pH 7). Cells in exponential growth phase were placed into 96-microwell plates in concentration 3x104/mL (total volume 200 µL/well) and incubated with drugs for 72 h at 37° C. in 5% CO2 humidified atmosphere. Following incubation, 28 µL of MTT 3-(4,5 dimethylthiazol-2-yl)-2,5 tetrazolium bromide] dye solution (Sigma, St. Louis, Mo.) was added to each well. During the following 6 h of incubation at 37° C., the purple formazan product was formed by conversion of the yellow MTT salt in the mitochondria of the viable cells. The resulting precipitate was dissolved in DMSO and the amount of converted MTT was quantified in an ELISA reader (MR 5000, Dynatech). Each product was tested in triplicate (3x8 wells). Every test contained at least one plate with HHT as control, added in the same concentration as the tested products.

### 2-Results

Table 1 represents the results of some examples of the compounds according to the present invention in K562 cell line compared to homoharringtonine tartrate as a salt involving an inactive anion, homoharringtonine as the reference harringtonines, cephalotaxine as inactive cephalotaxane, and finally each retinoid alone to compare to the corresponding harringtonine salt.

### EXAMPLE 31

### Testing on HL-60 leukemia cell line

### 1-Materials and Methods

Retinoates of harringtonines described in this invention were tested on HL-60 cell line, derived from acute promyelocytic leukemia (APL) cells. HL-60 is a promyelocytic cell line derived by S.J. Collins, et al. (Proc. Natl. Acad. Sci. USA 1978 p2458). Peripheral blood leukocytes were obtained by leukopheresis from a 36-year-old Caucasian female with APL (Gallagher R, et al. Blood 1978, p713) The cell line was purchased from ATCC (American Tissue Culture Collection) and the base growth medium was also completed with fetal bovine serum but at a final concentration of 20%, otherwise the same method than the one of example 10 was used.

### 2-Results

Preliminary screening using this tumor cell lines are gathered in table 1.

### EXAMPLE 32

### Testing on Breast Cancer cell lines

Operating Procedure were performed as described in the recent paper of Yakhni et al [Yakhni AM J CANCER RES 2019; 9(5) in press "Homoharringtonine, on approved anti-leukemia drug, suppresses triple negative breast cancer growth through a rapid reduction of anti-apoptotic protein abundance"]

### 1. Cell lines and culture conditions

The cell lines used in this study were MDA-MB-231 (ATCC® HTB-26™), MDA-MB-468 (ATCC® HTB-132™), MDA-MB-157 (ATCC® HTB-24™) and CAL-51. Those lines were chosen to represent the *BRCA1*/*2* non-mutated TNBC transcriptomic subtypes, currently most in need of efficacious treatment due to lower sensibility of *BRCA1* wild-type TNBC to the DNA-damaging chemotherapy (Masuda et al., Clin Cancer Res. 2013, p5533; Jiang et al., PLoS Med. 2016, pe1002193; Park et al. ESMO Open. 2018, pe000357). CAL-51 line represents the mesenchymal transcriptomic subtype whereas MDA-MB-157 and MDA-MB-231 represent the mesenchymal stem-like subtype (Lehmann et al. J Clin Invest. 2011, p2750). MDA-MB-468 was used as a model of aggressive, PTEN-negative TNBC, with hyperactivated EGFR signaling pathway due to *EGFR* amplification (Filmus et al., Biochem Biophys Res Commun. 1985, p898). MDA-MB-468 transcriptome classifies it into the basal-like 1 subtype (49). The MDA lines were purchased from the American Type Culture Collection (ATCC, Manassas, VA, USA). The CAL-51 was kindly provided by Dr Gerard Milano, from the Centre Antoine Lacassagne, Nice, France, where the line has been established (Gioanni et al., Br J Cancer 1990, p8). After initial expansion, the cell line stock vials were conserved, in liquid nitrogen, at the Biological Resource Center of Jean Perrin Comprehensive Cancer Center (biobank ID BB-0033-00075, Clermont-Ferrand, France). The MDA cell lines were cultured in the Leibovitz L-15 medium, whereas CAL-51 line was maintained in DMEM (both media from Invitrogen Life Technologies, Carlsbad, CA, USA). The media were supplemented with 10% heat-inactivated fetal bovine serum and 20 mg/mL gentamicin. CAL-51 line was kept at 37°C, in a humidified atmosphere with 5% CO₂; the MDA lines were kept under the same conditions, however, without CO₂, in a free gas exchange with atmospheric air. All cell lines were maintained in monolayer culture, in the sub-confluent status, with no more 30 passages of the cells thawed from the stock vial.

Homoharringtonine was supplied by LeukePharma Inc. (Houston, TX, USA) as water-soluble, crystalline tartaric acid salt. Initial stock solution of HHT-tartrate was prepared at 10 mM in sterile deionized water, aliquoted and kept at -80°C. For each experiment, a fresh HHT solution was prepared by dissolving the HHT stock in phosphate-buffered saline (PBS, GIBCO™, Invitrogen, Cergy-Pontoise, France).

### 2. Cell viability assay

Cell viability was assessed using the sulforhodamine B (SRB) assay (Sigma-Aldrich, St Louis, MO, USA) according to the method described by Vichai and Kirtikara (Nat Protoc. 2006, p1112). Cells were seeded in in 96-well plates, at 5x10³ or 1x10³ cells/well (the MDA cell lines and CAL-51, respectively), left overnight to adhere and then treated with growing concentrations of HHT for 24h, 48h or 72h. After incubation with the drug, cells were washed twice with PBS and left for 72 h in the corresponding complete culture medium. Then the cells were fixed with trichloroacetic acid (final concentration 10%) at 4°C for 1 h, followed with plate washing with water five times, air drying and staining with 0.4% SRB dissolved in 1% acetic acid, at room temperature, for 30 min. The plates were subsequently washed four times with 1% acetic acid to remove unbound stain. To solubilize the protein-bound dye, 10 mM Tris base solution was added to each well. The cell viability, estimated through the present protein biomass, was determined by measuring optical density of the dye, using a spectrophotometer at wavelength of 540 nm (BMG Labtech, Ortenberg, Germany).

### 3. Cell cycle analysis

Cells were seeded into 6-well plates at 5x10⁴ cells per well (each cell line), left overnight to adhere, and then treated or not with different concentrations of HHT for 48h. At the end of the incubation with the drug, the adherent cells were dissociated by 0.25% trypsin (59427C, Sigma-Aldrich, Saint-Quentin Fallavier, France) and collected by centrifugation at 500 g for 10 min. Cell pellets were washed twice with PBS, and cell membranes were disrupted by repeated cycles of freezing and thawing in liquid nitrogen. Then cells were incubated with 200 µl of ribonuclease A (1 mg/ml) and stained with 200 µl of propidium iodide solution (100 µg/ml) (both reagents from Sigma-Aldrich, Saint-Quentin Fallavier, France). Fluorescence of cells was analyzed on a flow-cytometer (Cytomics FC 500 MPL, Beckman Coulter, Brea, CA, USA). Cell cycle distributions were calculated using ModFit LT 2.0 software (Verity Software House, Topsham, ME, USA).

### 4. Results

Preliminary screening using these tumor cell lines are gathered in table 1.

### EXAMPLE 33

### Effet of Homoharringtonine Retinoate in Breast Cancer: Animal studies

The experiments were conducted according to the Guide for the Care and Use of Laboratory Animals published by the US National Institutes of Health (NIH Publication n°85-23, revised 1996) and approved by the Ethic Committee of Clermont-Ferrand, France. All mouse studies were conducted in the animal facility of the INSERM U1240. Six weeks old Swiss nu/nu mice were purchased from Charles River Laboratories France and housed in standard conditions (n=5 per cage on ventilated racks, at 21-24 °C, 60% humidity, 12 h light/12 h dark cycle) with ad libitum access to irradiated food and water.

For establishment of xenografts, groups of 10 mice were injected subcutaneously, in the right flank area with 1x10⁷ MDA-MB-231 or 5x10⁶ MDA-MB-468 cells suspended in 150 µl of culture medium/Matrigel (BD Biosciences, San Jose, CA, USA) in a 4:1 ratio.

For assessment of HHT toxicity, groups of 6 mice were injected with sterile saline or 1, 2, 4 or 8 mg/kg of the drug, subcutaneously, bi-daily, over 7 days. The animals were left to rest 2 more days and sacrificed at day 10. The solution for injection were freshly prepared by serial dilutions of the stock HHT-tartrate in sterile saline water (0.9% NaCl) and filtered through sterile filters having 0.25 µM pore size (Merck/Millipore, Burlington, MA, USA). The number of live animals was determined at days 4, 8 and 10, after treatment initiation.

For evaluation of HHT efficacy in vivo, when xenografts reached a volume of 200±20 mm³, mice were randomized into the control or the treated group. The MDA-MB-231 xenografts were treated by 1 mg/kg of HHT, using the same protocol as for determination of chronic HHT toxicity. The MDA-MB-468 xenografts were treated by 0.5 mg/kg of HHT, as 1 mg/kg of HHT was too toxic for the animals (all mice died after only a few days, in a preliminary experiment; data not shown). Tumor size was non-invasively assessed using a digital caliper on days 3, 6, 8 and 10 after treatment initiation. Tumor volume was calculated using the following formula: V=(length×width²)/2.

### EXAMPLE 34

### Testing on Neuroblastoma cell lines

### 1. Cell culture

Two cell lines derived from glioblastoma were used in this study: SF763 and U251MG, which were kindly provided by Dr. C. Delmas (Claudius Regaud Center, Toulouse, France).
these lines were cultured in DMEM medium (Dulbecco's modified Eagle's Me-dium, GIBCO ™, Invitrogen, Cergy-Pontoise, France) supplemented with non-essential amino acids (1%), sodium pyruvate (1%) and gentamycin (0.05%, GIBCO ™, Invitro-gen, Cergy-Pontoise, France). The media were supplemented with SVF (Fetal Veal Serum, GIBCO ™, Invitrogen, Cergy-Pontoise, France) at 10%. The culture was carried out in 25 cm 2 flasks under the condition of 37 ° C and 5% CO₂. Generally, the cells were subcultured twice a week when they were in confluence.

### 2. Results

Preliminary screening using these tumor cell lines are gathered in table 1.

### EXAMPLE 35

### Testing on neuroblastoma cell lines combined with irradiation

### Irradiation

After 3 hours of treatment with the compounds as described in example 14, irradiation was carried out with 2, 4, 6, 8 or 10 Gray. For each cell line, 3 independent experiments, each with 3 replicates, were carried out (control, the compound alone, irradiation alone and the compound plus irradiation. The irradiation was carried out at room temperature under a Novalis TX® linear accelerator with an energy of 6 MV and a dose rate of 400cGy / min (Department of Radiotherapy, Jean Perrin Cancer Center at Clermont-Ferrand in France). The doses were calculated and delivered by a specialized biophysicist.

### Results

Preliminary screening using these tumor cell lines are gathered in table 1.

**TABLE 1: SCREENING OF CYTOTOXICITY**

| Compound of example # | Cation | Anion | Salt code | Generation | K562 (CML) IC₅₀ (nM) | HL60 (APL) IC₅₀ (nM) | M DA-MB-468 (BC) IC₅₀ (nM) |
|---|---|---|---|---|---|---|---|
| 1 | HHT. | ATRA ATRA | HHT.ATRA | 1st | <50 | <100 | <50 |
| 2 | HA. | CRA | HA.ATRA | 1^{ST} | <100 | >100 | N/A |
| 3 | HHT. | | HHT.CRA | | <50 | N/A | |
| 4 | HHT. | ACITRET | HHT.ACITRET | 2nd | <50 | <100 | N/A |
| 5 | HHT. | Am80 | HHT.Am80 | 3rd | <20 | <50 | <20 |
| 6 | HHT. | ADARO | HHT.ADARO | 3rd | <50 | <100 | N/A |
| 7 | HHT. | BEXA | HHT.BEXA | 3rd | <20 | <20 | <20 |
| 8 | HHT. | ADAPA | HHT.ADAPA | 3rd | <50 | <100 | <50 |
| 9 | HA. | ADAPA | HA.ADAPA | 3rd | <100 | N/A | N/A |
| 10 | HHT. | BMS195 | HHT.BMS195 | 3rd | <50 | <100 | <50 |
| 11 | HHT. | AHPN | HHT.AHPN | 3rd | <50 | <100 | <50 |
| 12 | HHT. | CD1530 | HHT.CD1530 | 3rd | <50 | <100 | <50 |
| 13 | HHT. | AROT | HHT.AROT | 3rd | <50 | N/A | N/A |
| 14 | HA. | AROT | HA.AROT | 3rd | <100 | N/A | N/A |
| 15 | HHT. | BMS753 | HHT.BMS753 | 3rd | <50 | <100 | <50 |
| 16 | HHT. | CD3106 | HHT.CD3106 | 3rd | <50 | <100 | N/A |
| 17 | HHT. | TMPP | HHT.TMPP | 2nd | <50 | <100 | N/A |
| 18 | HHT. | MM11253 | HHT.MM11253 | 3rd | <50 | <100 | <50 |
| 19 | AHHT. | ATRA | AHHT.ATRA | 1st | >100 | N/A | >100 |
| 20 | AHHT. | ACITRET | AHHT.ACITRET | 2nd | N/A | N/A | N/A |
| 21 | AHHT. | ADAPA | AHHT.ADAPA | 3rd | N/A | N/A | N/A |
| 22 | EAHHT. | ADAPA | EAHHT.ADAPA | 3rd | N/A | N/A | N/A |
| 23 | HHT. | TAZAROT | HHT.TAZAROT | 3rd | <50 | <100 | <50 |
| 24 | HHT. | PALOVARO | HHT.PALOVARO | 3rd | <50 | <100 | N/A |
| 25 | HHT. | Am580 | HHT.Am580 | 3rd | <20 | <50 | <20 |
| 26 | OP5215 | ATRA | OP5215.ATRA | 1st | <10 | <20 | <10 |
| 27 | OP5215 | ACITRET | OP5215.ACITR | 2nd | <10 | <20 | <10 |
| 28 | OP5215 | Am80 | OP5215.Am80 | 3rd | <10 | <20 | <10 |
| 29 | OP5215 | Am580 | OP5215.Am58 | 3rd | <10 | <20 | <10 |
| 30 | OP5215 | ADARO | OP5215.ADAR | 3rd | <10 | <20 | N/A |
| 31 | OP5215 | BEXA | OP5215.BEXA | 3rd | <10 | <20 | <10 |

### Use of infrared spectroscopy to monitor the formation of salts of harringtonines in solution

Infrared spectroscopy was used for the identification of retinoates of harringtonines. In fact, the IR spectrum of the latter is not the addition of each component spectrum as if it were a simple mixture of a harringtonine and a retinoid, but we are in the presence of a new spectrum wherein, for example, the very strong bands in the range 1670-1700 cm⁻¹, characteristic of the C=O stretching of non-ionized aromatic or α-β ethylenic acid function, completely disappeared, demonstrating unambiguously the salt formation. This feature has even been used to monitor the kinetics of salt formation (see table 2). Reproductions of infrared spectra below exhibit on the same page the spectrum of the starting retinoid and its salt corresponding with a harringtonine for comparison.

High resolution ¹H-NMR spectroscopy shows much less difference as found in our previous research on pharmacologically inactive acid salts [Robin et al. PCT Int. Appl.(2015), WO 2015 101628 A1 2015 0709]. Use of X-ray diffraction for crystallin salts is in progress.

**TABLE 2: Carbonyl Infrared Features of Retinoates of Harringtonines and their precursors**

| ENT. # | HARRINGTONIN-NES or CATION | RETINOIDS or ANION | FIG # | Ex. | Formula # | C=O, cm¹ R**CO**OR' | C=O, cm¹ Ar-**CO**OH | OTHER | IR cm¹ Freq |
|---|---|---|---|---|---|---|---|---|---|
| | | First Generation Retinoids (directly derived from vitamine A) | | | | | | | |
| 1 | HHT | N/A | 1a | 1 | | 1742 | N/A | | |
| 2 | N/A | all-*trans*-retinoic acid | 1c | 1 | | N/A | 1687 | | |
| 3 | HHT | retinoate R-COO | 1d | 1 | 3.01 | 1745 | Abs. | | |
| 4 | HA | N/A | 2a | 2 | | 1748 | N/A | | |
| 5 | HA | retinoate R-COO | 2b | 2 | 3.02 | 1745 | Abs. | | |
| 6 | N/A | 13-*cis*-retinoic acid | 3a | 3 | | N/A | 1670 | | |
| 7 | HHT | cis -retinoate R-COO | 3b | 3 | 3.03 | 1747 | Abs. | | |

| | | Second Generation Retinoids (substituted 1st generation derivatives) | | | | | | | |
|---|---|---|---|---|---|---|---|---|---|
| 8 | N/A | acitretin | 4a,17a | 4 | | N/A | 1697 | | |
| 9 | HHT | acitretinate (oate) | 4b | 4 | 3.04 | 1746 | Abs. | | |
| 10 | HA | acitretinate (oate) | 17b | 17 | | 1748 | Abs. | | |

| | | Third Generation Retinoids (retinobenzoic and related derivatives) | | | | | | | |
|---|---|---|---|---|---|---|---|---|---|
| 11 | N/A | tamibarotene (AM80) | 5a | 5 | | N/A | 1700 | ArNH-COAr | 1649 |
| 12 | HHT | tamibarotenoate | 5b | 5 | 3.05 | 1743 | Abs. | ArNH-COAr | 1653 |
| 13 | N/A | adarotene (ST1926) | 6a | 6 | | N/A | 1687 | | |
| 14 | HHT | adarotenoate | 6b | 6 | 3.06 | 1742 | Abs. | | |
| 15 | N/A | bexarotene (LG69) | 7b | 7 | | N/A | 1677* | | |
| 16 | HHT | bexarotenoate | 7c | 7 | 3.07 | 1744 | Abs. | | |
| 17 | N/A | adapalene (CD271) | 8a | 8 | | N/A | 1687 | | |
| 18 | HHT | adapalenoate | 8b | 8 | 3.08 | 1746 | Abs. | | |
| 19 | N/A | adapalene (CD271) | 9a | 9 | | N/A | 1684 | | |
| 20 | HA | adapalenoate | 9b | 9 | 3.09 | 1747 | Abs. | | |
| 21 | N/A | BMS 195'614 | 10a | 10 | | N/A | 1681 | | |
| 22 | HHT | BMS 195'614, HHT | sal 10b | 10 | 3.10 | 1744 | Abs. | | |
| 23 | N/A | AHPN(CD 437) | 11a | 11 | | N/A | 1686 | | |
| 24 | HHT | AHPN, HHT salt | 11b | 11 | 3.11 | 1746 | Abs. | | |
| 25 | N/A | CD 1530 | 12a | 12 | | N/A | 1677 | | |
| 26 | HHT | CD 1530, HHT salt | 12b | 12 | 3.12 | 1745 | Abs. | | |
| 27 | N/A | arotinoic acid (TTNPB) | 13a | 13 | | N/A | 1671 | | |
| 28 | HHT | Arotinoate | 13b | 13 | 3.13 | 1746 | Abs. | | |
| 29 | HA | Arotinoate | 14b | 14 | 3.14 | 1748 | Abs. | | |
| 30 | N/A | BMS 753 | 15a | 15 | | N/A | 1685 | >C=O | 1747 |
| 31 | HHT | BMS 753 salt | 15b | 15 | 3.15 | 1744 | Abs. | ArNH-COAr | 1654 |
| 32 | N/A | CD 3106 | 16a | 16 | | N/A | 1682 | | |
| 33 | HHT | CD 3106, HHT salt | 16b | 16 | 3.16 | 1744 | Abs. | | |

| | | Misc. retinoates (three generations | | | | | | | |
|---|---|---|---|---|---|---|---|---|---|
| 34 | HA | Acitretinate | 17b | 17 | 3.17 | 1748 | Abs. | | |
| 35 | AHHT | Anhydro-HHT | 18a | 18 | | 1738 | Abs. | | |
| 36 | AHHT | *Trans*-retinoate | 18b | 18 | 3.18 | 1739 | Abs. | | |
| 37 | AHHT | Acitretinate | 19a | 19 | 3.19 | 1739 | Abs. | | |
| 38 | N/A | Adapalene | 20a | 20 | | N/A | 1686 | | |
| 39 | AHHT | Adapalenoate | 20b | 20 | 3.20 | 1738 | Abs. | | |

Structural formula are given in the corresponding example cited in column "Ex."

### Abbreviations

HHT is homoharringtonine (formula 1.7); HHT⁺ is HHT cation; HA is harringtonine (formula 1.6); HA⁺ is HA cation; AHHT is anhydrohomoharringtonine (formula 1.8); AHHT⁺ is AHHT cation; EAHHT is 2'-epi-anhydrohomoharringtonine; EAHHT⁺ is EAHHT cation; structural formulas are depicted in corresponding examples; N/A means non applicable.

## Claims

1. Salt of the following formula (I): 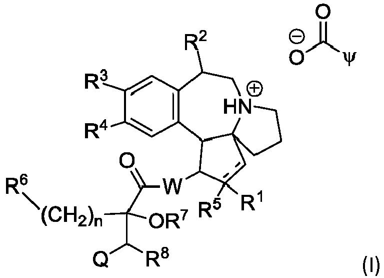 wherein
W represents O, S or NH,
Q and R⁶ are each independently selected in the group consisting of:
a linear or branched C₁-C₁₂ hydrocarbonated chain, said hydrocarbonated chain being optionally interrupted by one or more heteroatoms or groups selected among O, S, N or NH and optionally substituted with one or more groups selected among C₁-C₆ alkyl, OH, O-(C₁-C₆)-alkyl, oxo, halogen, an aryl and a heteroaryl,
an aryl,
a heteroaryl
a (C₃-C₇)-cycloalkyl, and
a heterocycle,
R¹ is selected in the group consisting of H, OH, O-(C₁-C₁₂)-alkyl, O-aryl-(C₁-C₁₂)-alkyl, O-(C₂-C₁₂)-alkenyl and O-(C₃-C₇)-cycloalkyl, and
R² is selected in the group consisting of H or OH, or
R¹ and R² form together -O-,
R³ and R⁴ both represent an O-CH₃ group or R³ and R⁴ form together -OCH₂O-,
R⁵ is selected in the group consisting of H, C₁-C₁₂ alkyl or O-(C₁-C₆)-alkyl,
R⁷ is selected in the group consisting of H and (C₁-C₆)alkyl,
or R⁶ and R⁷ form together -CMe₂-,
R⁸ is H, OH, C₁-C₁₂ alkyl or O-C₁-C₁₂ alkyl, 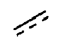 represents a single or a double bond,
n is an integer between 0 and 8,
ψ is selected in the group consisting of the following formulas (A), (B) and (C): 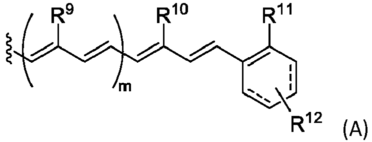 wherein:
R⁹ to R¹¹ are each independently selected in the group consisting of
a linear or branched C₁-C₁₂ hydrocarbonated chain, said hydrocarbonated chain being optionally interrupted by one or more heteroatoms or groups selected among O, S, N or NH and optionally substituted with one or more groups selected among C₁-C₆ alkyl, OH, O-(C₁-C₆)-alkyl, oxo, halogen, an aryl, a heteroaryl, a (C₃-C₇)-cycloalkyl and a heterocyclyl,
an aryl,
a heteroaryl
a (C₃-C₇)-cycloalkyl, and
a heterocyclyl,
R¹² represent one or more substituents each independently selected in the group consisting of OH, halogen, (C₁-C₆)-alkyl, (C₂-C₆)-alkenyl, C₂-C₆-alkynyl and O-(C1-C6)-alkyl,
m is an integer between 0 and 6 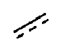
represents a single or a double bond, 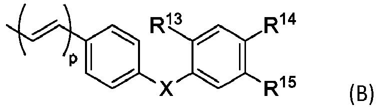
wherein:
X represents a single bond or a (C₁-C₆) hydrocarbonated chain, said hydrocarbonated chain being optionally interrupted by one or more heteroatoms or groups selected among O, S, N or NH and optionally substituted with one or more groups selected among C₁-C₆ alkyl, C₂-C₆ alkenyl, OH, O-(C₁-C₆)-alkyl, oxo and halogen,
R¹³ is H or (C₁-C₆)alkyl, optionally substituted with a heteroaryl, and
R¹⁴ and R¹⁵ are each independently selected in the group consisting of
a linear or branched C₁-C₁₂ hydrocarbonated chain, said hydrocarbonated chain being optionally interrupted by one or more heteroatoms or groups selected among O, S, N or NH and optionally substituted with one or more groups selected among C₁-C₆ alkyl, OH, O-(C₁-C₆)-alkyl, oxo, halogen, an aryl, a heteroaryl, a (C₃-C₇)-cycloalkyl and a heterocyclyl,
an OH group,
an aryl,
a heteroaryl
a (C₃-C₇)-cycloalkyl, and
a heterocyclyl, or
R¹⁴ and R¹⁵ form together a carbocycle or a heterocycle optionally substituted with one or more groups selected among C₁-C₆ alkyl, OH, O-(C₁-C₆)-alkyl, oxo, halogen, an aryl, a heteroaryl, a (C₃-C₇)-cycloalkyl and a heterocyclyl,
p is 0 or 1, 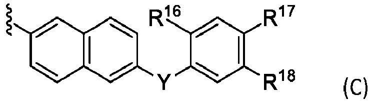 wherein
Y represents a single bond or a (C₁-C₆) -hydrocarbonated chain, said hydrocarbonated chain being optionally interrupted by one or more heteroatoms or groups selected among O, S, N or NH and optionally substituted with one or more groups selected among C₁-C₆ alkyl, C₂-C₆ alkenyl, OH, O-(C₁-C₆)-alkyl, oxo, halogen, an aryl, a heteroaryl, a (C₃-C₇)-cycloalkyl or a heterocyclyl,
R¹⁶ is H or (C₁-C₆)alkyl, and
R¹⁷ and R¹⁸ are each independently selected in the group consisting of
a linear or branched C₁-C₁₂ hydrocarbonated chain, said hydrocarbonated chain being optionally interrupted by one or more heteroatoms or groups selected among O, S, N or NH and optionally substituted with one or more groups selected among C₁-C₆ alkyl, OH, O-(C₁-C₆)-alkyl, oxo, halogen, an aryl, a heteroaryl, a (C₃-C₇)-cycloalkyl and a heterocyclyl,
an OH group,
an aryl,
a heteroaryl
a (C₃-C₇)-cycloalkyl, and
a heterocyclyl, or
R¹⁷ and R¹⁸ form together a carbocycle or a heterocycle optionally substituted with one or more groups selected among C₁-C₆ alkyl, OH, O-(C₁-C₆)-alkyl, oxo, halogen, an aryl, a heteroaryl, a (C₃-C₇)-cycloalkyl and a heterocyclyl.

2. The salt according to claim 1, wherein it corresponds to the following formula (I'):

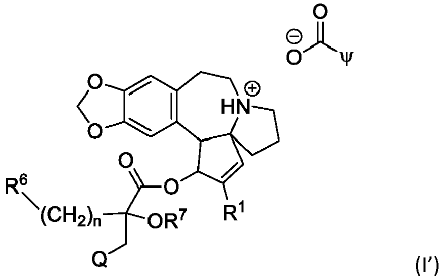

3. The salt according to claim 1, wherein the cationic part of the salt of formula (I) is selected in the group consisting of:

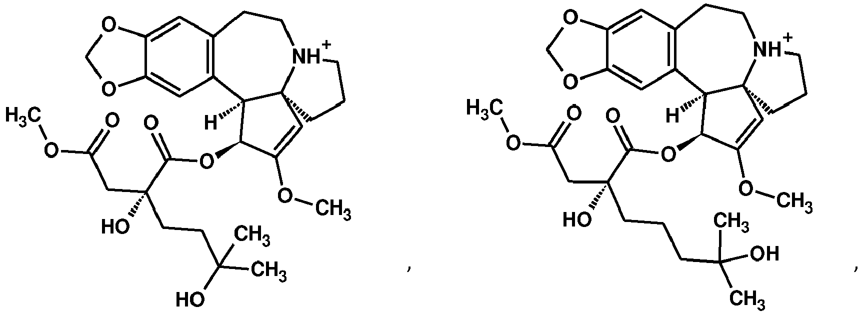

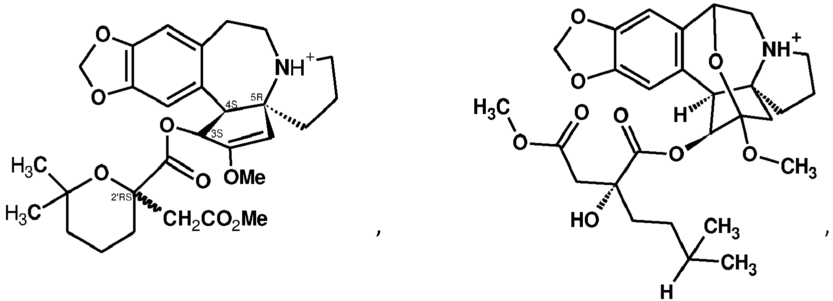

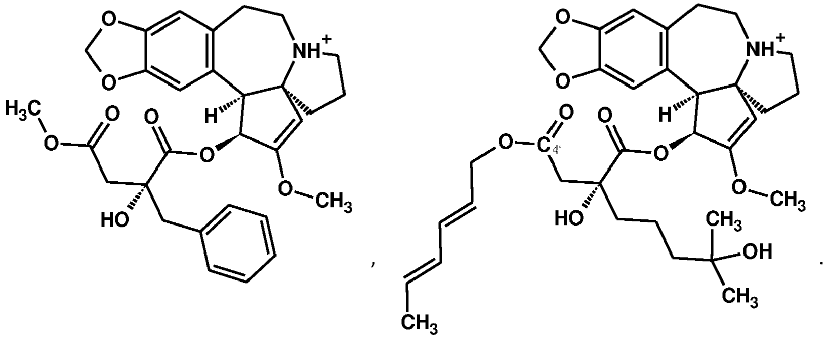

4. The salt according to claim 1, wherein the anionic part is selected in the group consisting of:

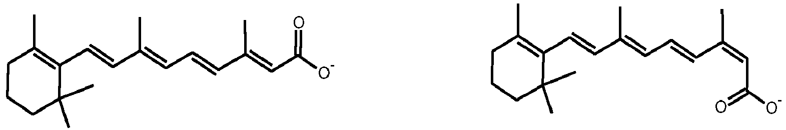

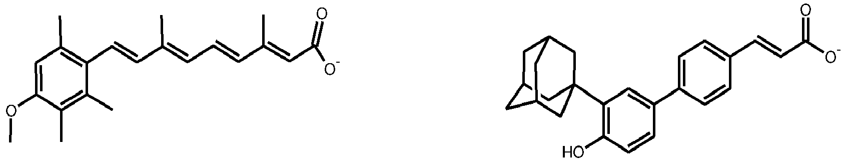

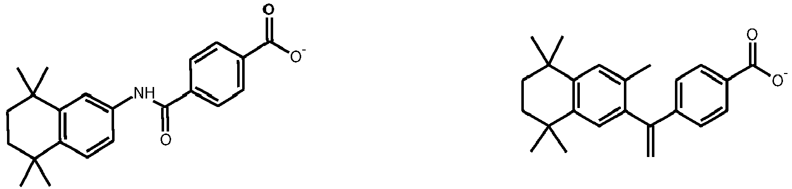

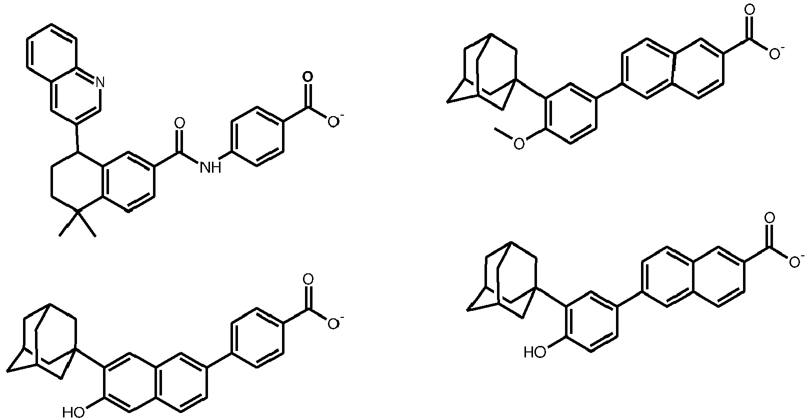

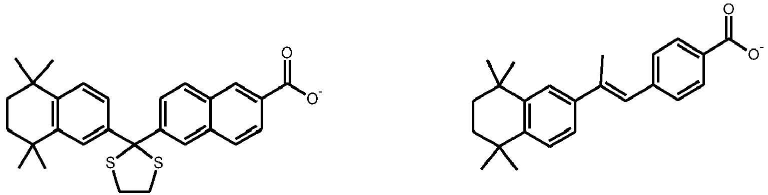

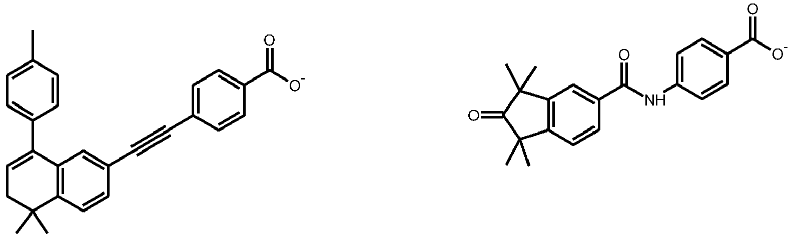

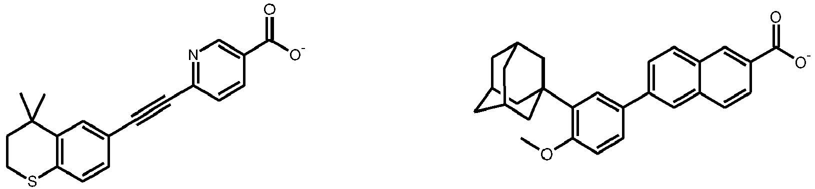

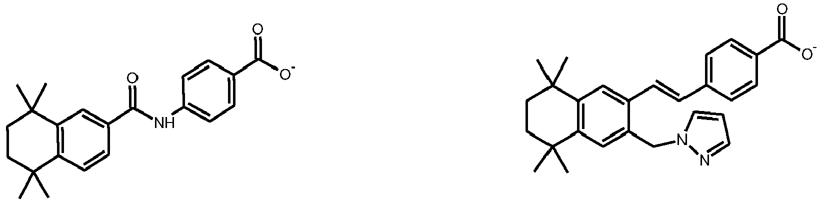

5. The salt according to claim 1, wherein it is selected in the group consisting of :

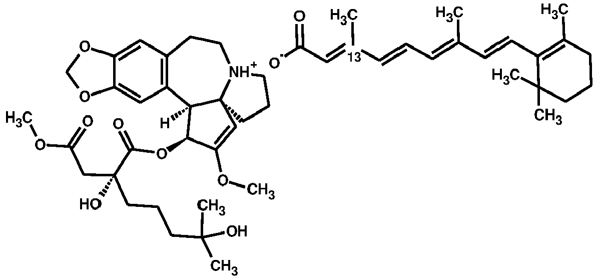

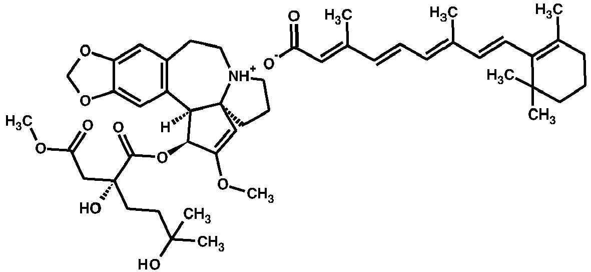

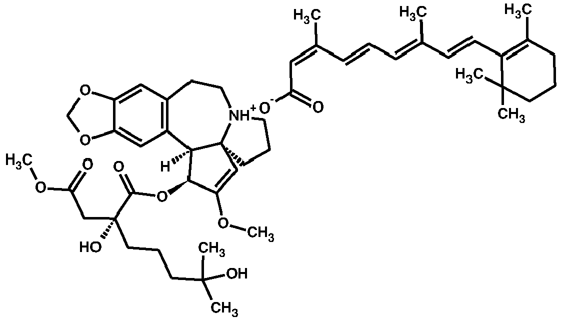

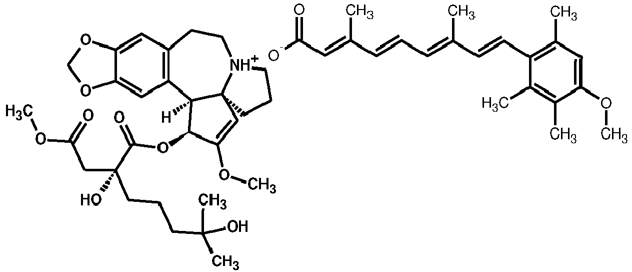

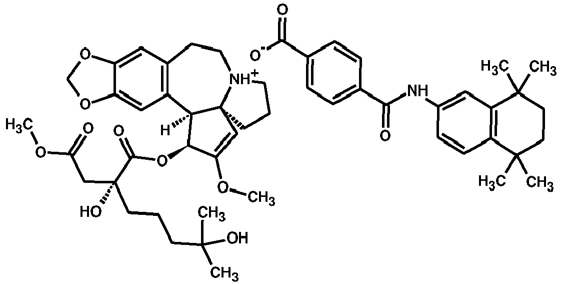

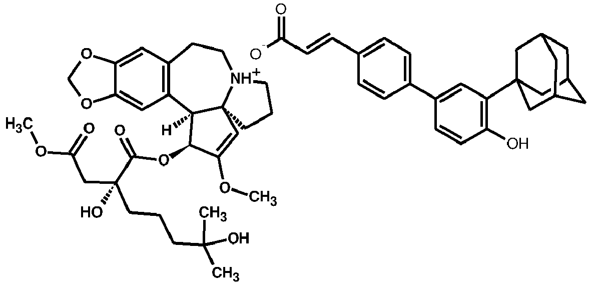

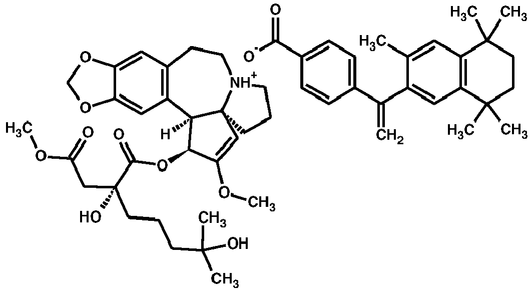

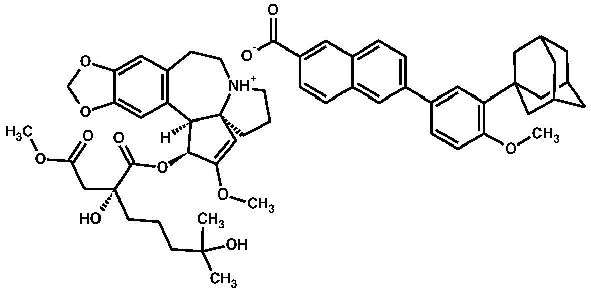

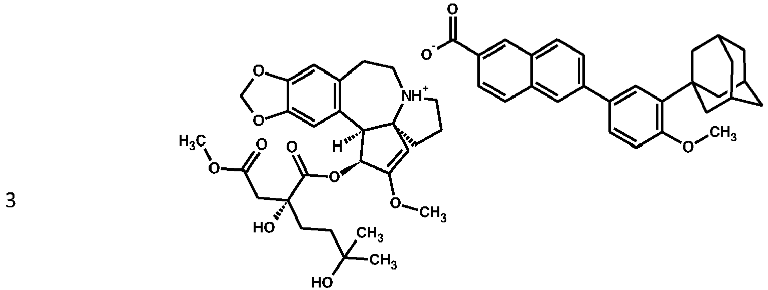

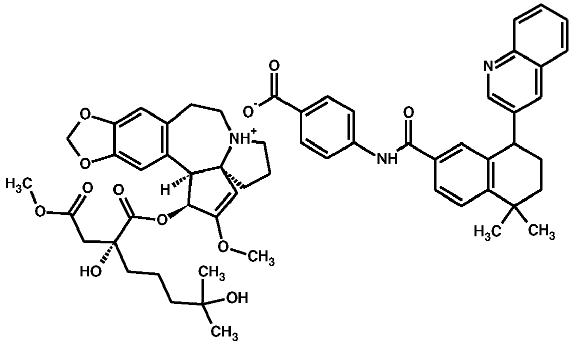

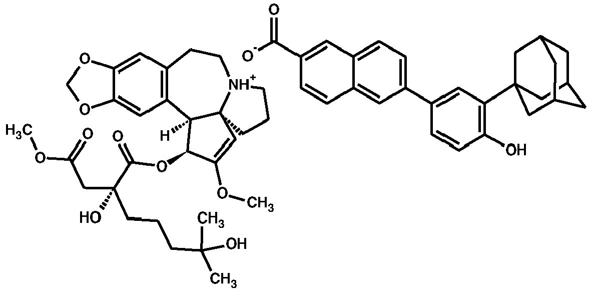

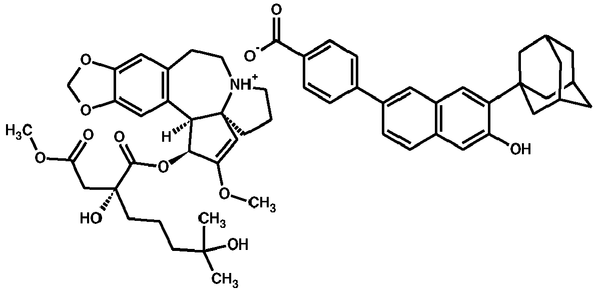

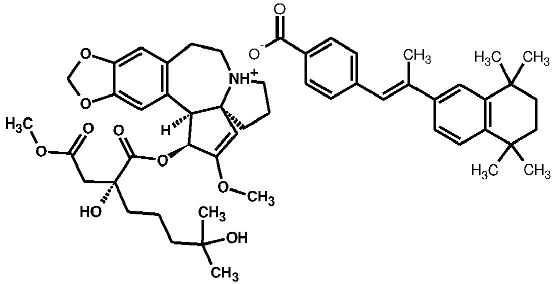

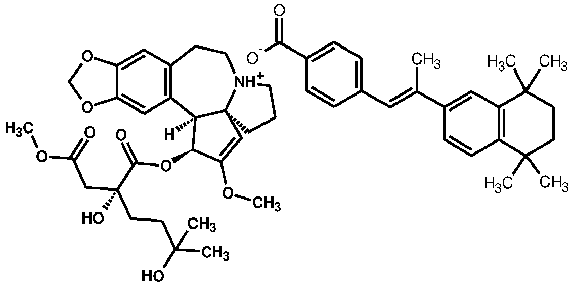

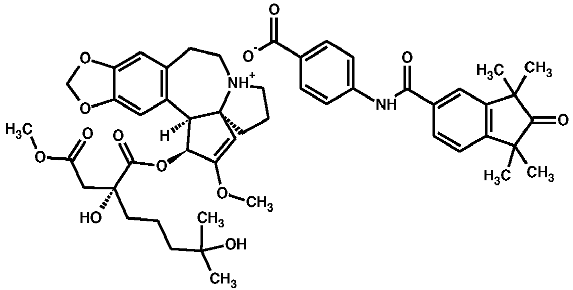

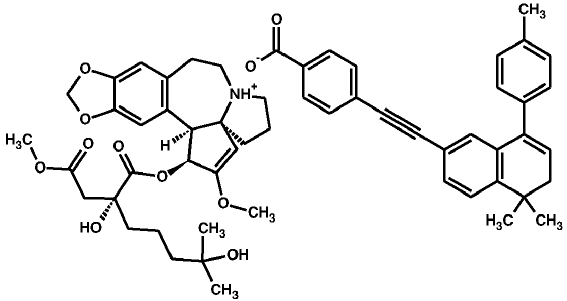

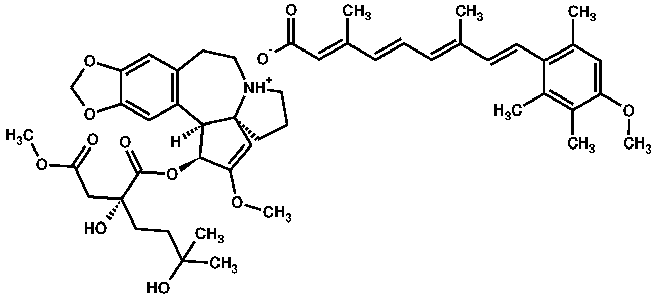

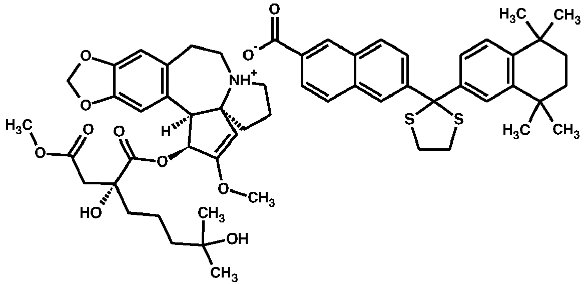

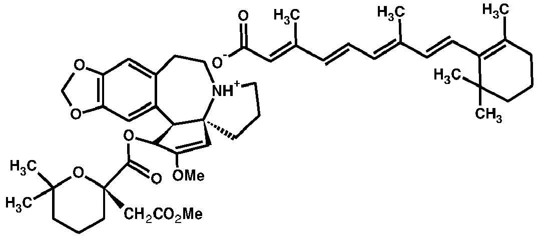

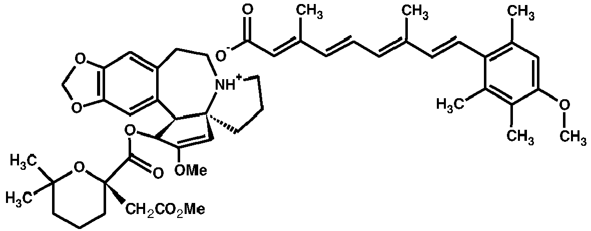

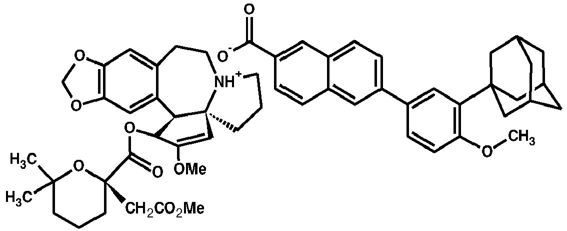

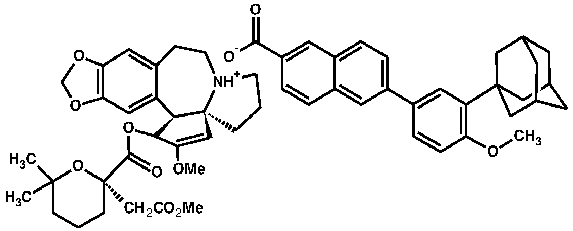

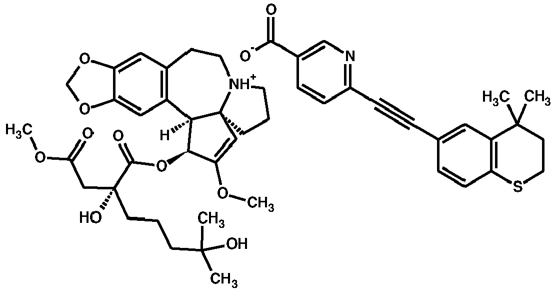

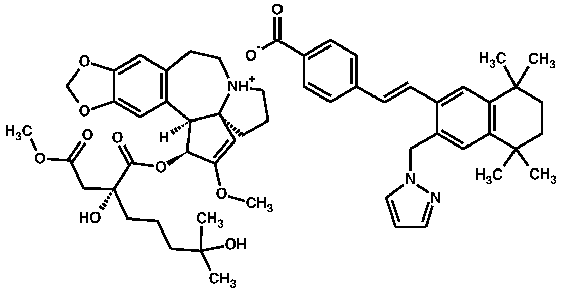

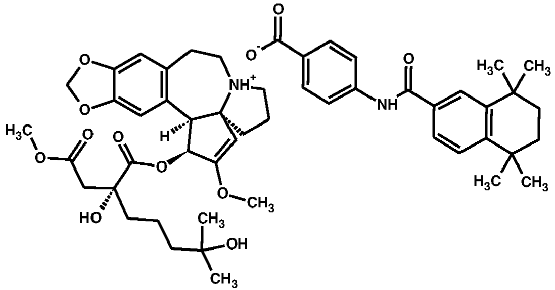

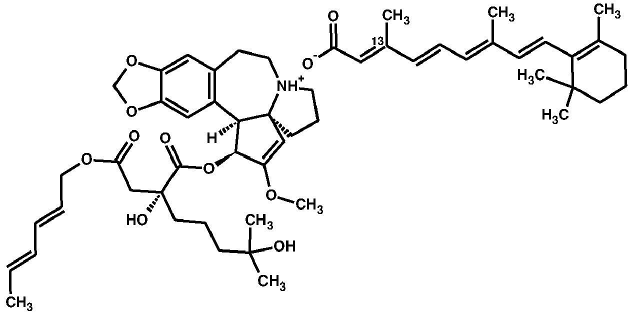

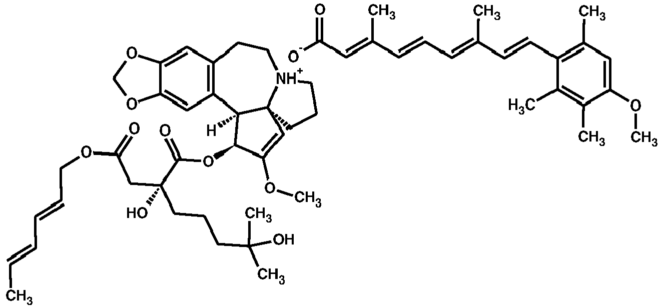

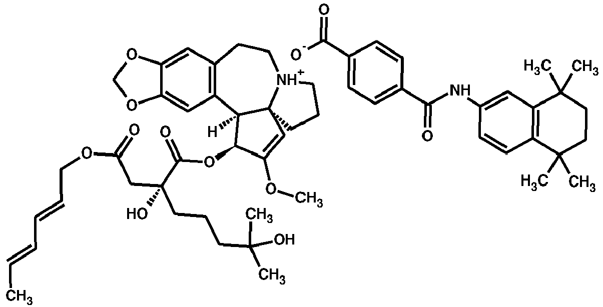

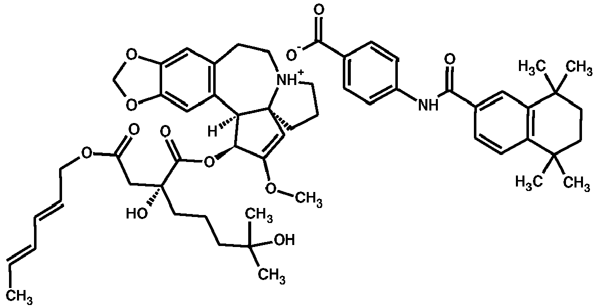

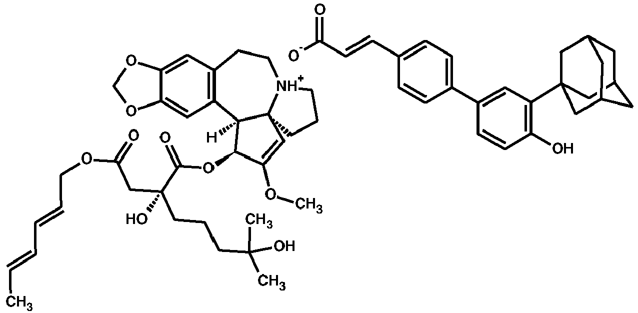

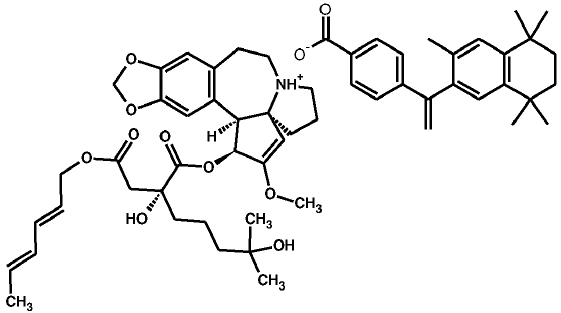

6. A pharmaceutical composition comprising a salt of formula (I) as defined in claim 1 and at least one pharmaceutically acceptable excipient.

7. A method for preventing or treating diseases selected among cancer, leukemias, lymphomas, myelodysplastic syndrome, autoimmune diseases, skin diseases, neurological diseases such as Alzheimer's disease, inflammatory bowel disease, such as Crohn's disease, and viral infections comprising administering to a patient in need thereof an effective dose of the salt of formula (I) as defined in claim 1.

8. The method according to claim 7, wherein cancer is triple negative breast cancer.

9. A method for preventing or treating diseases selected among cancer, leukemias, lymphomas, myelodysplastic syndrome, autoimmune diseases, skin diseases, neurological diseases such as Alzheimer's disease, inflammatory bowel disease, such as Crohn's disease, and viral infections comprising administering to a patient in need thereof the pharmaceutical composition as defined in claim 6.

10. The method according to claim 9, wherein cancer is triple negative breast cancer.

11. A method for preventing or treating viral infection comprising administering to a patient in need thereof an effective dose of a compound of the following formula (III): 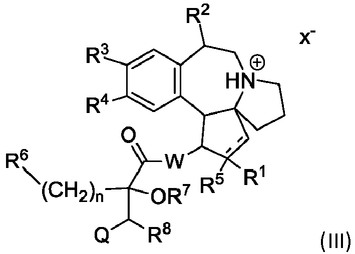
wherein Q, W and R¹ to R⁸ are as described in claim 1 and,
X- is a mineral anion such as, but not limited to, hydrochorid, sulfate and phosphate or an organic carboxylate comprising 1 to 12 carbon atoms, preferably selected among fumarate, maleate, citrate, malate, tartrate, tartronate, succinate, itaconate, pamoate, citramalate, malonate, benzoate, benzenesulfonate, methanesulfonate, 1,5-naphtalene disulfonate, cyclohexanesulfamate, formate, lactate, mandelate, vanillate, oxaloacetate, glutarate, adipate, squarate, 3,4,5-trimethoxybenzoic, ascorbate and salicylate,
or comprising administering a pharmaceutical composition comprising thereof.

12. A method of preparation of salt of formula (I) of claim 1, said method comprising the following steps:
(a) mixing the harringtonine of the following formula (II): 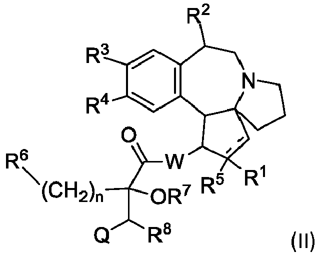 with a carboxylic acid of formula ψ-COOH,
(b) bringing the mixture resulting from step (a) to the liquid state and heating it,
(c) monitoring the kinetic of the reaction using a spectrometric method to control the disappearance of the acid function of ψ-COOH,
(d) removing the solvent and drying the resulting solid under vacuum to afford crystal salt of formula (I).
